(19) Europäisches Patentamt
European Patent Office
Office européen des brevets

(11) EP 4 631 955 A1

(12) EUROPEAN PATENT APPLICATION
published in accordance with Art. 153(4) EPC

(43) Date of publication:
**15.10.2025 Bulletin 2025/42**

(21) Application number: **23912291.4**

(22) Date of filing: **27.12.2023**

(51) International Patent Classification (IPC):
***C07K 1/32*** *(2006.01)* ***C07K 5/12*** *(2006.01)*

(52) Cooperative Patent Classification (CPC):
**C07K 1/32; C07K 5/12**

(86) International application number:
**PCT/JP2023/047127**

(87) International publication number:
**WO 2024/143514 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022212468**

(71) Applicant: **CHUGAI SEIYAKU KABUSHIKI KAISHA**
**Tokyo 115-8543 (JP)**

(72) Inventors:
- **TAMIYA Minoru**
  **Yokohama City, Kanagawa 244-8602 (JP)**
- **NII Keiji**
  **Yokohama City, Kanagawa 244-8602 (JP)**
- **IIKURA Hitoshi**
  **Yokohama City, Kanagawa 244-8602 (JP)**
- **EBIHARA Akira**
  **Yokohama City, Kanagawa 244-8602 (JP)**
- **INOMATA Yuuki**
  **Yokohama City, Kanagawa 244-8602 (JP)**

(74) Representative: **Vossius & Partner**
**Patentanwälte Rechtsanwälte mbB**
**Siebertstrasse 3**
**81675 München (DE)**

# (54) PURIFICATION METHOD AND PRODUCTION METHOD OF CYCLIC PEPTIDE

(57) A purification method of a cyclic peptide, the purification method comprising the following separation step (i) or (ii):
(i) separating a cyclic peptide that is a purification target from a mixture containing the cyclic peptide that is a purification target as a composite with a metal atom or a metal ion, or
(ii) separating a cyclic peptide that is a purification target or a peptide that is an impurity from a mixture containing the cyclic peptide that is a purification target and the peptide that is an impurity as a composite with a metal atom or a metal ion, wherein
the metal atom or metal ion is at least one selected from the group consisting of an alkali metal atom or ion, an alkaline earth metal atom or ion, a transition metal atom or ion, a poor metal atom or ion, and a rare earth metal atom or ion.

EP 4 631 955 A1

## Description

[Technical Field]

The present invention relates to a purification method of a cyclic peptide and a production method of a cyclic peptide using the same.

[Background Art]

Conventionally, purification by column chromatography (Non Patent Literature 1) has been known as a method for purifying a cyclic peptide. On the other hand, Patent Literature 1 describes that "isolating and purifying cyclic peptide compounds produced without isolating and purifying intermediates by crystallization enables to provide crystals of cyclic peptide compounds without using column chromatography".

[Citation List]

[Patent Literature]

[Patent Literature 1] International Publication No. WO 2022/234864

[Non Patent Literature]

[Non Patent Literature 1] K. Nomura et al., "Broadly Applicable and Comprehensive Synthetic Method for N-Alkyl-Rich Drug-like Cyclic Peptides", J. Med. Chem., 2022, 65, 19, 13401-13412

[Summary of Invention]

[Technical Problem]

However, purification by column chromatography tends to be inefficient, especially for peptide synthesis on industrial scales. In addition, many cyclic peptides have physical properties of difficulty in crystallization, and there are some cyclic peptides that are difficult to be purified by crystallization.

An object of the present invention is to provide a method for easily purifying and producing a cyclic peptide, where the method can be applied to cyclic peptides that are difficult to crystallize.

[Solution to Problem]

The present invention relates to, for example, each of the following inventions [1] to [150].

[1] A purification method of a cyclic peptide,

the purification method comprising the following separation step (i) or (ii):

(i) separating a cyclic peptide that is a purification target from a mixture containing the cyclic peptide that is a purification target as a composite with a metal atom or a metal ion, or
(ii) separating a cyclic peptide that is a purification target or a peptide that is an impurity from a mixture containing the cyclic peptide that is a purification target and the peptide that is an impurity as a composite with a metal atom or a metal ion, wherein

the metal atom is at least one selected from the group consisting of an alkali metal atom, an alkaline earth metal atom, a transition metal atom, a poor metal atom, and a rare earth metal atom, and
the metal ion is at least one selected from the group consisting of an alkali metal ion, an alkaline earth metal ion, a transition metal ion, a poor metal ion, and a rare earth metal ion.

[2] The purification method according to [1], comprising a step (1) of mixing the mixture containing the cyclic peptide that is a purification target or the mixture containing the cyclic peptide that is a purification target and the peptide that is an impurity with the metal atom or the metal ion as a pre-step of the separation step.
[3] The purification method according to [2], wherein the step (1) is performed in a first solvent.

[4] A purification method of a cyclic peptide, comprising

a step (1)' of mixing, in a first solvent, a mixture containing a cyclic peptide that is a purification target or a mixture containing a cyclic peptide that is a purification target and a peptide that is an impurity with a metal atom or a metal ion, wherein
the metal atom is at least one selected from the group consisting of an alkali metal atom, an alkaline earth metal atom, a transition metal atom, a poor metal atom, and a rare earth metal atom, and
the metal ion is at least one selected from the group consisting of an alkali metal ion, an alkaline earth metal ion, a transition metal ion, a poor metal ion, and a rare earth metal ion.

[5] The purification method according to [4], wherein in the step (1)', a composite of the cyclic peptide that is a purification target or the peptide that is an impurity with the metal atom or the metal ion is formed in the first solvent.
[6] The purification method according to any one of [3] to [5], further comprising a step (2) of mixing a mixture obtained in the step (1) or the step (1)' with a second solvent, wherein
the first solvent and the second solvent are different solvents from each other.
[7] The purification method according to [6], comprising a step (2)' of removing at least a portion of the first solvent as a pre-step of the step (2).
[8] The purification method according to any one of [2] to [7], wherein in the (1) step or the (1)' step, the metal atom or the metal ion is 0.2 molar equivalents to 12 molar equivalents with respect to the cyclic peptide that is a purification target.
[9] The purification method according to any one of [2] to [8], wherein in the (1) step or the (1)' step, the metal atom or the metal ion is 0.5 molar equivalents to 4 molar equivalents with respect to the cyclic peptide that is a purification target.
[10] The purification method according to any one of [2] to [9], wherein in the (1) step or the (1)' step, the metal atom or the metal ion is 0.8 molar equivalents to 1.2 molar equivalents with respect to the cyclic peptide that is a purification target.
[10-1] The purification method according to any one of [2] to [9], wherein in the (1) step or the (1)' step, the cyclic peptide that is a purification target is 0.1 mg/mL to 1000 mg/mL with respect to the first solvent.
[10-2] The purification method according to any one of [2] to [9], wherein in the (1) step or the (1)' step, the cyclic peptide that is a purification target is 1.0 mg/mL to 500 mg/mL with respect to the first solvent.
[10-3] The purification method according to any one of [2] to [9], wherein in the (1) step or the (1)' step, the cyclic peptide that is a purification target is 2.7 mg/mL to 150 mg/mL with respect to the first solvent.
[11] The purification method according to any one of [6] to [10], wherein, in the step (2), a mixture obtained in the step (1) or the step (1)' is mixed with the second solvent at a liquid temperature of -20 to 100°C.
[12] The purification method according to any one of [6] to [11], wherein, in the step (2), a mixture obtained in the step (1) or the step (1)' is mixed with the second solvent at a liquid temperature of 20 to 30°C.
[13] The purification method according to any one of [6] to [12], wherein, in the step (2), a mixture obtained in the step (1) or the step (1)' is mixed with the second solvent for 0.5 to 72 hours.
[14] The purification method according to any one of [6] to [13], wherein, in the step (2), a mixture obtained in the step (1) or the step (1)' is mixed with the second solvent for 1 to 2 hours.
[15] The purification method according to any one of [6] to [14], wherein, in the step (2), a concentration of the composite in the second solvent is 0.1 mg/mL to 200 mg/mL.
[15-1] The purification method according to any one of [6] to [14], wherein, in the step (2), a concentration of the composite in the second solvent is 0.5 mg/mL to 50 mg/mL.
[16] The purification method according to any one of [6] to [15], wherein, in the step (2), a concentration of the composite in the second solvent is 1.0 mg/mL to 30 mg/mL.
[17] The purification method according to any one of [6] to [16], further comprising a step (3) of separating the composite from a mixed solution of a mixture obtained in the step (1) or the step (1)' and the second solvent.
[18] A purification method of a cyclic peptide, comprising

a step (4) of mixing a composite of a cyclic peptide that is a purification target or a peptide that is an impurity with a metal atom or a metal ion and a second solvent, wherein
the metal atom is at least one selected from the group consisting of an alkali metal atom, an alkaline earth metal atom, a transition metal atom, a poor metal atom, and a rare earth metal atom, and
the metal ion is at least one selected from the group consisting of an alkali metal ion, an alkaline earth metal ion, a transition metal ion, a poor metal ion, and a rare earth metal ion.

[19] The purification method according to [18], further comprising a step (5) of separating the composite from a mixed

solution of the composite and the second solvent.

[20] The purification method according to [1], [2], [3], [17] or [19], wherein the separation of the composite is performed by solid-liquid separation or liquid-liquid partition.

[20-1] The purification method according to [1], [2], [3], [17] or [19], wherein the separation of the composite is performed by solid-liquid separation.

[20-2] The purification method according to [1], [2], [3], [17] or [19], wherein the separation of the composite is performed by liquid-liquid partition.

[21] The purification method according to [20], wherein the solid-liquid separation is centrifugation or filtration.

[22] The purification method according to [1], [2], [3], [17], [19], [20] or [21], further comprising a step (6) of removing the metal atom or the metal ion from the separated composite.

[23] The purification method according to [22], wherein the step (6) comprises a step of mixing the separated composite, a third solvent and water and separating them into an aqueous phase and an organic phase to remove the metal atom or the metal ion present in the aqueous phase.

[24] The purification method according to [23], wherein the step (6) further comprises a step of removing the third solvent from the organic phase and obtaining the cyclic peptide that is a purification target.

[25] The purification method according to [24], wherein the removing the third solvent from the organic phase is performed by distillation under reduced pressure.

[26] The purification method according to [22], wherein the step (6) further comprises a step of mixing the separated composite, a fourth solvent, and a ligand or a compound that generates a ligand or an anion or a compound that generates an anion, and forming a first complex or a first metal salt of the metal atom or the metal ion with the ligand or the anion.

[27] The method according to [26], wherein the step (6) further comprises a step of removing the first complex or the first metal salt.

[28] The purification method according to any one of [1] to [4] and [6] to [27], further comprising a step (7) of confirming that the cyclic peptide that is a purification target or the peptide that is an impurity and the metal atom or the metal ion form the composite.

[29] The purification method according to [28], wherein the step (7) is performed by comparing an NMR peak of the cyclic peptide that is a purification target or the peptide that is an impurity to an NMR peak of a mixture obtained by mixing the cyclic peptide that is a purification target or the peptide that is an impurity and the metal atom or the metal ion.

[30] The purification method according to any one of [1] to [4] and [6] to [29], wherein the composite is a composite of the cyclic peptide that is a purification target with the metal atom or the metal ion.

[31] The purification method according to any one of [1] to [4] and [6] to [29], wherein the composite is a composite of the peptide that is an impurity with the metal atom or the metal ion.

[32] The purification method according to any one of [1] to [4] and [6] to [31], wherein the composite is a composite obtained by contacting the cyclic peptide that is a purification target or the peptide that is an impurity with the metal atom or the metal ion.

[33] The purification method according to any one of [1] to [4] and [6] to [32], wherein the composite is a complex.

[34] The purification method according to [33], wherein the complex comprises a solid, a crystal, a liquid, or an amorphous.

[35] The purification method according to [33] or [34], wherein the complex comprises a crystal.

[36] The purification method according to [33] or [34], wherein the complex comprises an amorphous.

[37] The purification method according to any one of [1] to [17], wherein the mixture containing the cyclic peptide that is a purification target or the mixture containing a cyclic peptide that is a purification target and a peptide that is an impurity is a crude product obtained by producing the cyclic peptide that is a purification target.

[38] The method according to any one of [1] to [37], wherein the number of amino acid residues of the cyclic peptide that is a purification target is 5 to 20.

[39] The method according to any one of [1] to [38], wherein the number of amino acid residues of the cyclic peptide that is a purification target is 10 to 14.

[40] The purification method according to any one of [1] to [39], wherein the number of amino acid residues of a cyclic moiety of the cyclic peptide that is a purification target is 5 to 15.

[41] The purification method according to any one of [1] to [40], wherein the number of amino acid residues of a cyclic moiety of the cyclic peptide that is a purification target is 8 to 14.

[42] The purification method according to any one of [1] to [41], wherein the number of amino acid residues of a cyclic moiety of the cyclic peptide that is a purification target is 8, 11, 13, or 14.

[43] The purification method according to any one of [1] to [42], wherein the number of amino acid residues of a cyclic moiety of the cyclic peptide that is a purification target is 11.

[44] The purification method according to any one of [1] to [43], wherein the cyclic peptide that is a purification target

comprises a non-natural amino acid.

[44-1] The purification method according to any one of [1] to [43], wherein the cyclic peptide that is a purification target comprises an N-substituted amino acid.

[45] The purification method according to any one of [1] to [44], wherein the cyclic peptide that is a purification target comprises three or more N-substituted amino acids.

[46] The purification method according to any one of [1] to [45], wherein the cyclic peptide that is a purification target comprises five or more N-substituted amino acids.

[47] The purification method according to any one of [1] to [46], wherein the cyclic peptide that is a purification target comprises six or more N-substituted amino acids.

[48] The purification method according to any one of [1] to [47], wherein the number of N-substituted amino acid residues of the cyclic peptide that is a purification target is 45% or more with respect to the number of amino acid residues of the cyclic moiety of the cyclic peptide.

[49] The purification method according to any one of [44] to [48], wherein the N-substituted amino acid is at least one selected from the group consisting of N-methylamino acid, N-ethylamino acid, and N-propylamino acid.

[50] The purification method according to any one of [44] to [49], wherein the N-substituted amino acid is an N-methylamino acid.

[51] The purification method according to any one of [1] to [50], wherein the cyclic peptide that is a purification target comprises an N-unsubstituted amino acid.

[52] The purification method according to [51], wherein the N-unsubstituted amino acid is a non-natural amino acid.

[53] The purification method according to [50] or [51], wherein a proportion of the number of N-unsubstituted amino acids in the cyclic peptide that is a purification target is 55% or less with respect to a total number of the amino acid residues in the cyclic peptide that is a purification target.

[54] The purification method according to any one of [1] to [53], wherein a ClogP/the number of amino acid residues of the cyclic peptide that is a purification target is 1.0 or more and 1.8 or less.

[55] The purification method according to any one of [1] to [54], wherein the peptide that is an impurity is a peptide generated in a synthesis process of the cyclic peptide that is a purification target.

[56] The purification method according to any one of [1] to [55], wherein the peptide that is an impurity is a cyclic peptide that is different from the cyclic peptide that is a purification target.

[57] The purification method according to any one of [1] to [56], wherein the peptide that is an impurity is a cyclic peptide having the number of amino acids twice the number of amino acids of the cyclic peptide that is a purification target, a cyclic peptide having the number of amino acids three times the number of amino acids of the cyclic peptide that is a purification target, or an isomer of the cyclic peptide that is a purification target.

[58] The purification method according to any one of [1] to [57], wherein the metal atom or the metal ion is an atom or ion of an alkali metal.

[59] The purification method according to [58], wherein the alkali metal is at least one selected from the group consisting of lithium, sodium, potassium, rubidium, and cesium.

[60] The purification method according to [58] or [59], wherein the alkali metal is lithium or potassium.

[61] The purification method according to any one of [1] to [57], wherein the metal atom or the metal ion is an atom or ion of an alkaline earth metal.

[62] The purification method according to [61], wherein the alkaline earth metal is at least one selected from the group consisting of magnesium, calcium, strontium, and barium.

[63] The purification method according to [60] or [61], wherein the alkaline earth metal is at least one selected from the group consisting of magnesium, calcium, and barium.

[64] The purification method according to any one of [1] to [57], wherein the metal atom or the metal ion is an atom or ion of a transition metal.

[65] The purification method according to [64], wherein the transition metal is at least one selected from the group consisting of scandium, manganese, iron, zinc, and tungsten.

[66] The purification method according to [64] or [65], wherein the transition metal is at least one selected from the group consisting of scandium, manganese, and zinc.

[67] The purification method according to any one of [1] to [57], wherein the metal atom or the metal ion is an atom or ion of a rare earth metal.

[68] The purification method according to [67], wherein the rare earth metal is at least one selected from the group consisting of cerium, samarium, and ytterbium.

[69] The purification method according to any one of [1] to [57], wherein the metal atom or the metal ion is an atom or ion of a poor metal.

[70] The purification method according to [69], wherein the poor metal is at least one selected from the group consisting of bismuth and indium.

[71] The purification method according to any one of [1] to [57], wherein the metal atom is at least one selected from the

group consisting of a magnesium atom, a scandium atom, and a samarium atom; and
the metal ion is at least one selected from the group consisting of a magnesium ion, a scandium ion, and a samarium ion.

[72] The purification method according to any one of [1] to [57], wherein the metal atom comprises a magnesium atom, and
the metal ion comprises a magnesium ion.

[73] The purification method according to any one of [1] to [72], wherein the metal atom or the metal ion is an atom or ion derived from a metal salt or a solvate thereof or a second complex.

[74] The purification method according to any one of [1] to [73], wherein the metal atom or the metal ion is an atom or ion derived from a metal salt or a solvate thereof.

[75] The purification method according to [73] or [74], wherein the metal salt is at least one selected from the group consisting of an iodide salt, a bromide salt, a chloride salt, a perchlorate salt, an oxide salt, a trifluoromethanesulfonate salt, a toluenesulfonate salt, an isopropyl sulfonate salt, a methanesulfonate salt, a carbonate salt, an acetate salt, a bis(trifluoromethanesulfonate)imide salt, an ethylmalonate salt, a nitrite salt, and a sulfate salt.

[75-1] The purification method according to [73] or [74], wherein the metal salt is at least one selected from the group consisting of an iodide salt, a bromide salt, a chloride salt, a perchlorate salt, an oxide salt, a trifluoromethanesulfonate salt, a toluenesulfonate salt, an isopropyl sulfonate salt, a methanesulfonate salt, a carbonate salt, an acetate salt, a bis(trifluoromethanesulfonate)imide salt, an ethylmalonate salt, and a nitrite salt.

[76] The purification method according to any one of [73] to [75], wherein the metal salt is at least one selected from the group consisting of an iodide salt, a bromide salt, a perchlorate salt, and a trifluoromethanesulfonate salt.

[77] The purification method according to any one of [73] to [76], wherein the metal salt is a perchlorate salt or a trifluoromethanesulfonate salt.

[78] The purification method according to any one of [73] to [76], wherein the metal salt is an iodide salt.

[79] The purification method according to [78], wherein the iodide salt is at least one selected from the group consisting of lithium iodide, potassium iodide, barium iodide, magnesium iodide, calcium iodide, strontium iodide, samarium(III) iodide, zinc iodide, and indium iodide.

[80] The purification method according to any one of [73] to [76], wherein the metal salt is a bromide salt.

[81] The purification method according to [80], wherein the bromide salt is at least one selected from the group consisting of lithium bromide, magnesium bromide, calcium bromide, samarium(III) bromide, zinc bromide, and indium bromide.

[82] The purification method according to any one of [73] to [75], wherein the metal salt is a chloride salt.

[83] The purification method according to [82], wherein the chloride salt is samarium chloride, cerium chloride, or magnesium chloride.

[83-1] The purification method according to [82], wherein the chloride salt is samarium chloride or cerium chloride.

[84] The purification method according to any one of [73] to [77], wherein the metal salt is a perchlorate salt.

[85] The purification method according to [84], wherein the perchlorate salt is at least one selected from the group consisting of lithium perchlorate, barium perchlorate, magnesium perchlorate, calcium perchlorate, and zinc perchlorate.

[86] The purification method according to any one of [73] to [75], wherein the metal salt is an oxide salt.

[87] The purification method according to [86], wherein the oxide salt is magnesium oxide.

[88] The purification method according to any one of [73] to [77], wherein the metal salt is a trifluoromethanesulfonate salt.

[89] The purification method according to [88], wherein the trifluoromethanesulfonate salt is at least one selected from the group consisting of magnesium trifluoromethanesulfonate, calcium trifluoromethanesulfonate, scandium trifluoromethanesulfonate, samarium(III) trifluoromethanesulfonate, cerium trifluoromethanesulfonate, ytterbium trifluoromethanesulfonate, zinc trifluoromethanesulfonate, manganese trifluoromethanesulfonate, indium trifluoromethanesulfonate, iron(III) trifluoromethanesulfonate, and copper trifluoromethanesulfonate.

[90] The purification method according to any one of [73] to [75], wherein the metal salt is a toluenesulfonate salt.

[91] The purification method according to [90], wherein the toluenesulfonate salt is zinc(II) toluenesulfonate.

[92] The purification method according to any one of [73] to [75], wherein the metal salt is an isopropyl sulfonate salt.

[93] The purification method according to [92], wherein the isopropyl sulfonate salt is zinc(II) isopropyl sulfonate.

[94] The purification method according to any one of [73] to [75], wherein the metal salt is a methanesulfonate salt.

[95] The purification method according to [94], wherein the methanesulfonate salt is at least one selected from the group consisting of cerium(III) methanesulfonate and zinc difluoromethanesulfonate.

[96] The purification method according to any one of [73] to [75], wherein the metal salt is a carbonate salt.

[97] The purification method according to [96], wherein the carbonate salt is at least one selected from the group consisting of potassium carbonate, calcium carbonate, and zinc carbonate.

[98] The purification method according to any one of [73] to [75], wherein the metal salt is an acetate salt.

[99] The purification method according to [98], wherein the acetate salt is at least one selected from the group consisting of potassium acetate and magnesium acetate.

[100] The purification method according to any one of [73] to [75], wherein the metal salt is a bis(trifluoromethanesulfonate)imide salt.

[101] The purification method according to [100], wherein the bis(trifluoromethanesulfonate)imide salt is at least one selected from the group consisting of magnesium bis(trifluoromethanesulfonate)imide, zinc bis(trifluoromethanesulfonate)imide, and iron(II) bis(trifluoromethanesulfonate)imide.

[102] The purification method according to any one of [73] to [75], wherein the metal salt is an ethylmalonate salt.

[103] The purification method according to [102], wherein the ethylmalonate salt is magnesium ethylmalonate.

[104] The purification method according to any one of [73] to [75], wherein the metal salt is a nitrite salt.

[105] The purification method according to [104], wherein the nitrite salt is potassium nitrite.

[105-1] The purification method according to any one of [73] to [75], wherein the metal salt is a sulfate salt.

[105-2] The purification method according to any one of [105-1], wherein the sulfate salt is magnesium sulfate.

[106] The purification method according to any one of [1] to [74], wherein the metal atom or the metal ion is an atom or an ion derived from at least one selected from the group consisting of lithium iodide, lithium perchlorate, lithium tetrafluoroborate, lithium bromide, lithium chloride, potassium iodide, lithium fluoride, potassium carbonate, potassium nitrite, potassium acetate, potassium tetrafluoroborate, potassium hexafluorophosphate, barium iodide, barium perchlorate, magnesium ethylmalonate, magnesium bis(trifluoromethanesulfonic acid) imide, magnesium oxide, magnesium bromide, magnesium perchlorate, magnesium iodide, magnesium trifluoromethanesulfonate, magnesium sulfate, magnesium acetate, magnesium chloride, magnesium fluoride, calcium iodide, calcium perchlorate, calcium bromide, calcium trifluoromethanesulfonate, calcium carbonate, strontium iodide, scandium trifluoromethanesulfonate, scandium chloride, samarium(III) trifluoromethanesulfonate, samarium(III) iodide, samarium(III) bromide, samarium(III) chloride, cerium(III) methanesulfonate, cerium(III) chloride, cerium(III) chloride heptahydrate, cerium trifluoromethanesulfonate, ytterbium trifluoromethanesulfonate, zinc trifluoromethanesulfonate, zinc(II) tetrafluoroborate, zinc perchlorate, zinc difluoromethanesulfonate, zinc(II) isopropyl sulfonate, zinc(II) toluenesulfonate, zinc bis(trifluoromethanesulfonic acid) imide, zinc bromide, zinc chloride, zinc carbonate, zinc fluoride, zinc iodide, manganese trifluoromethanesulfonate, manganese chloride, indium trifluoromethanesulfonate, indium bromide, indium chloride, indium iodide, iron(III) trifluoromethanesulfonate, iron(II) bis(trifluoromethanesulfonic acid) imide, tungsten(VI) fluoride, tungsten(VI) chloride, and copper trifluoromethanesulfonate.

[107] The purification method according to any one of [1] to [74] and [106], wherein the metal atom or the metal ion is an atom or an ion derived from at least one metal salt or a solvate thereof selected from the group consisting of lithium iodide, lithium perchlorate, potassium iodide, barium iodide, barium perchlorate, magnesium bromide, magnesium perchlorate, magnesium iodide, magnesium trifluoromethanesulfonate, calcium bromide, scandium trifluoromethanesulfonate, samarium trifluoromethanesulfonate, cerium trifluoromethanesulfonate, ytterbium trifluoromethanesulfonate, zinc trifluoromethanesulfonate, manganese trifluoromethanesulfonate, indium trifluoromethanesulfonate, magnesium sulfate, magnesium acetate, and magnesium chloride.

[107-1] The purification method according to any one of [1] to [74] and [106], wherein the metal atom or the metal ion is an atom or an ion derived from at least one metal salt or a solvate thereof selected from the group consisting of lithium iodide, lithium perchlorate, potassium iodide, barium iodide, barium perchlorate, magnesium bromide, magnesium perchlorate, magnesium iodide, magnesium trifluoromethanesulfonate, calcium bromide, scandium trifluoromethanesulfonate, samarium trifluoromethanesulfonate, cerium trifluoromethanesulfonate, ytterbium trifluoromethanesulfonate, zinc trifluoromethanesulfonate, manganese trifluoromethanesulfonate, and indium trifluoromethanesulfonate.

[108] The purification method according to any one of [1] to [74], [106] and [107], wherein the metal atom or the metal ion is an atom or an ion derived from at least one metal salt or a solvate thereof selected from the group consisting of lithium perchlorate, potassium iodide, magnesium bromide, magnesium perchlorate, magnesium iodide, and manganese trifluoromethanesulfonate.

[109] The purification method according to any one of [1] to [73], wherein the metal atom or the metal ion is an atom or ion derived from the second complex.

[110] The purification method according to [73] or [109], wherein a ligand of the second complex is at least one selected from the group consisting of an olefinic compound, a carbonyl-based compound, a phosphine-based compound, and carbon monoxide.

[111] The purification method according to [73], [109] or [110], wherein a ligand of the second complex is at least one selected from the group consisting of ethylene, dibenzylidene acetone, acetyl acetonate, triphenylphosphine and carbon monoxide.

[112] The purification method according to any one of [73] and [109] to [111], wherein the second complex is at least one selected from the group consisting of iron(III) tris (acetylacetonate), ferrocene, zinc(II) acetylacetonate, and tungsten hexacarbonyl.

[113] The purification method according to any one of [3] to [17] and [20] to [112], wherein the first solvent is a solvent capable of forming a composite of the cyclic peptide that is a purification target or the peptide that is an impurity with the metal atom or the metal ion.

[113-1] The purification method according to any one of [3] to [17] and [20] to [112], wherein the first solvent includes at least one selected from the group consisting of an alcohol-based solvent, a nitrile-based solvent, a benzene-based solvent, an ether-based solvent, a ketone-based solvent, a halogen-based solvent, an ester-based solvent, a sulfoxide-based solvent, and an amide-based solvent.

[113-2] The purification method according to any one of [3] to [17] and [20] to [112], wherein the first solvent includes at least one selected from the group consisting of an alcohol-based solvent, a nitrile-based solvent, a benzene-based solvent, an ether-based solvent, a ketone-based solvent, a halogen-based solvent, and an ester-based solvent.

[114] The purification method according to any one of [3] to [17] and [20] to [113], wherein the first solvent is an alcohol-based solvent.

[115] The purification method according to any one of [113-1], [113-2], and [114], wherein the alcohol-based solvent is at least one selected from the group consisting of 2-propanol, tert-butanol, 2,2,2-trifluoroethanol, ethanol, and methanol.

[115-1] The purification method according to any one of [113-1], [113-2], and [114], wherein the alcohol-based solvent is at least one selected from the group consisting of 2-propanol, tert-butanol, and 2,2,2-trifluoroethanol.

[116] The purification method according to any one of [3] to [17] and [20] to [113], wherein the first solvent is a nitrile-based solvent.

[117] The purification method according to [116], wherein the nitrile-based solvent is acetonitrile.

[118] The purification method according to any one of [3] to [17] and [20] to [113], wherein the first solvent is a benzene-based solvent.

[119] The purification method according to [118], wherein the benzene-based solvent is toluene or xylene.

[120] The purification method according to any one of [3] to [17] and [20] to [113], wherein the first solvent is an ether-based solvent.

[121] The purification method according to [120], wherein the ether-based solvent is at least one selected from the group consisting of tetrahydrofuran, 1,4-dioxane, 2-methyltetrahydrofuran, MTBE (tert-butyl methyl ether), DME (dimethyl ether) and CPME (cyclopentyl pentyl ether).

[122] The purification method according to any one of [3] to [17] and [20] to [113], wherein the first solvent is a ketone-based solvent.

[123] The purification method according to [122], wherein the ketone-based solvent is acetone or methyl ethyl ketone.

[124] The purification method according to any one of [3] to [17] and [20] to [113], wherein the first solvent is a halogen-based solvent.

[125] The purification method according to [124], wherein the halogen-based solvent is dichloromethane.

[125-1] The purification method according to any one of [3] to [17] and [20] to [113], wherein the first solvent is a sulfoxide-based solvent.

[125-2] The purification method according to [125-1], wherein the sulfoxide-based solvent is dimethyl sulfoxide.

[125-3] The purification method according to any one of [3] to [17] and [20] to [113], wherein the first solvent is an amide-based solvent.

[125-4] The purification method according to [125-3], wherein the amide-based solvent is dimethylformamide or dimethylacetamide. [126]

The purification method according to any one of [3] to [17] and [20] to [113], wherein the first solvent is at least one selected from the group consisting of 2-propanol, tert-butanol, 2,2,2-trifluoroethanol, acetonitrile, tetrahydrofuran, toluene, 1,4-dioxane, acetone, dichloromethane, ethanol, methanol, dimethyl sulfoxide, dimethylformamide and dimethyl acetamide.

[126-1] The purification method according to any one of [3] to [17] and [20] to [113], wherein the first solvent is at least one selected from the group consisting of 2-propanol, tert-butanol, 2,2,2-trifluoroethanol, acetonitrile, tetrahydrofuran, toluene, 1,4-dioxane, acetone, and dichloromethane.

[127] The purification method according to any one of [3] to [17], [20] to [113] and [126], wherein the first solvent is at least one selected from the group consisting of 2,2,2-trifluoroethanol, acetonitrile, tetrahydrofuran, and acetone.

[128] The purification method according to any one of [6] to [127], wherein the second solvent comprises at least one selected from the group consisting of an ether-based solvent, a benzene-based solvent, a ketone-based solvent, a halogen-based solvent, an ester-based solvent, a nitrile-based solvent, a hydrocarbon-based solvent, an alcohol-based solvent, a sulfoxide-based solvent, and an amide-based solvent.

[128-1] The purification method according to any one of [6] to [127], wherein the second solvent comprises at least one selected from the group consisting of an ether-based solvent, a benzene-based solvent, a ketone-based solvent, a halogen-based solvent, an ester-based solvent, a nitrile-based solvent, and a hydrocarbon-based solvent.

[129] The purification method according to any one of [6] to [128], wherein the second solvent is an ether-based

solvent.

[130] The purification method according to [129], wherein the ether-based solvent is at least one selected from the group consisting of 2-methyltetrahydrofuran, tetrahydrofuran, 1,4-dioxane, MTBE (tert-butyl methyl ether), DME (dimethyl ether) and CPME (cyclopentyl methyl ether).

[131] The purification method according to any one of [6] to [128], wherein the second solvent is a benzene-based solvent.

[132] The purification method according to [128] or [131], wherein the benzene-based solvent is toluene or xylene.

[133] The purification method according to any one of [6] to [128], wherein the second solvent is a ketone-based solvent.

[134] The purification method according to [128] or [133], wherein the ketone-based solvent is acetone or methyl ethyl ketone.

[135] The purification method according to any one of [6] to [128], wherein the second solvent is a halogen-based solvent.

[136] The purification method according to [128] or [135], wherein the halogen-based solvent is dichloromethane.

[137] The purification method according to any one of [6] to [128], wherein the second solvent is a hydrocarbon-based solvent.

[138] The purification method according to [128] or [137], wherein the hydrocarbon-based solvent is at least one selected from the group consisting of hexane, heptane, cyclohexane, methylcyclohexane, or isooctane.

[139] The purification method according to any one of [6] to [128], wherein the second solvent is an ester-based solvent.

[139-1] The purification method according to [128] or [139], wherein the ester-based solvent is isopropyl acetate or ethyl acetate.

[140] The purification method according to any one of [6] to [128], wherein the second solvent is a nitrile-based solvent.

[140-1] The purification method according to [128] or [140], wherein the nitrile-based solvent is acetonitrile.

[140-2] The purification method according to any one of [6] to [128], wherein the second solvent is an alcohol-based solvent.

[140-3] The purification method according to [128] or [140-2], wherein the alcohol-based solvent is at least one selected from the group consisting of methanol, ethanol, butanol, or benzyl alcohol.

[140-4] The purification method according to any one of [6] to [128], wherein the second solvent is a sulfoxide-based solvent.

[140-5] The purification method according to [140-4], wherein the sulfoxide-based solvent is dimethyl sulfoxide.

[140-6] The purification method according to any one of [6] to [128], wherein the second solvent is an amide-based solvent.

[140-7] The purification method according to [140-6], wherein the amide-based solvent is dimethylformamide.

[140-8] The purification method according to any one of [3] to [17] and [20] to [113], wherein the second solvent is a combination of isopropyl acetate or methyl ethyl ketone and heptane, or a combination of acetonitrile and MTBE.

[141] The purification method according to any one of [23] to [140], wherein the third solvent is a solvent that is not miscible with water.

[142] The purification method according to any one of [23] to [141], wherein the third solvent is at least one selected from the group consisting of 2-methyltetrahydrofuran, tetrahydrofuran, dimethyl carbonate, anisole, isopropyl acetate, ethyl acetate, MTBE (tert-butyl methyl ether), diethyl ether, dichloromethane, chloroform, DME (dimethyl ether), CPME (cyclopentyl methyl ether), 4-methyltetrahydropyran, heptane, and toluene.

[143] The purification method according to any one of [26] to [142], wherein the fourth solvent is at least one selected from the group consisting of methanol, ethanol, 1-propanol, 2-propanol, 1-butanol, 2-butanol, t-butanol, ethylene glycol, cyclohexane, methylcyclohexane, heptane, isooctane, toluene, xylene, dimethyl sulfoxide, and acetonitrile.

[144] The purification method according to any one of [1] to [143], wherein the purification method is a method for purifying the cyclic peptide that is a purification target to have a purity of 85% or more, wherein the purity(%) of the cyclic peptide that is a purification target is a proportion of the peak area of the cyclic peptide that is a purification target with respect to the sum of each peak area of the entire mixture containing the cyclic peptide that is a purification target at 220 nm in which UV spectrum of the mixture containing the cyclic peptide that is a purification target is measured using HPLC and PDA (photodiode array detector).

[144-1] The purification method according to any one of [1] to [143], wherein the purification method is a method for purifying a cyclic peptide that is a purification target to have a purity of 85% or more, wherein the purity(%) of the cyclic peptide that is a purification target is a proportion of the peak area of the cyclic peptide that is a purification target with respect to the sum of each peak area of the entire mixture containing the cyclic peptide that is a purification target at 225 nm in which UV spectrum of the mixture containing the cyclic peptide that is a purification target is measured using HPLC and PDA (photodiode array detector).

[144-2] The purification method according to any one of [1] to [143], wherein a purification efficiency of an impurity

contained in the mixture containing the cyclic peptide that is a purification target is a positive value.

[144-3] The method according to [144-2], wherein the purification efficiency of the impurity is determined by the following formula:

purification efficiency of the impurity (%) = {1 - (ratio of cyclic peptide that is a purification target to impurity after purification operation) / (ratio of cyclic peptide that is a purification target to impurity before purification operation)} $\times$ 100.

[145] A production method of a cyclic peptide, comprising the purification method according to any one of [1] to [144].

[146] The production method according to [145], further comprising a step of obtaining the cyclic peptide that is a purification target by a liquid phase synthesis method.

[147] The production method according to [145], further comprising a step of obtaining the cyclic peptide that is a purification target by a solid phase synthesis method.

[148] The production method according to [145], further comprising a step of obtaining the cyclic peptide that is a purification target by a culture method.

[149] A method of improving a purity of a cyclic peptide as compared to not forming a composite with a metal atom or a metal ion in a purification method of the cyclic peptide, characterized in that the cyclic peptide that is a purification target is separated from a mixture containing the cyclic peptide that is a purification target as a composite with a metal atom or a metal ion.

[150] A method of reducing a content of an impurity as compared to not forming a composite with a metal atom or a metal ion in a purification method of the cyclic peptide, characterized in that the cyclic peptide that is a purification target is separated from a mixture containing the cyclic peptide that is a purification target as a composite with a metal atom or a metal ion.

In the above numberings, the number cited in the dependent item includes the same number but with a different branch number, unless otherwise mentioned. For example, [20] cited in the dependent item shows that not only [20] but also [20-1] are included. The same applies to other numberings.

[Advantageous Effects of Invention]

According to the present invention, a method for easily purifying and producing a cyclic peptide can be provided, where the method can be applied to cyclic peptides that are difficult to crystallize. Some cyclic peptides are difficult to crystallize, but it has been found that even such a cyclic peptide can be easily purified by forming a composite of the cyclic peptide with a metal atom and a metal ion.

[Brief Description of Drawings]

[Figure 1] Figure 1 is a schematic diagram illustrating a basic synthesis method of a cyclic peptide.

[Figure 2] Figure 2 is a diagram showing $^1$H-NMR spectrum of RUN 1 measured in Reference Example 2.

[Figure 3] Figure 3 is a diagram showing $^1$H-NMR spectrum of RUN 2 measured in Reference Example 2.

[Figure 4] Figure 4 is a diagram showing $^1$H-NMR spectrum of RUN 3 measured in Reference Example 2.

[Figure 5] Figure 5 is a diagram showing $^1$H-NMR spectrum of RUN 4 measured in Reference Example 2.

[Figure 6] Figure 6 is a diagram showing $^1$H-NMR spectrum of RUN 5 measured in Reference Example 2.

[Figure 7] Figure 7 is a diagram showing $^1$H-NMR spectrum of RUN 6 measured in Reference Example 2.

[Figure 8] Figure 8 is a diagram showing an LC chart of RUN 29 measured in Example 2-1.

[Figure 9] Figure 9 is a diagram showing an LC chart of RUN 2 measured in Example 2-3-1.

[Figure 10] Figure 10 is a diagram showing the $^1$H-NMR spectrum measured in Example 3-1.

[Description of Embodiments]

Hereinafter, the method of the present invention will be described in detail.

In the present invention, the term "mixing" sometimes mean "operation" of mixing one substance and another substance, and does not necessarily mean making only the miscible state of one substance with another substance. The term "mixing (i) and (ii)" as used herein includes any embodiments of adding (i) to (ii), adding (ii) to (i), and adding (i) and (ii) simultaneously. As used herein, the term "to" that indicates a range includes values of both ends thereof. For example, "A

to B" means the range of A or more and B or less. The term "about" as used herein, when used in combination with a numeric value, means the range of + 1 0% and -10% of the numeric value. In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The "purification of a cyclic peptide" as used herein means, for example, increasing the proportion (purity) of the cyclic peptide that is a purification target with respect to the entire mixture containing the cyclic peptide that is a purification target.

One embodiment of the present invention relates to a purification method of a cyclic peptide, in which the purification method may comprise a step of separating a cyclic peptide that is a purification target from a mixture containing the cyclic peptide that is a purification target as a composite with a metal atom or a metal ion, or a step of separating a cyclic peptide that is a purification target or a peptide that is an impurity from a mixture containing the cyclic peptide that is a purification target and the peptide that is an impurity as a composite with a metal atom or a metal ion, wherein the metal atom may be at least one selected from the group consisting of an alkali metal atom, an alkaline earth metal atom, a transition metal atom, a poor metal atom, and a rare earth metal atom, and the metal ion may be at least one selected from the group consisting of an alkali metal ion, an alkaline earth metal ion, a transition metal ion, a poor metal ion, and a rare earth metal ion.

The separation step may comprise a step (1) of mixing the mixture containing the cyclic peptide that is a purification target or the mixture containing the cyclic peptide that is a purification target and the peptide that is an impurity with the metal atom or the metal ion, as a pre-step. The step (1) may be performed in a first solvent.

A purification method according to another embodiment of the present invention relates to a purification method of a cyclic peptide, in which the purification method may comprise a step (1)' of mixing, in a first solvent, a mixture containing a cyclic peptide that is a purification target or a mixture containing a cyclic peptide that is a purification target and a peptide that is an impurity with a metal atom or a metal ion, wherein the metal atom may be at least one selected from the group consisting of an alkali metal atom, an alkaline earth metal atom, a transition metal atom, a poor metal atom, and a rare earth metal atom, and the metal ion may be at least one selected from the group consisting of an alkali metal ion, an alkaline earth metal ion, a transition metal ion, a poor metal ion, and a rare earth metal ion.

In the step (1)', a composite of the cyclic peptide that is a purification target or the peptide that is an impurity with the metal atom or the metal ion may be formed in the first solvent.

A purification method according to another embodiment of the present invention may further comprise a step (2) of mixing a mixture obtained in the step (1) or the step (1)' with a second solvent. In a mixture obtained in the step (1) or the step (1)', a composite of the cyclic peptide that is a purification target or the peptide that is an impurity with the metal atom or the metal ion may be formed before mixing with the second solvent. a mixture obtained in the step (1) or the step (1)' is a "mixture of a mixture containing the cyclic peptide that is a purification target or a mixture containing the cyclic peptide that is a purification target and the peptide that is an impurity with the metal atom or the metal ion". The first solvent and the second solvent are different solvents from each other. For example, when the first solvent is a rich solvent for the composite, the second solvent may be a poor solvent for the composite. Conversely, when the first solvent is a poor solvent for the composite, the second solvent may be a rich solvent for the composite. The step (2) may comprise a step (2)' of removing at least a portion of the first solvent as a pre-step of the step (2).

**In** the step (1) or the step (1)', the metal atom or the metal ion may be 0.2 molar equivalents or more, 0.3 molar equivalents or more, 0.4 molar equivalents or more, 0.5 molar equivalents or more, 0.6 molar equivalents or more, 0.7 molar equivalents or more, 0.8 molar equivalents or more, 0.9 molar equivalents or more, or 1 molar equivalent or more, and 12 molar equivalents or less, 10 molar equivalents or less, 8 molar equivalents or less, 6 molar equivalents or less, 4 molar equivalents or less, 2 molar equivalents or less, or 1.2 molar equivalents or less with respect to the cyclic peptide that is a purification target. **In** the step (1) or the step (1)', the metal atom or the metal ion may be 0.2 molar equivalents to 12 molar equivalents, 0.2 molar equivalents to 4 molar equivalents, 0.2 molar equivalents to 1.2 molar equivalents, 0.5 molar equivalents to 12 molar equivalents, 0.5 molar equivalents to 4 molar equivalents, 0.5 molar equivalents to 1.2 molar equivalents, 0.8 molar equivalents to 12 molar equivalents, 0.8 molar equivalents to 4 molar equivalents, or 0.8 molar equivalents to 1.2 molar equivalents with respect to the cyclic peptide that is a purification target. **In** the step (1) or the step (1)', the metal atom or the metal ion is preferably 0.2 molar equivalents to 12 molar equivalents, more preferably 0.5 molar equivalents to 4 molar equivalents, and most preferably 0.8 molar equivalents to 1.2 molar equivalents.

**In** the step (1) or the step (1)', the liquid temperature of the first solvent may be -20°C or higher, -10°C or higher, 0°C or higher, 4°C or higher, 8°C or higher, 12°C or higher, 16°C or higher, or 20°C or higher, and may be 100°C or lower, 80°C or lower, 50°C or lower, 40°C or lower, 35°C or lower, 30°C or lower, or 25°C or lower. **In** the step (1) or the step (1)', the liquid temperature of the first solvent may be -20 to 100°C, 4 to 30°C, 20 to 40°C or 20 to 30°C. **In** the step (1) or the step (1)', the liquid temperature of the first solvent is preferably -20 to 100°C, more preferably 20 to 40°C, and most preferably 20 to 30°C.

**In** the step (1) or the step (1)', the time for mixing, in the first solvent, a mixture containing a cyclic peptide that is a

purification target or a mixture containing a cyclic peptide that is a purification target and a peptide that is an impurity with a metal atom or a metal ion may be 0.5 hours or more, 1 hour or more, 1.5 hours or more, or 2 hours or more, and may be 72 hours or less, 48 hours or less, 36 hours or less, 24 hours or less, 12 hours or less, 6 hours or less, or 5 hours or less. In the step (1) or the step (1)', the time for mixing, in the first solvent, a mixture containing a cyclic peptide that is a purification target or a mixture containing a cyclic peptide that is a purification target and a peptide that is an impurity with a metal atom or a metal ion may be 0.5 to 72 hours, 0.5 to 48 hours, 0.5 to 5 hours, 2 to 48 hours, 2 to 5 hours. In the step (1) or the step (1)', the time for mixing, in the first solvent, a mixture containing a cyclic peptide that is a purification target or a mixture containing a cyclic peptide that is a purification target and a peptide that is an impurity with a metal atom or a metal ion is preferably 0.5 to 72 hours, more preferably 0.5 to 48 hours, and most preferably 2 to 5 hours.

In the step (1) or the step (1)', the concentration of the cyclic peptide that is a purification target in the first solvent may be 0.1 mg/mL or more, 0.5 mg/mL or more, 1.0 mg/mL or more, 2.0 mg/mL or more, or 2.7 mg/mL or more, and may be 1000 mg/mL or less, 800 mg/mL or less, 500 mg/mL or less, 300 mg/mL or less, or 150 mg/mL or less. In the step (1) or the step (1)', the concentration of the cyclic peptide that is a purification target in the first solvent may be 0.1 mg/mL to 1000 mg/mL, 0.5 mg/mL to 800 mg/mL, 1.0 mg/mL to 500 mg/mL, 2.0 mg/mL to 300 mg/mL, or 2.7 mg/mL to 150 mg/mL. In the step (1) or the step (1)', the concentration of the cyclic peptide that is a purification target in the first solvent is preferably 0.1 mg/mL to 1000 mg/mL, more preferably 1.0 mg/mL to 500 mg/mL, and most preferably 2.7 mg/mL to 150 mg/mL.

In the step (2), the liquid temperature when mixing a mixture obtained in the step (1) or the step (1)' and the second solvent may be -20°C or higher, -10°C or higher, 0°C or higher, 4°C or higher, 8°C or higher, 12°C or higher, 16°C or higher, or 20°C or higher, and may be 100°C or lower, 80°C or lower, 50°C or lower, 40°C or lower, 35°C or lower, 30°C or lower, or 25°C or lower. In the step (2), the liquid temperature when mixing the residual and the second solvent may be -20 to 100°C, 4 to 30°C, 20 to 40°C or 20 to 30°C. In the step (2), the liquid temperature when mixing a mixture obtained in the step (1) or the step (1)' and the second solvent is preferably -20 to 100°C, more preferably 20 to 40°C, and most preferably 20 to 30°C.

In the step (2), the time for mixing a mixture obtained in the step (1) or the step (1)' and the second solvent may be 0.5 hours or more, 1 hour or more, 1.5 hours or more, or 2 hours or more, and may be 72 hours or less, 48 hours or less, 36 hours or less, 24 hours or less, 12 hours or less, 6 hours or less, or 5 hours or less. In the step (2), the time for mixing the residual and the second solvent may be 0.5 to 72 hours, 0.5 to 48 hours, 0.5 to 5 hours, 2 to 48 hours, or 2 to 5 hours. In the step (2), the time for mixing a mixture obtained in the step (1) or the step (1)' and the second solvent is preferably 0.5 to 72 hours, more preferably 0.5 to 48 hours, and most preferably 1 to 2 hours.

In the step (2), the concentration of the composite in the second solvent may be 0.1 mg/mL or more, 0.3 mg/mL or more, 0.5 mg/mL or more, 0.8 mg/mL or more, or 1.0 mg/mL or more, and may be 200 mg/mL or less, 100 mg/mL or less, 50 mg/mL or less, 45 mg/mL or less, or 30 mg/mL or less. In the step (2), the concentration of the composite in the second solvent may be 0.1 mg/mL to 200 mg/mL, 0.3 mg/mL to 100 mg/mL, 0.5 mg/mL to 50 mg/mL, 0.8 mg/mL to 45 mg/mL, or 1.0 mg/mL to 30 mg/mL. In the step (2), the concentration of the composite in the second solvent is preferably 0.1 mg/mL to 200 mg/mL, more preferably 0.5 mg/mL to 50 mg/mL, and most preferably 1.0 mg/mL to 30 mg/mL.

A purification method according to one embodiment of the present invention may further comprise a step (3) of separating the composite from a mixed solution of a mixture obtained in the step (1) or the step (1)' and the second solvent.

A purification method according to still another embodiment of the present invention relates to a purification method of a cyclic peptide, in which the purification method may comprise a step (4) of mixing a composite of a cyclic peptide that is a purification target or a peptide that is an impurity with a metal atom or a metal ion and a second solvent, wherein the metal atom may be at least one selected from the group consisting of an alkali metal atom, an alkaline earth metal atom, a transition metal atom, a poor metal atom, and a rare earth metal atom, and the metal ion may be at least one selected from the group consisting of an alkali metal ion, an alkaline earth metal ion, a transition metal ion, a poor metal ion, and a rare earth metal ion.

A purification method according to still another embodiment of the present invention may further comprise a step (5) of separating the composite from a mixed solution of the composite and the second solvent.

As used herein, the separation of the composite is performed by solid-liquid separation or liquid-liquid partition. Examples of the method of solid-liquid separation include centrifugation and filtration. Examples of the method of liquid-liquid partition include centrifugation and decantation.

As used herein, the purification method of a cyclic peptide may comprise a step (6) of removing the metal atom or the metal ion from the separated composite. The step (6) may comprise a step of mixing the separated composite, a third solvent and water, and separating them into an aqueous phase and an organic phase to remove the metal atom or the metal ion present in the aqueous phase. The step (6) may further comprise a step of removing the third solvent from the organic phase and obtaining the cyclic peptide that is a purification target. Specific examples of the method for removing the third solvent from the organic phase include distillation under normal pressure, distillation under heating, and distillation under reduced pressure. The removing the third solvent from the organic phase may be performed by distillation under reduced pressure.

The step (6) may further comprise a step of mixing the separated composite, a fourth solvent, and a ligand or a compound that generates a ligand or an anion or a compound that generates an anion, and forming a first complex or a first

metal salt of the metal atom or the metal ion with the ligand or the anion. The step (6) may further comprise a step of removing the first metal complex or the first metal salt. The first complex may mean a composite (complex) of a cyclic peptide that is a purification target or a peptide that is an impurity with a metal atom or a metal ion and a complex different from the second complex, and may use a complex of a cyclic peptide that is a purification target or a peptide that is an impurity with a metal atom or a metal ion and the same complex as the second complex.

As used herein, the purification method of a cyclic peptide may further comprise a step (7) of confirming that the cyclic peptide that is a purification target or the peptide that is an impurity and the metal atom or the metal ion form the composite. The step (7) may be performed by comparing an NMR peak of the cyclic peptide that is a purification target or the peptide that is an impurity to an NMR peak of a mixture obtained by mixing the cyclic peptide that is a purification target or the peptide that is an impurity with the metal atom or the metal ion. The measurement of NMR can be performed in a manner well known to those skilled in the art. For example, NMR can be measured by adding a solution in which the compound to be measured is dissolved in a solvent suitable for measuring NMR to a sample tube for NMR measurement, and setting the sample tube in a measuring device. The solvent suitable for measuring NMR is preferably a solvent capable of dissolving the compound to be measured, and may be a commercially available deuterated solvent. The measurement conditions of the NMR can be the conditions well known to those skilled in the art and the conditions described in the manual of the measuring device. The measurement conditions are not particularly limited as long as the target peak component can be measured. For example, the measurement temperature can be in the range of 273 K to 320 K, the integration time can be in the range of 1 second to 7 days, and the number of revolutions of the sample tube can be in the range of 0 to 20 Hz. Specifically, in the step (7), $^1$H-NMR of (A) a solution obtained by dissolving the cyclic peptide or the peptide that is an impurity in acetonitrile-d3 (deuteroacetonitrile, $CD_3CN$, CAS: 2206-26-0) (concentration of the cyclic peptide or the peptide that is an impurity: 1.4 mM); and (B) a solution obtained by mixing the metal atom or the metal ion and the cyclic peptide or the peptide that is an impurity in acetonitrile-d3 (deuteroacetonitrile, $CD_3CN$, CAS: 2206-26-0) so that the metal atom or the metal ion is 1 to 6 molar equivalents with respect to the cyclic peptide or the peptide that is an impurity (concentration of the cyclic peptide or the peptide that is an impurity: 1.4 mM) are measured at 278 K and 298 K, respectively. When a new peak is observed for the cyclic peptide that is a purification target or the peptide that is an impurity in the peak component of (B) compared to the peak component of (A), when a change in the peak intensity ratio is observed in the peak component of (B) compared to the peak component of (A), or when a sharpening or broadening of the peak is observed in the peak component of (B) compared to the peak component of (A), it may be determined that the cyclic peptide that is a purification target or the peptide that is an impurity and the metal atom or the metal ion form the composite.

Herein, the composite may be a composite of the cyclic peptide that is a purification target with the metal atom or the metal ion, and may be a composite of the peptide that is an impurity with the metal atom or the metal ion. The composite may be a composite obtained by contacting the metal with the cyclic peptide or the peptide that is an impurity. As used herein, the composite is a compound in which the cyclic peptide that is a purification target or the peptide that is an impurity interacts with the metal atom or the metal ion. The composite may include a solid, a crystal, a liquid, or an amorphous.

The composite may be a complex. As used herein, the complex of the cyclic peptide that is a purification target or the peptide that is an impurity with a metal atom or a metal ion is a compound in a state in which the metal atom or the metal ion is coordinated with the cyclic peptide that is a purification target or the peptide that is an impurity. The state in which the metal atom or the metal ion is coordinated with the cyclic peptide that is a purification target or the peptide that is an impurity is determined, for example, by X-ray crystal structure analysis and change in absorption spectrum (see "Complex Chemistry - Fundamentals and Recent Topics" edited by the society of basic coordination chemistry, KODANSHA SCIENTIFIC LTD., 1994, p. 39-49). The complex may include a solid, a crystal, a liquid, or an amorphous.

As used herein, the mixture containing the cyclic peptide that is a purification target or the mixture containing a cyclic peptide that is a purification target and a peptide that is an impurity may be a crude product obtained by producing the cyclic peptide that is a purification target.

As used herein, the number of amino acid residues of the cyclic peptide that is a purification target may be 5 or more, 7 or more, 9 or more, or 10 or more, and may be 20 or less, 18 or less, 16 or less, or 14 or less. The number of amino acid residues of the cyclic peptide that is a purification target may be 5 to 20, 5 to 14, 10 to 20, or 10 to 14.

As used herein, the number of amino acid residues of the cyclic moiety of the cyclic peptide that is a purification target may be 5 or more, 7 or more, 8 or more, or 10 or more, and may be 15 or less, 14 or less, 13 or less, 12 or less, or 11 or less. The number of amino acid residues of the cyclic moiety of the cyclic peptide that is a purification target may be 5 to 15, 5 to 14, 10 to 15, or 8 to 14, and may be 8, 11, 13, or 14.

The term "amino acid" as used herein includes a natural amino acid and a non-natural amino acid. The term "amino acid" as used herein may mean an amino acid residue. The "natural amino acid" as used herein refer to Gly, L-Ala, L-Ser, L-Thr, L-Val, L-Leu, L-Ile, L-Phe, L-Tyr, L-Trp, L-His, L-Glu, L-Asp, L-Gln, L-Asn, L-Cys, L-Met, L-Lys, L-Arg, and L-Pro. The "non-natural amino acid" is not particularly limited, and examples thereof include β-amino acids, γ-amino acids, D-type amino acids, N-substituted amino acids, α,α-disubstituted amino acids, and amino acids in which the side chain differs from that of natural amino acids. As the amino acid as used herein, any steric configuration is acceptable. The selection of a side chain of the amino acid is not particularly limited, and the side chain is freely selected from, in addition to a

hydrogen atom, for example, an alkyl group, an alkenyl group, an alkynyl group, an aryl group, a heteroaryl group, an aralkyl group, a heteroaralkyl group, a cycloalkyl group, and a spiro-bonded cycloalkyl group. A substituent may be further added to each of the substituents. Such a substituent is not limited and can be one or two or more substituents each independently freely selected from any substituents including a halogen atom, an oxygen atom, a nitrogen atom, a sulfur atom, a boron atom, a silicon atom, or a phosphorus atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group that is optionally substituted, or oxo, aminocarbonyl, and a halogen atom.

The "side chain of amino acid" as used herein, in the case of an α-amino acid, means an atomic group bonded to a carbon (α-carbon) to which an amino group and a carboxyl group are bonded. For example, the methyl group of Ala is a side chain of the amino acid. In the case of a β-amino acid, an atomic group attached to an α-carbon and/or a β-carbon can be a side chain of the amino acid, and in the case of a γ-amino acid, an atomic group attached to an α-carbon, a β-carbon, and/or a γ-carbon can be a side chain of the amino acid.

The term "main chain of an amino acid" in the present specification means a chain portion formed by an amino group, α-carbon and a carboxyl group in the case of an α-amino acid, a chain portion formed by an amino group, β-carbon, α-carbon and a carboxyl group in the case of a β-amino acid, and a chain portion formed by an amino group, γ-carbon, β-carbon, α-carbon and a carboxyl group in the case of a γ-amino acid.

The amino group of the main chain of the amino acid may be unsubstituted ($-NH_2$) or substituted (that is, -NHR). R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position α may form a ring, like proline.

Examples of the "halogen atom-containing substituent" as used herein include fluoro (-F), chloro (-Cl), bromo (-Br), and iodo (-I).

Examples of the "oxygen atom-containing substituent" as used herein include hydroxy (-OH), oxy (-OR), carbonyl (-C(=O)-R), carboxy ($-CO_2H$), oxycarbonyl (-C=O-OR), carbonyloxy (-O-C=O-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxylcarbonyl ($-C(=O)-CO_2H$).

Examples of the "nitrogen atom-containing substituent" as used herein include azido ($-N_3$, also referred to as "azide group"), cyano (-CN), primary amino ($-NH_2$), secondary amino (-NH-R), tertiary amino (-NR(R')), amidino ($-C(=NH)-NH_2$), substituted amidino (-C(=NR)-NR'R"), guanidino ($-NH-C(=NH)-NH_2$), substituted guanidino (-NR-C(=NR"')-NR'R"), and aminocarbonylamino (-NR-CO-NR'R").

Examples of the "sulfur atom-containing substituent" as used herein include thiol (-SH), thio (-S-R), sulfinyl (-S=O-R), sulfonyl ($-S(O)_2-R$), sulfo ($-SO_3H$), and pentafluorosulfanyl ($-SF_5$).

Examples of the "boron atom-containing substituent" as used herein include boryl (-BR(R')), dioxyboryl (-B(OR)(OR')), and trifluoroborate salts ($-BF_3^-$). Specific examples include a "boron atom-derived substituent" in which the two substituents R and R' are each independently selected from alkyl, cycloalkyl, alkenyl, alkynyl, aryl, heteroaryl, and aralkyl, or a "boron atom-derived substituent" in which these two substituents R and R' are taken together with the atoms to which R and R' are each attached to form a ring, that is a cyclic boryl group.

Examples of the "phosphorus atom-containing substituent" as used herein include phosphoryl ($-P(O)-R_1R_2$), phosphonyl ($-O-P(O)-R_1R_2$), and phospho ($-PO_3H_2$).

As used herein, an amino acid in which the amino group of the main chain is substituted is referred to as an "N-substituted amino acid". Examples of the N-substituted amino acid include an $N-C_1-C_6$ alkylamino acid substituted with an alkyl group ($C_1-C_6$ alkyl group) having 1 to 6 carbon atoms, preferably an $N-C_1-C_4$ alkylamino acid. Specific examples of the N-substituted amino acid include N-methylamino acid, N-ethylamino acid, and N-propylamino acid. The N-propyl amino acid includes N-isopropyl amino acid and N-normalpropyl amino acid. As used herein, the cyclic peptide that is a purification target may comprise at least one or more non-natural amino acids. As used herein, the cyclic peptide that is a purification target may comprise an N-substituted amino acid. The cyclic peptide that is a purification target may comprise 3 or more, 4 or more, 5 or more, or 6 or more N-substituted amino acids, and may comprise 15 or less, 13 or less, or 10 or less N-substituted amino acids. The number of N-substituted amino acid residues of the cyclic peptide that is a purification target may be 45% or more, 50% or more, 55% or more, or 60% or more, may be 80% or less, 75% or less, 70% or less, or 65% or less, and is preferably 45% to 80% with respect to the number of amino acid residues of the cyclic moiety of the cyclic peptide.

As used herein, the N-substituted amino acid may be at least one selected from the group consisting of N-methylamino acid, N-ethylamino acid and N-propylamino acid, and may be N-methylamino acid.

As used herein, an amino acid in which the amino group of the main chain is not substituted is referred to as an "N-unsubstituted amino acid". As used herein, the cyclic peptide that is a purification target may comprise an N-unsubstituted amino acid. The N-unsubstituted amino acid may be a non-natural amino acid. The proportion of the number of N-unsubstituted amino acids in the cyclic peptide that is a purification target may be 55% or less, 50% or less, 45% or less, or

40% or less, and may be 20% or more, 25% or more, 30% or more, or 35% or more, and is preferably 20% to 55% with respect to a total number of the amino acid residues in the cyclic peptide that is a purification target.

The compound described herein may contain an isotopic atom at a non-natural ratio in one or more atoms that constitute the compounds. The compound in which any atom in the compound is replaced with another isotopic atom having the same atomic number (number of protons) and a different mass number (sum of the numbers of protons and neutrons), thereby the abundance ratio of the isotope is different from the abundance ratio in nature, that is, a compound labeled with an isotopic atom, is also included in the present invention. Examples of the isotopic element contained in the compounds herein include a hydrogen atom, a carbon atom, a nitrogen atom, an oxygen atom, a phosphorus atom, a sulfur atom, a fluorine atom, and a chlorine atom, and they include $^{2}H$ , $^{3}H$; $^{13}C$, $^{14}C$; $^{15}N$; $^{17}O$, $^{18}O$; $^{32}P$; $^{35}S$; $^{18}F$; $^{36}Cl$; and the like, respectively. The compound labeled with an isotopic atom is useful as a therapeutic agent, a prophylactic agent, a research reagent (e.g., assay reagent), or a diagnostic agent (e.g., in vivo imaging diagnostic agent). For the compounds as used herein, all the compounds containing any proportions of radioactive or non-radioactive isotopic element are encompassed within the scope of the present invention. The compound labeled with an isotopic atom can be produced in a manner similar to methods for producing unlabeled compounds by using a reagent or a solvent containing a corresponding isotopic atom.

As used herein, the ClogP/the number of amino acid residues of the cyclic peptide that is a purification target may be 1.0 or more, 1.1 or more, 1.2 or more, or 1.3 or more, and may be 1.8 or less, 1.7 or less, 1.6 or less, or 1.5 or less. The ClogP/the number of amino acid residues of the cyclic peptide that is a purification target may be 1.0 or more and 1.8 or less. The ClogP is a partition coefficient calculated by a computer, and can be determined in accordance with the principle described in "CLOGP Reference Manual Daylight Version 4.9 (release date: August 1, 2011, https://www.daylight.com/dayhtml/doc/clogp/)[access date: December 25, 2023]". Examples of the method for calculating the ClogP include calculating using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

As used herein, the peptide that is an impurity may be a peptide generated in a synthesis process of the cyclic peptide that is a purification target. The peptide that is an impurity may be a cyclic peptide that is different from the cyclic peptide that is a purification target. The peptide that is an impurity may be a cyclic peptide having the number of amino acids twice the number of amino acids of the cyclic peptide that is a purification target, a cyclic peptide having the number of amino acids three times the number of amino acids of the cyclic peptide that is a purification target, or an isomer of the cyclic peptide that is a purification target. Examples of the isomer preferably include a diastereomer.

As used herein, the metal atom or the metal ion may be an atom or ion of an alkali metal atom, an alkaline earth metal atom, a transition metal atom, a rare earth metal atom, or a poor metal atom.

The alkali metal is preferably at least one selected from the group consisting of lithium, sodium, potassium, rubidium, and cesium, and more preferably lithium or potassium.

The alkaline earth metal is preferably at least one selected from the group consisting of magnesium, calcium, strontium, and barium, and more preferably at least one selected from the group consisting of magnesium, calcium, and barium.

The transition metal is preferably at least one selected from the group consisting of scandium, manganese, iron, zinc, and tungsten, and more preferably at least one selected from the group consisting of scandium, manganese, and zinc.

The rare earth metal is preferably at least one selected from the group consisting of cerium, samarium, and ytterbium.

The poor metal is preferably at least one selected from the group consisting of bismuth and indium.

The metal atom is preferably at least one selected from the group consisting of a magnesium atom, a scandium atom and a samarium atom, and more preferably a magnesium atom. The metal ion is preferably at least one selected from the group consisting of a magnesium ion, a scandium ion and a samarium ion, and more preferably a magnesium ion.

The metal atom or the metal ion may be an atom or ion derived from a metal salt or a solvate thereof or a second complex, and may be an atom or ion derived from a metal salt or a solvate thereof. The second complex may mean a composite (complex) of a cyclic peptide that is a purification target or a peptide that is an impurity with a metal atom or a metal ion and the complex different from the first complex, and may use a complex of a cyclic peptide that is a purification target or a peptide that is an impurity with a metal atom or a metal ion and the same complex as the first complex. The metal salt may mean a metal salt that is different from the first metal salt, and may use the same metal salt as the first metal salt.

The metal salt is preferably at least one selected from the group consisting of an iodide salt, a bromide salt, a chloride salt, a perchlorate salt, an oxide salt, a trifluoromethanesulfonate salt, a toluenesulfonate salt, an isopropyl sulfonate salt, a methanesulfonate salt, a carbonate salt, an acetate salt, a bis(trifluoromethanesulfonate)imide salt, an ethylmalonate salt, a nitrite salt, and a sulfate salt, more preferably at least one selected from the group consisting of an iodide salt, a bromide salt, a perchlorate salt, and a trifluoromethanesulfonate salt, and most preferably a perchlorate salt and a trifluoromethanesulfonate salt.

The iodide salt may be at least one selected from the group consisting of lithium iodide, potassium iodide, barium

iodide, magnesium iodide, calcium iodide, strontium iodide, samarium(III) iodide, zinc iodide, and indium iodide.

The bromide salt may be at least one selected from the group consisting of lithium bromide, magnesium bromide, calcium bromide, samarium(III) bromide, zinc bromide, and indium bromide.

The chloride salt may be samarium chloride, cerium chloride, or magnesium chloride, and may be samarium chloride or cerium chloride.

The perchlorate salt may be at least one selected from the group consisting of lithium perchlorate, barium perchlorate, magnesium perchlorate, calcium perchlorate, and zinc perchlorate.

The oxide salt may be magnesium oxide.

The trifluoromethanesulfonate salt may be at least one selected from the group consisting of magnesium trifluoromethanesulfonate, calcium trifluoromethanesulfonate, scandium trifluoromethanesulfonate, samarium(III) trifluoromethanesulfonate, cerium trifluoromethanesulfonate, ytterbium trifluoromethanesulfonate, zinc trifluoromethanesulfonate, manganese trifluoromethanesulfonate, indium trifluoromethanesulfonate, iron(III) trifluoromethanesulfonate, and copper trifluoromethanesulfonate.

The toluenesulfonate salt may be zinc(II) toluenesulfonate.

The isopropyl sulfonate salt may be zinc(II) isopropyl sulfonate.

The methanesulfonate salt may be at least one selected from the group consisting of cerium(III) methanesulfonate and zinc difluoromethanesulfonate.

The carbonate salt may be at least one selected from the group consisting of potassium carbonate, calcium carbonate, and zinc carbonate.

The acetate salt may be at least one selected from the group consisting of potassium acetate and magnesium acetate.

The bis(trifluoromethanesulfonate)imide salt may be at least one selected from the group consisting of magnesium bis(trifluoromethanesulfonate)imide, zinc bis(trifluoromethanesulfonate)imide, and iron(II) bis(trifluoromethanesulfonate)imide.

The ethylmalonate salt may be magnesium ethylmalonate.

The nitrite salt may be potassium nitrite.

The sulfate salt may be magnesium sulfate.

The metal atom or the metal ion may be an atom or an ion derived from at least one selected from the group consisting of lithium iodide, lithium perchlorate, lithium tetrafluoroborate, lithium bromide, lithium chloride, potassium iodide, lithium fluoride, potassium carbonate, potassium nitrite, potassium acetate, potassium tetrafluoroborate, potassium hexafluorophosphate, barium iodide, barium perchlorate, magnesium ethylmalonate, magnesium bis(trifluoromethanesulfonic acid) imide, magnesium oxide, magnesium bromide, magnesium perchlorate, magnesium iodide, magnesium trifluoromethanesulfonate, magnesium sulfate, magnesium acetate, magnesium chloride, magnesium fluoride, calcium iodide, calcium perchlorate, calcium bromide, calcium trifluoromethanesulfonate, calcium carbonate, strontium iodide, scandium trifluoromethanesulfonate, scandium chloride, samarium(III) trifluoromethanesulfonate, samarium(III) iodide, samarium(III) bromide, samarium(III) chloride, cerium(III) methanesulfonate, cerium(III) chloride, cerium(III) chloride heptahydrate, cerium trifluoromethanesulfonate, ytterbium trifluoromethanesulfonate, zinc trifluoromethanesulfonate, zinc(II) tetrafluoroborate, zinc perchlorate, zinc difluoromethanesulfonate, zinc(II) isopropyl sulfonate, zinc(II) toluenesulfonate, zinc bis(trifluoromethanesulfonic acid) imide, zinc bromide, zinc chloride, zinc carbonate, zinc fluoride, zinc iodide, manganese trifluoromethanesulfonate, manganese chloride, indium trifluoromethanesulfonate, indium bromide, indium chloride, indium iodide, iron(III) trifluoromethanesulfonate, iron(II) bis(trifluoromethanesulfonic acid) imide, tungsten(VI) fluoride, tungsten(VI) chloride, and copper trifluoromethanesulfonate.

The metal atom or the metal ion is preferably an atom or an ion derived from at least one metal salt or a solvate thereof selected from the group consisting of lithium iodide, lithium perchlorate, potassium iodide, barium iodide, barium perchlorate, magnesium bromide, magnesium perchlorate, magnesium iodide, magnesium trifluoromethanesulfonate, calcium bromide, scandium trifluoromethanesulfonate, samarium trifluoromethanesulfonate, cerium trifluoromethanesulfonate, ytterbium trifluoromethanesulfonate, zinc trifluoromethanesulfonate, manganese trifluoromethanesulfonate, indium trifluoromethanesulfonate, magnesium sulfate, magnesium acetate, and magnesium chloride.

The metal atom or the metal ion may be an atom or an ion derived from at least one metal salt or a solvate thereof selected from the group consisting of lithium perchlorate, potassium iodide, magnesium bromide, magnesium perchlorate, magnesium iodide, and manganese trifluoromethanesulfonate. When the metal atom or the metal ion is an atom or ion derived from the metal salt or a solvate thereof described in this paragraph, the purity of the cyclic peptide after purification can be further improved.

The ligand of the second complex may be at least one selected from the group consisting of an olefinic compound, a carbonyl-based compound, a phosphine-based compound, and carbon monoxide, and may be at least one selected from the group consisting of ethylene, dibenzylidene acetone, acetyl acetonate, triphenylphosphine and carbon monoxide.

The second complex may be at least one selected from the group consisting of iron(III) tris (acetylacetonate),

ferrocene, zinc(II) acetylacetonate, and tungsten hexacarbonyl.

The first solvent may be a solvent capable of forming a composite of the cyclic peptide that is a purification target or the peptide that is an impurity with the metal atom or the metal ion, and may include at least one selected from the group consisting of an alcohol-based solvent, a nitrile-based solvent, a benzene-based solvent, an ether-based solvent, a ketone-based solvent, a halogen-based solvent, an ester-based solvent, a sulfoxide-based solvent, and an amide-based solvent. When the first solvent is the solvent described in this paragraph, it is easier to form a complex of a cyclic peptide that is a purification target or a peptide that is an impurity with a metal atom or a metal ion.

Specific examples of the alcohol-based solvent herein can include methanol, ethanol, 1-propanol, 2-propanol, tert-butanol, 2,2,2-trifluoroethanol, and ethylene glycol. Specific examples of the nitrile-based solvent include chain-like nitriles such as acetonitrile, propionitrile, and acrylonitrile; and cyclic nitriles such as benzonitrile. Specific examples of the benzene-based solvent include toluene, o-dichlorobenzene, 1,2,4-trichlorobenzene, and xylene. Specific examples of the ether-based solvent can include diethyl ether, DME (dimethyl ether), tetrahydrofuran, glyme, 1,4-dioxane, 2-methyltetrahydrofuran, MTBE (tert-butyl methyl ether, methyl tert-butyl ether, 2-methoxy-2-methylpropane, CAS: 1634-04-4), CPME (cyclopentyl methyl ether), tetrahydropyran, and dimethoxyethane. Specific examples of the ketone-based solvent can include acetone, methyl ethyl ketone, methyl isobutyl ketone, and cyclohexanone. Specific examples of the halogen-based solvent can include haloalkanes such as dichloromethane and dichloroethane, and haloarenes such as chlorobenzene. Specific examples of the ester-based solvent can include methyl acetate, ethyl acetate, isopropyl acetate, butyl acetate, gamma-butyrolactone (GBL), ethylene carbonate (EC), propylene carbonate, butylene carbonate, dimethyl carbonate, diethyl carbonate (DEC), di-n-propyl carbonate, ethyl methyl carbonate (EMC), methyl-n-propyl carbonate, and ethyl-n-propyl carbonate.

The alcohol-based solvent as the first solvent is preferably at least one selected from the group consisting of 2-propanol, tert-butanol, 2,2,2-trifluoroethanol, ethanol, and methanol. The nitrile-based solvent as the first solvent is preferably acetonitrile. The benzene-based solvent as the first solvent is preferably toluene or xylene. The ether-based solvent as the first solvent is preferably at least one selected from the group consisting of tetrahydrofuran, 1,4-dioxane, 2-methyltetrahydrofuran, MTBE (tert-butyl methyl ether), DME (dimethyl ether) and CPME (cyclopentyl methyl ether). The ketone-based solvent as the first solvent is preferably acetone or methyl ethyl ketone. The halogen-based solvent as the first solvent is preferably dichloromethane. The sulfoxide-based solvent as the first solvent is preferably dimethyl sulfoxide. The amide-based solvent as the first solvent is preferably dimethylformamide or dimethylacetamide.

The first solvent is preferably at least one selected from the group consisting of 2-propanol, tert-butanol, 2,2,2-trifluoroethanol, acetonitrile, tetrahydrofuran, toluene, 1,4-dioxane, acetone, dichloromethane, ethanol, methanol, dimethyl sulfoxide, dimethylformamide and dimethyl acetamide, and more preferably at least one selected from the group consisting of 2,2,2-trifluoroethanol, acetonitrile, tetrahydrofuran, and acetone.

The second solvent may comprise at least one selected from the group consisting of an ether-based solvent, a benzene-based solvent, a ketone-based solvent, a halogen-based solvent, an ester-based solvent, a nitrile-based solvent, a hydrocarbon-based solvent, an alcohol-based solvent, a sulfoxide-based solvent, and an amide-based solvent.

The ether-based solvent as the second solvent is preferably at least one selected from the group consisting of 2-methyltetrahydrofuran, tetrahydrofuran, 1,4-dioxane, MTBE (tert-butyl methyl ether), DME (dimethyl ether) and CPME (cyclopentyl methyl ether). The benzene-based solvent as the second solvent is preferably toluene or xylene. The ketone-based solvent as the second solvent is preferably acetone or methyl ethyl ketone. The halogen-based solvent as the second solvent is preferably dichloromethane. The hydrocarbon-based solvent as the second solvent is preferably at least one selected from the group consisting of hexane, heptane, cyclohexane, methylcyclohexane, or isooctane. The ester-based solvent as the second solvent is preferably isopropyl acetate or ethyl acetate. The nitrile-based solvent as the second solvent is preferably acetonitrile. The alcohol-based solvent as the second solvent is preferably at least one selected from the group consisting of methanol, ethanol, butanol, or benzyl alcohol. The sulfoxide-based solvent as the second solvent is preferably dimethyl sulfoxide. The amide-based solvent as the second solvent is preferably dimethylformamide.

The second solvent is preferably a combination of isopropyl acetate or methyl ethyl ketone and heptane, a combination of dichloromethane and hexane, or a combination of acetonitrile and MTBE.

The third solvent herein may be a solvent that is not miscible with water (e.g., a solvent having a low solubility in water, a solvent having a high octanol/water partition coefficient (log Kow), or a solvent having a high octanol/water partition coefficient prediction value).

The octanol/water partition coefficient (Log Kow) can be determined by any method known in the art or described herein. The octanol/water partition coefficient (Log Kow) prediction value may be determined by known means in separate explicit measurements by, for example, but not particularly limited to, database search or literature search. Examples of the method for measuring the octanol/water partition coefficient include, but are not limited to, the method in accordance with the Japanese Industrial Standard JIS 7260-107: 2000 Measurement of Partition coefficient (1-octanol/water) Shake flask method (https://kikakurui.com/z7/Z7260-107-2000-01.html)[access date: December 25, 2023].

In an embodiment, the organic solvent that is not miscible with water includes, but is not intended to be particularly

limited to, an organic solvent having low water solubility (e.g., having a solubility in water of 200 g/L or less, preferably 150 g/L or less). The organic solvent that is not miscible with water may contain a trace amount of, for example, 0.01 wt% or less, other organic solvents that are miscible with water. The solubility in water can be determined by any method known in the art or described herein. Examples of the method for determining solubility include, but are not intended to be particularly limited to, gas chromatography, and the solubility can be determined by measuring the concentration of the organic solvent in water prepared by mixing the organic solvent with the same volume of water at room temperature (e.g., 15°C to 40°C, preferably 20°C to 30°C).

In an embodiment, for determining the miscibility of an organic solvent with water, the miscibility can also be shown by the separation of the solvent and water into two layers when the same volume of the solvent and water are mixed in a vessel, for example, at room temperature (e.g., 15°C to 40°C, preferably 20°C to 30°C). Whether the solvent and water separate into two layers can be determined, for example, by checking visually, or by collecting and checking the liquids in the upper and lower layers in the vessel. When it is confirmed that the solvent and water are separated into two layers in this way, the solvent may be referred to as a solvent that is not miscible with water. However, even a solvent that is miscible with water, depending on the solute in the solvent and the salt concentration in water, the solvent may form an interface with water and separate into two layers.

In an embodiment, the solvent that is not miscible with water can be characterized as an ester having 3 or more and 10 or less carbon atoms, and specific examples thereof include ethyl acetate, isopropyl acetate, n-propyl acetate, t-butyl acetate, methyl propionate or ethyl propionate.

In an embodiment, the solvent that is not miscible with water can be characterized as a cyclic ether having 4 or more and 10 or less carbon atoms, and specific examples thereof include 2-methyltetrahydrofuran, tetrahydrofuran, 4-methyltetrahydropyran, or 1,4-dioxane. In an embodiment, the solvent that is not miscible with water can be characterized as an acyclic ether having 4 or more and 10 or less carbon atoms, and specific examples thereof include MTBE (tert-butyl methyl ether), diisopropyl ether or diethyl ether.

In an embodiment, the solvent that is not miscible with water can be characterized as an ether having both cyclic and acyclic alkyl groups having 6 or more and 10 or less carbon atoms, and specific examples thereof include CPME.

In an embodiment, the solvent that is not miscible with water can be characterized as a carbonate ester having 3 or more and 10 or less carbon atoms, and specific examples thereof include dimethyl carbonate, diethyl carbonate, or diisopropyl carbonate.

In an embodiment, the solvent that is not miscible with water can be characterized as a hydrocarbon having 5 or more and 10 or less carbon atoms, and specific examples thereof include pentane, hexane or heptane.

In an embodiment, the solvent that is not miscible with water can be characterized as an aromatic hydrocarbon ring having 6 or more and 10 or less carbon atoms, and specific examples thereof include toluene, xylene, or benzene.

In an embodiment, the solvent that is not miscible with water can be characterized as having a low boiling point at ambient pressure (about 1 atm). In an embodiment, examples of the low boiling point at normal pressure (near 1 atm) include 35°C or higher and lower than 140°C.

The solvent that is not miscible with water is preferably at least one selected from the group consisting of 2-methyltetrahydrofuran, tetrahydrofuran, dimethyl carbonate, anisole, isopropyl acetate, ethyl acetate, MTBE (tert-butyl methyl ether), diethyl ether, dichloromethane, chloroform, DME (dimethyl ether), CPME (cyclopentyl methyl ether), 4-methyltetrahydropyran, heptane, and toluene.

The fourth solvent herein may be at least one selected from the group consisting of methanol, ethanol, 1-propanol, 2-propanol, 1-butanol, 2-butanol, t-butanol, ethylene glycol, cyclohexane, methylcyclohexane, heptane, isooctane, toluene, xylene, dimethylsulfoxide, acetone, methyl ethyl ketone, tetrahydrofuran, tetrahydropyran, 4-methyltetrahydropyran, 2-methyltetrahydrofuran, 1,4-dioxane, MTBE (tert-butyl methyl ether), DME (dimethyl ether), CPME (cyclopentyl methyl ether), methyl acetate, ethyl acetate, isopropyl acetate, butyl acetate, methylisobutyl ketone, cyclohexanone and acetonitrile.

Specific examples of the ligand or the compound that generates a ligand include an olefinic compound, a carbonyl-based compound, a phosphine-based compound, and carbon monoxide. Specific examples of the anion or the compound that generates an anion include an iodide salt, a bromide salt, a chloride salt, a perchlorate salt, an oxide salt, a trifluoromethanesulfonate salt, a toluenesulfonate salt, an isopropyl sulfonate salt, a methanesulfonate salt, a carbonate salt, an acetate salt, a bis(trifluoromethanesulfonate)imide salt, an ethylmalonate salt, 1,5-cyclooctadiene, dibenzylidene acetone, triphenylphosphine, and a nitrite salt.

The purification method of a cyclic peptide herein may be a method for purifying a cyclic peptide that is a purification target to have a purity of 85% or more, 90% or more, or 95% or more, and may be a method for purifying a cyclic peptide that is a purification target to have a purity of 100%, 98% or more, or 97% or more. The purification method of a cyclic peptide herein may be such that the purity of the cyclic peptide that is a purification target is increased by the purification operation described herein. For example, the purity of the cyclic peptide that is a purification target having 80% purity becomes 83%, 86%, 89%, 92%, 96%, or 100%, or the purity of the cyclic peptide that is a purification target becomes 1.03 times, 1.08 times, 1.11 times, 1.15 times, or 1.20 times the purity before the purification operation, up to 100% purity.

The purification method of a cyclic peptide herein can also be referred to as a method of reducing a content of impurities in a mixture containing a cyclic peptide that is a purification target and an impurity, a method of reducing a ratio of impurities to a cyclic peptide that is a purification target by a purification operation described herein, or a method in which the purification efficiency of impurities, which can be an indicator of the removal of the impurities before and after the purification operation described herein, is a positive value. The purity (%) of the cyclic peptide that is a purification target can be determined, for example, from a proportion of the peak area of the cyclic peptide that is a purification target with respect to the sum of each peak area of the entire mixture containing the cyclic peptide that is a purification target at 220 nm or 225 nm in which UV spectrum of the mixture containing the cyclic peptide that is a purification target is measured using HPLC and PDA (photodiode array detector). The mixing proportion (%) of a peptide that is an impurity (e.g., an isomer of a cyclic peptide that is a purification target) can be determined, for example, from a proportion of the peak area of the peptide that is an impurity with respect to the sum of each peak area of the entire mixture containing the cyclic peptide that is a purification target at 220 nm or 225 nm in which UV spectrum of the mixture containing the cyclic peptide that is a purification target is measured using HPLC and PDA (photodiode array detector).

The purification efficiency of an impurity can be determined, for example, by measuring UV spectrum of a mixture containing a cyclic peptide that is a purification target using HPLC and PDA (photodiode array detector), calculating a ratio of the peak area of the impurity (e.g., an isomer) to the peak area of the cyclic peptide that is a purification target at 220 nm or 225 nm, and comparing the ratios before and after the purification operation described herein. For example, the purification efficiency of an impurity (%) can be determined by the following expression.

Purification efficiency of impurity (%) = {1 - (ratio of cyclic peptide that is a purification target to impurity after purification operation)/(ratio of cyclic peptide that is a purification target to impurity before purification operation)} $\times$ 100 {1 - (ratio of cyclic peptide that is a purification target to impurity before purification operation)/(ratio of cyclic peptide that is a purification target to impurity before purification operation)} $\times$ 100

Still another embodiment of the present invention relates to a production method of a cyclic peptide, comprising the purification method of a cyclic peptide according to the invention. The production method of a cyclic peptide may further comprise a step of obtaining the cyclic peptide that is a purification target by a liquid phase synthesis method, may further comprise a step of obtaining the cyclic peptide that is a purification target by a solid phase synthesis method, and may further comprise a step of obtaining the cyclic peptide that is a purification target by a culture method. The step of obtaining the cyclic peptide that is a purification target by a liquid phase synthesis method means that the liquid phase synthesis comprises a step of obtaining the cyclic peptide that is a purification target. The step of obtaining the cyclic peptide that is a purification target by a solid phase synthesis method means that the solid phase synthesis comprises a step of obtaining the cyclic peptide that is a purification target. The liquid phase synthesis method herein include a method of synthesizing a peptide using an organic tag (also referred to as a hydrophobic tag). The method for synthesizing a peptide using an organic tag is not particularly limited, but examples thereof include those described in WO 2012/029794, WO 2007/122847, and WO 2019/009317.

This application claims priority to Japanese Patent Application No. 2022-212468 filed December 28, 2022, the contents of which are incorporated herein by reference in their entirety. All references cited herein, including the following references, with inclusion of patent applications and publications, are incorporated herein by reference in their entirety: International Publication No. WO 2013/100132; International Publication No. WO 2018/225851; International Publication No. WO 2018/225864; International Publication No. WO 2019/117274; International Publication No. WO 2020/111238; International Publication No. WO 2020/122182; International Publication No. WO 2021/075478; International Publication No. WO 2021/090856; International Publication No. WO 2021/132545; International Publication No. WO 202/1246471; International Publication No. WO 2022/097540; International Publication No. WO 2022/138891; International Publication No. WO 2022/145444; International Publication No. WO 2022/234864; and International Publication No. WO 2023/127869.

[Examples]

The present invention will be described in further detail by way of Examples and Reference Examples, but the present invention is not limited to those. All starting materials and reagents were obtained from commercial suppliers, or synthesized using known methods. The analysis conditions of LC/MS are shown in Table 1.

[Table 1]

| Analysis condition | Device | Column (Particle size, I.D.x length) | Mobile phase | Gradient (A/B) | Flow rate (mL/min) | Column temperature (°C) | Wavelength |
|---|---|---|---|---|---|---|---|
| SQDAA05 | Acquity UPLC/SQD or Acquity UPLC/SQD2 | Ascentis Express C18 (5.0 µm, 2.1 x 50 mm) | A) 10 mM AA,water B) methanol | 95/5 (initial) => 0/100 (1.0 min) => 0/100 (0.4 min) | 1.0 | 35 | 210-400 nm PDA total |
| SQDAA50 | Acquity UPLC/SQD or Acquity UPLC/SQD2 | Ascentis Express C18 (5.0 µm, 2.1 x 50 mm) | A) 10 mM AA,water B) methanol | 50/50 (initial) => 0/100 (0.7 min) => 0/100 (0.7 min) | 1.0 | 35 | 210-400 nm PDA total |
| SQDFA05 | Acquity UPLC/SQD or Acquity UPLC/SQD2 | Ascentis Express C18 (2.7 µm, 2.1 x 50 mm) | A) 0.1% FA, water B) 0.1% FA, MeCN | 95/5 (initial) => 0/100 (1.0 min) => 0/100 (0.4 min) | 1.0 | 35 | 210-400 nm PDA total |
| SQDFA50 | Acquity UPLC/SQD or Acquity UPLC/SQD2 | Ascentis Express C18 (2.7 µm, 2.1 x 50 mm) | A) 0.1% FA, water B) 0.1% FA, MeCN | 50/50 (initial) => 0/100 (0.7 min) => 0/100 (0.7 min) | 1.0 | 35 | 210-400 nm PDA total |
| SQDFA05Long | Acquity UPLC/SQD or Acquity UPLC/SQD2 | Ascentis Express C18 (2.7 µm, 2.1 x 50 mm) | A) 0.1% FA, water B) 0.1% FA, MeCN | 95/5 (initial) => 0/100 (4.5 min) => 0/100 (0.5 min) | 1.0 | 35 | 210-400 nm PDA total |
| Purity analysis condition A | Nexera X2 | ACQUITY UPLC CSH C18 (1.7 µm, 2.1 x 150 mm) | A) 0.1% FA, water B) 0.1% FA, MeCN | 50/50 (initial) => 0/100 (15 min) => 0/100 (5 min) =>50/50 (0.1 min) | 0.3 | 50 | 210-400 nm PDA total (Analysis wavelength: 220 nm) |
| Purity analysis condition B | Vanquish | ACQUITY UPLC CSH Fluoro-Phenyl (1.7 µm, 2.1 x 150 mm) | A) 0.1% FA, water B) 0.1% FA, MeCN | 80/20 (initial) => 0/100 (15 min) => 0/100 (5 min) =>80/20 (0.1 min) | 0.3 | 50 | 210-400 nm PDA total (Analysis wavelength: 220 nm) |

(continued)

| Analysis condition | Device | Column (Particle size, I.D.x length) | Mobile phase | Gradient (A/B) | Flow rate (mL/min) | Column temperature (°C) | Wavelength |
|---|---|---|---|---|---|---|---|
| Purity analysis condition C | Vanquish | ACQUITY UPLC CSH Fluoro-Phenyl (1.7 μm, 2.1 x 150 mm) | A) 0.1% FA, water B) 0.1% FA, MeCN | 80/20 (initial) => 0/100 (15 min) => 0/100 (5 min) =>80/20 (0.1 min) | 0.3 | 50 | 210-400 nm PDA total (Analysis wavelength: 225 nm) |

In Examples and Reference Examples, the following abbreviations were used.

AA: ammonium acetate
COMU: (1-cyano-2-ethoxy-2-oxoethylideneaminooxy)dimethylamino-morpholino-carbenium hexafluorophosphate
DBU: 1,8-diazabicyclo[5.4.0]-7-undecene
DCM: dichloromethane
DCE: 1,2-dichloroethane
DMF: N,N-dimethylformamide
DIC: N,N'-diisopropylcarbodiimide
DIPEA: N,N-diisopropylethylamine
DVB: divinylbenzene
t-Bu group: tert-butyl group
FA: formic acid (methanoic acid)
Fmoc group: 9-fluorenylmethyloxycarbonyl group
NMP: N-methyl-2-pyrrolidone
NMR: nuclear magnetic resonance spectroscopy
mM: mmol/L
PDA: photodiode array detector
TFA: trifluoroacetic acid
TFE: 2,2,2-trifluoroethanol
THF: tetrahydrofuran
THP: tetrahydropyranyl
MTBE: tert-butyl methyl ether
HOAt: 1-hydroxy-7-azabenzotriazole
HOBt: 1-hydroxybenzotriazole
IPAC: isopropyl acetate
MEK: methyl ethyl ketone
HPLC: high-performance liquid chromatography
oxyma: ethyl cyano(hydroxyimino)acetate
EDCI·HCl: 1-ethyl-3-(3-dimethylaminopropyl)carbodiimide hydrochloride
DMSO: dimethyl sulfoxide
MeCN: acetonitrile, $CH_3CN$
$^1H$: proton
v/v: volume/volume

NMR measurements were performed at an absolute temperature of 298 K or 278 K using the AVANCE III 600 Cryo-TCI, Avance III HD 600 SMART-BBFO, AVANCE NEO 600 iProbeTBO or AVANCE NEO 600 Cryo-TCI-H & F manufactured by Bruker.

[Reference Example 1: Synthesis of Cyclic Peptide]

< General Synthesis Method of Amino Acids >

The Fmoc group-protected amino acids (Fmoc-amino acids) used in Examples and Reference Examples can be produced by general amino acid synthesis methods, for example, by the methods described in WO 2021/090855. The Fmoc group-protected amino acids are also available from commercial suppliers. In Examples and Reference Examples, an amino acid in which an Fmoc group is introduced at the N-terminus of the commercially available amino acid or an amino acid in which the C-terminus of the commercially available amino acid is deprotected can also be used for the extension reaction of the peptide of interest. The attachment or removal reaction of the protecting group can be performed, for example, by the methods described in "Greene's, "Protective Groups in Organic Synthesis" (5th edition, John Wiley & Sons 2014)". An amino acid having a side chain (functional group on the alpha carbon of the amino acid) that is different from that of naturally occurring amino acids can be produced by appropriately converting a side chain of an amino acid (naturally occurring amino acid) available from commercial suppliers to the side chain of interest. The conversion of the side chain can be performed, for example, by the methods described in R. C. Larock, Comprehensive Organic Transformations, A Guide to Functional Group Preparations, 3rd Edition; M. B. March, March's Advanced Organic Chemistry: Reactions, Mechanisms, and Structure, 7th Edition; and the like. An N-terminal alkylated amino acid (N-alkylamino acid) can be produced by reacting an amino acid whose N-terminal end is not alkylated with an aldehyde according to the method by Freidinger et al. (J. Org. Chem., 1983, 48(1), 77-81) to produce an oxazolidinone compound in which a cyclic protecting group is introduced, and performing a ring-opening reaction of the cyclic protecting group.

The Fmoc-amino acids used in Examples and Reference Examples are shown in Tables 2 to 4. The Fmoc-amino acids described in Tables 2 to 4 were purchased from commercial suppliers or synthesized with reference to the method described in WO 2021/090855.

[Table 2]

| Abbreviation | Amino acid structural formula | Name | CAS No. |
|---|---|---|---|
| Fmoc-Val-OH | 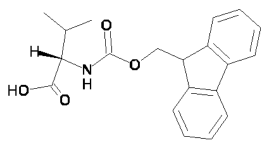 | (2S)-2-(9H-Fluoren-9-ylmethoxycarbonylamino)-3-methylbutanoic acid | 68858-20-8 |
| Fmoc-Thr-OH | 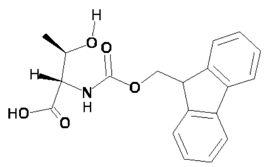 | (2S,3R)-2-(9H-Fluoren-9-ylmethoxycarbonylamino)-3-hydroxybutanoic acid | 73731-37-0 |
| Fmoc-Ser(n-Pr)-OH | 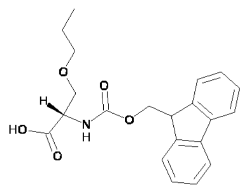 | (2S)-2-(9H-Fluoren-9-ylmethoxycarbonylamino)-3-propoxypropanoic acid | 2255321-09-4 |
| Fmoc-Ser-OH | 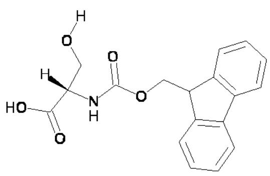 | (2S)-2-(9H-Fluoren-9-ylmethoxycarbonylamino)-3-hydroxypropanoic acid | 73724-45-5 |
| Fmoc-Pro-OH | 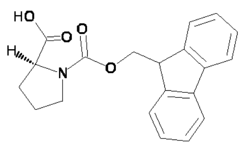 | (2S)-1-(9H-Fluoren-9-ylmethoxycarbonyl) pyrrolidine-2-carboxylic acid | 71989-31-6 |

(continued)

| Abbreviation | Amino acid structural formula | Name | CAS No. |
|---|---|---|---|
| Fmoc-Phe-OH | 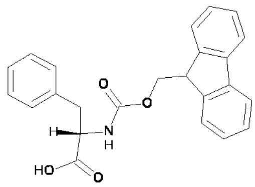 | (2S)-2-(9H-Fluoren-9-ylmethoxycarbonylamino)-3-phenylpropanoic acid | 35661-40-6 |
| Fmoc-MeVal-OH | 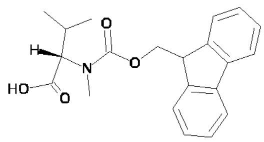 | (2S)-2-[9H-Fluoren-9-ylmethoxycarbonyl(methyl)ami no]-3-methylbutanoic acid | 84000-11-3 |
| Fmoc-MePhe(4-F)-OH | 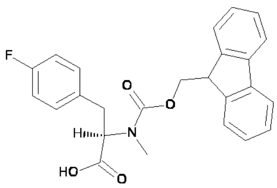 | (2S)-2-[9H-Fluoren-9-ylmethoxycarbonyl(methyl)ami no]-3-(4-fluorophenyl)propanoic acid | 1979176-87-8 |

[Table 3]

| Abbreviation | Amino acid structural formula | Name | CAS No. |
|---|---|---|---|
| Fmoc-MePhe(34-F2)-OH | 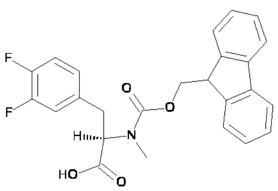 | (2S)-3-(3,4-Difluorophenyl)-2-[9H-fluoren-9-ylmethoxycarbonyl(methyl)a mino]propanoic acid | 2306437-98-7 |
| Fmoc-MePhe-OH | 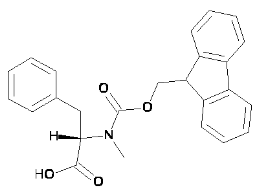 | (2S)-2-[9H-Fluoren-9-ylmethoxycarbonyl(methyl)a mino]-3-phenylpropanoic acid | 77128-73-5 |
| Fmoc-MeLeu-OH | 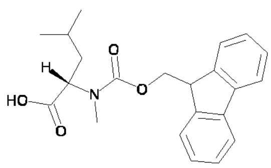 | (2S)-2-[9H-Fluoren-9-ylmethoxycarbonyl(methyl)a mino]-4-methylpentanoic acid | 103478-62-2 |
| Fmoc-Melle-OH | 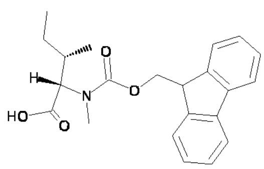 | (2S,3S)-2-[9H-Fluoren-9-ylmethoxycarbonyl(methyl)a mino]-3-methylpentanoic acid | 138775-22-1 |
| Fmoc-MeGly-OH | 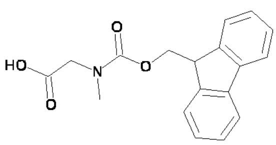 | 2-[9H-Fluoren-9-ylmethoxycarbonyl(methyl) a mino]acetic acid | 77128-70-2 |

(continued)

| Abbreviation | Amino acid structural formula | Name | CAS No. |
|---|---|---|---|
| Fmoc-MeAla-OH | 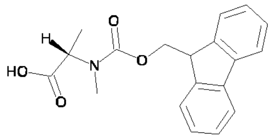 | (2S)-2-[9H-Fluoren-9-ylmethoxycarbonyl(methyl)a mino]propanoic acid | 84000-07-7 |
| Fmoc-Leu-OH | 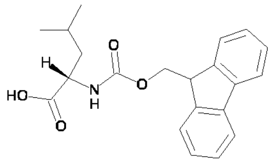 | (2S)-2-(9H-Fluoren-9-ylmethoxycarbonylamino)-4-methylpentanoic acid | 35661-60-0 |
| Fmoc-Ile-OH | 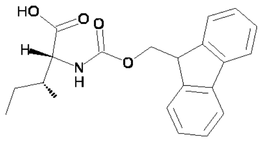 | (2S,3S)-2-(9H-Fluoren-9-ylmethoxycarbonylamino)-3-methylpentanoic acid | 71989-23-6 |

[Table 4]

| Abbreviation | Amino acid structural formula | Name | CAS No. |
|---|---|---|---|
| Fmoc-O-Val-OH | 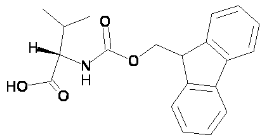 | (2R)-2-(9H-Fluoren-9-ylmethoxycarbonylamino)-3-methylbutanoic acid | 84624-17-9 |
| Fmoc-D-MeAla-OH | 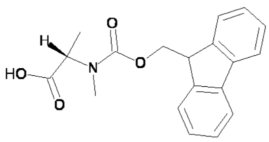 | (2R)-2-[9H-Fluoren-9-ylmethoxycarbonyl(methyl)a mino]propanoic acid | 138774-92-2 |
| Fmoc-Cha-OH | 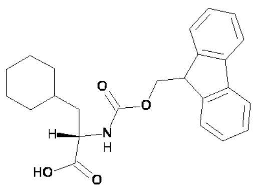 | (2S)-3-Cyclohexyl-2-(9H-fluoren-9-ylmethoxycarbonylamino)pro panoic acid | 135673-97-1 |
| Fmoc-bMeAla-OH | 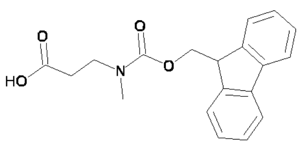 | 3-[9H-Fluoren-9-ylmethoxycarbonyl(methyl)a mino]propanoic acid | 172965-84-3 |
| Fmoc-Ala(cPen-t)-OH | 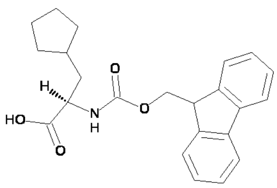 | (2S)-3-Cyclopentyl-2-(9H-fluoren-9-ylmethoxycarbonylamino)pro panoic acid | 371770-32-0 |
| Fmoc-Ala-OH | 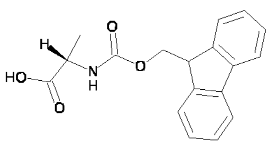 | (2S)-2-(9H-Fluoren-9-ylmethoxycarbonylamino)pro panoic acid | 35661-39-3 |

(continued)

| Abbreviation | Amino acid structural formula | Name | CAS No. |
|---|---|---|---|
| Fmoc-Thr(THP)-OH | 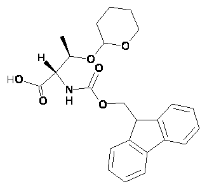 | (2S,3R)-2-(9H-Fluoren-9-ylmethoxycarbonylamino)-3-(oxan-2-yloxy)butanoic acid | 918531-00-7 |
| Fmoc-Ser(THP)-OH | 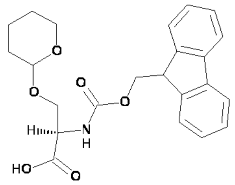 | (2S)-2-(9H-Fluoren-9-ylmethoxycarbonylamino)-3-(oxan-2-yloxy)propanoic acid | 2254698-75-2 |

< Synthesis Method of Fmoc-Amino Acid-Support Resin >

Compound aa01, compound aa02, and compound aa03, which are Fmoc-amino acids, were synthesized as shown in formula (1) below. The obtained compound aa03 was used to synthesize compound aa03-resin ((3S)-3-(9H-fluoren-9-ylmethoxycarbonylamino)-4-oxo-4-pyrrolidine-1-ylbutanoic acid-2-chlorotritylresin (Fmoc-Asp(O-Trt(2-Cl)-resin)-pyrro), in which compound aa03 was supported on a resin. Compound aa03-resin was used for peptide synthesis with a peptide synthesizer. The supporting reaction of the Fmoc-amino acid to a resin was performed by the method described in WO 2013/100132 or WO 2018/225864. 2-Chlorotrityl chloride resin (100-200 mesh, 1% DVB) was purchased from WATANABE CHEMICAL INDUSTRIES, LTD. and Chem-Impex International, Inc. Note that pyrro means pyrrolidine. For example, in compound aa03-resin, the C-terminal carboxyl group forms an amide bond with pyrrolidine.

[Formula 1]

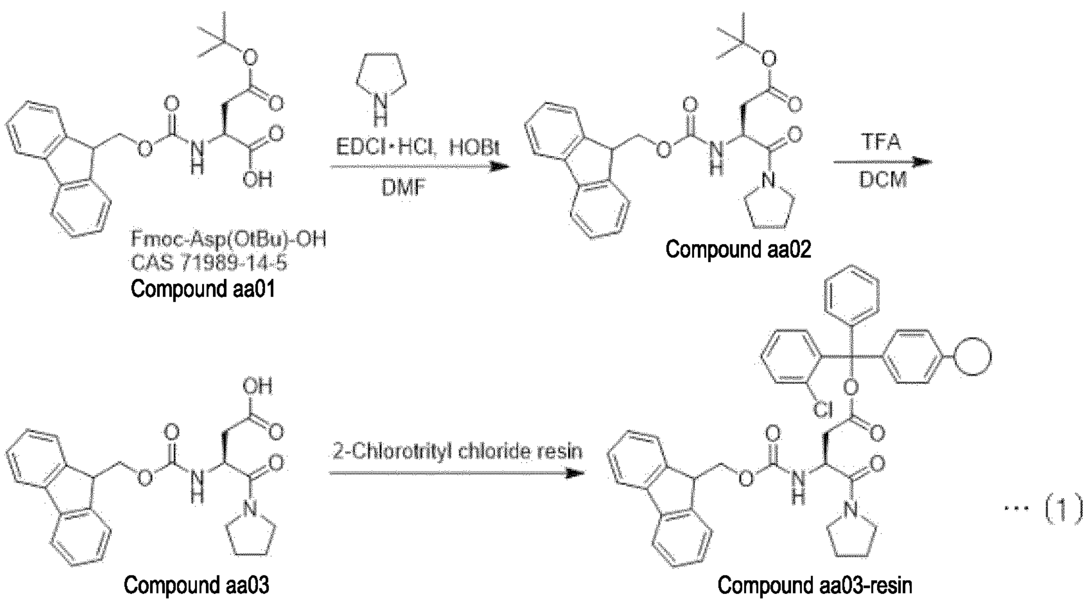


Herein, in the chemical formula of a compound obtained by binding a polymer or resin to an amino acid or peptide compound, a portion or all of the polymer or resin may be denoted as circle (○). The chemical structure of a binding site of a resin and an amino acid or peptide compound may be denoted to clarify the structure of the binding site. For example, in compound aa03-resin of the formula (1) above, a portion of the resin is indicated by ○ and it is shown that the 2-chlorotrityl group of the resin and the side chain carboxyl group of Asp are bound via an ester bond.

In the formula (1), compound aa01 was purchased from a commercial supplier. The synthesis method of compound aa02 will be described in detail. EDCI·HCl (67.1 g, 350 mmol), HOBt (43.4 g, 321 mmol) and Fmoc-Asp(OtBu)-OH (compound aa01, CAS: 71989-14-5) (120 g, 292 mmol) were mixed sequentially into DMF (600 mL) under nitrogen atmosphere at 0°C. The obtained mixture was stirred at 0°C for 1 hour. To the obtained reaction solution, pyrrolidine (26.3 mL, 321 mmol) was slowly added, and the mixture was stirred at 0°C for 1 hour and 30 minutes. To this reaction solution, ethyl acetate (10 v/w) and 0.5 mol/L hydrochloric acid water (2 v/w) were added at 0°C, and the organic phase was separated. The obtained organic phase was washed sequentially with 0.5 mol/L hydrochloric acid water, water, saturated aqueous sodium bicarbonate solution/water (1/1 (v/v)), and saturated aqueous sodium chloride solution/water (1/1 (v/v)). The washed organic phase was dried over anhydrous sodium sulfate, and the solvent was distilled off under reduced pressure to obtain compound aa02 as a crude product (137.1 g, quant.).

LCMS (ESI) m/z = 465 $(M+H)^+$
Retention time: 1.05 minutes (analysis condition: SQDAA05)

The synthesis method of compound aa03 will be described in detail. TFA (271 mL) was slowly added to a solution of compound aa02 (137 g, 395 mmol) in DCM (137 mL) under cooling with ice in such a manner that the internal temperature did not exceed 10°C. The obtained mixed solution was stirred at room temperature for 1 hour, and diisopropyl ether (3.4 L) was added in four portions. The precipitated solid was taken by filtration, and dried to obtain compound aa03 ((3s)-3-(9H-fluoren-9-ylmethoxycarbonylamino)-4-oxo-4-pyrrolidin-1-ylbutanoic acid, Fmoc-Asp-pyrro) (108.4 g, 90%).

LCMS (ESI) m/z = 409 $(M+H)^+$
Retention time: 0.83 minutes (analysis condition: SQDAA05)

The synthesis method of compound aa03-resin will be described in detail. A reaction vessel with a filter was charged with 2-chlorotrityl chloride resin (1.60 mmol/g, 100-200 mesh, 1% DVB, 48.7 g) and dehydrated dichloromethane (500 mL), and the reaction vessel was shaken at room temperature for 20 minutes. After nitrogen pressure was applied in the reaction vessel with a filter to remove the dehydrated dichloromethane, a mixed solution of compound aa03 (15.91 g), dehydrated dichloromethane (350 mL), dehydrated methanol (12.63 mL) and diisopropylethylamine (DlPEA) (32.6 mL) was added to the reaction vessel, and the reaction vessel was shaken for 60 minutes. After nitrogen pressure was applied in the reaction vessel to remove the liquid component, a mixed solution of dehydrated dichloromethane (350 mL), dehydrated methanol (97.3 mL) and diisopropylethylamine (DIPEA) (32.6 mL) was added to the reaction vessel, and the reaction vessel was shaken for 1 hour and 30 minutes. After applying nitrogen pressure to remove the liquid component, dichloromethane (350 mL) was added, and the reaction vessel was shaken for 5 minutes. After applying nitrogen pressure in the reaction vessel to remove the liquid component, dichloromethane (350 mL) was added, and the reaction vessel was shaken for 5 minutes. Thus, the washing of the resin with dichloromethane was repeated five times. The resin after washing was dried overnight under reduced pressure to obtain (3S)-3-(9H-fluoren-9-ylmethoxycarbonylamino)-4-oxo-4-pyrrolidin-1-ylbutanoic acid-2-chlorotrityl resin (Fmoc-Asp(O-Trt(2-Cl)-resin)-pyrro, compound aa03-resin, 59.79 g).

For determination of the support ratio, the obtained aa03-resin (12.6 mg) was put in the reaction vessel, DMF (2 mL) was added, and the reaction vessel was shaken at room temperature for 1 hour. DBU (40 μL) was added to the reaction vessel, and the reaction vessel was shaken at 30°C for 30 minutes. After that, DMF (8 mL) was mixed in the reaction vessel, and 1 mL of the reaction solution was collected from the reaction vessel. 1 mL of the collected reaction solution was diluted with DMF (11.5 mL). The absorbance of the obtained diluted solution (294 nm) was measured (SHIMADZU CORPORATION, UV-1600 PC (cell length: 1.0 cm)). The support amount of compound aa03 was calculated as 0.464 mmol/g by measuring the absorbance of dibenzofluorobenzene derived from the Fmoc group of Fmoc-amino acids supported on the resin. Another lot with a different amount supported, which had been similarly synthesized, was also used for peptide synthesis, studies, and the like.

Compound aa04-resin was synthesized in accordance with the description of WO 2013/100132.

[Formula 2]

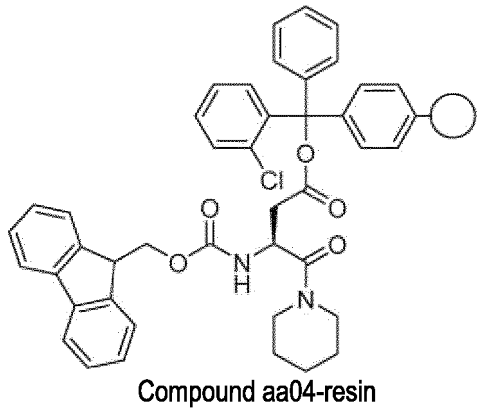

Compound aa04-resin

< Synthesis Method of Fmoc-Peptide-Support Resin >

[Formula 3]

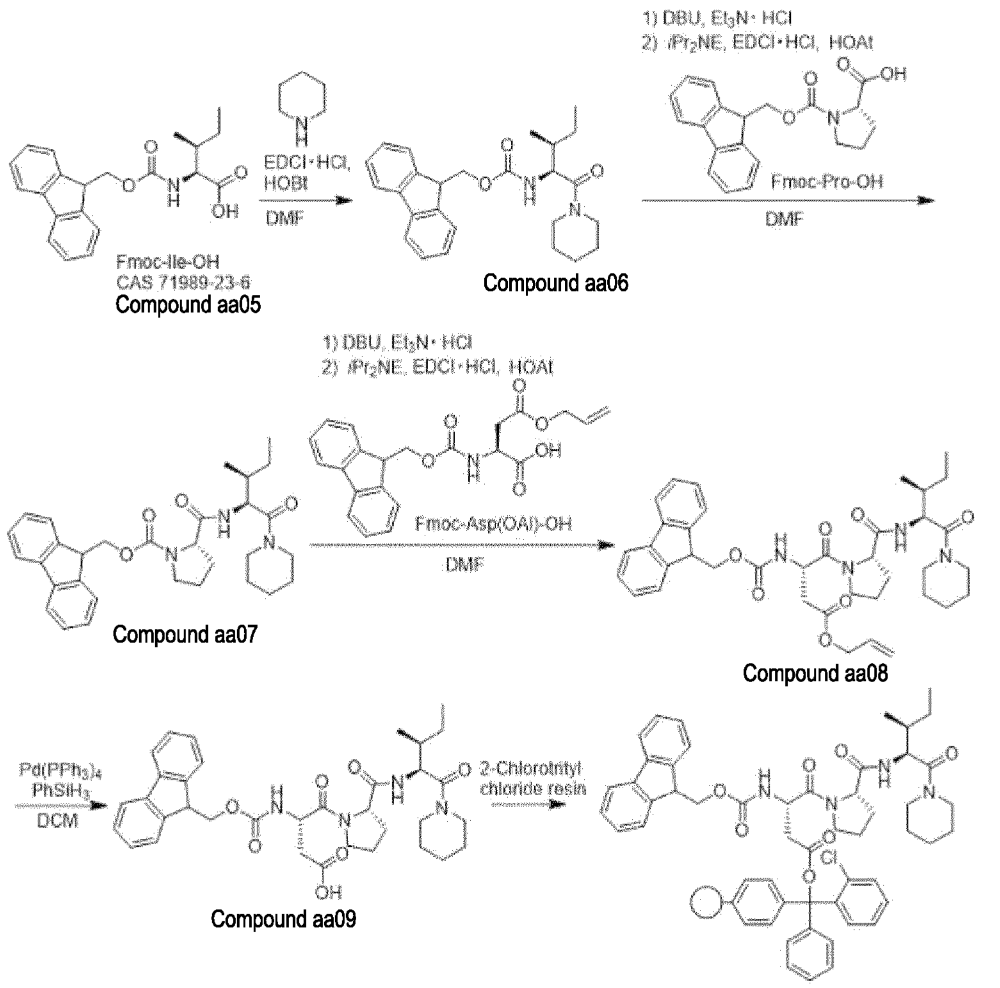


Fmoc-Ile-OH
CAS 71989-23-6
Compound aa05

Compound aa06

Compound aa07

Compound aa08

Compound aa09

Compound aa09-resin

··· (3)

In the formula (3) above, compound aa05 (Fmoc-Ile-OH, CAS: 71989-23-6), Fmoc-Pro-OH (CAS: 71989-31-6),

and Fmoc-Asp(OAl)-OH (CAS: 146982-24-3) were purchased from commercial suppliers. The synthesis method of compound aa06-resin (Fmoc-Ile-pip) will be described in detail. Under nitrogen atmosphere, EDCI·HCl (3.25 g, 17.0 mmol) was mixed with DMF (30 mL), and the mixture was stirred at room temperature for 10 minutes and then cooled to 0°C. HOBt (2.10 g, 3.25 mmol) and compound aa05 (Fmoc-Ile-OH, CAS: 71989-23-6) (15.00 g, 14.15 mmol) were mixed sequentially, and the mixture was stirred at 0°C for 1 hour. To the obtained reaction solution, piperidine (1.27 g, 14.9 mmol) was slowly added, and the mixture was stirred at 0°C for 1 hour. To this reaction solution, ethyl acetate (10 v/w) and 0.5 mol/L hydrochloric acid water (10 v/w) were added, and the organic phase was separated. The obtained organic phase was washed sequentially with 0.5 mol/L hydrochloric acid water, water, 5% aqueous sodium carbonate solution, and saturated aqueous sodium chloride solution/water (1/1 (v/v)). The washed organic phase was dried over anhydrous sodium sulfate, and the solvent was distilled off under reduced pressure to obtain compound aa06 (Fmoc-Ile-pip) as a crude product (6.11 g).

LCMS (ESI) m/z = 421 $(M+H)^+$
Retention time: 0.98 minutes (analysis condition: SQDFA05)

The synthesis method of compound aa07 (Fmoc-Pro-Ile-pip) will be described in detail. To a solution of compound aa06 (Fmoc-Ile-pip) in DMF (50 mL), 1,8-diazabicyclo[5.4.0]-7-undecene (DBU, 2.21 g, 14.53 mmol) was added, and then the mixture was stirred at room temperature for 10 minutes. To this reaction solution, triethylamine hydrochloride (2.00 g, 14.53 mmol), N,N-diisopropylethylamine (DIPEA, 2.54 mL, 14.53 mmol), Fmoc-Pro-OH (CAS: 71989-31-6) (4.90, 14.53 mmol), EDCI·HCl (3.90 g, 20.35 mmol), and HOAt (2.37 g, 17.43 mmol) were sequentially added, and the mixture was stirred at room temperature for 1 hour. To this reaction solution, ethyl acetate (10 v/w) and 0.5 mol/L hydrochloric acid water (10 v/w) were added, and the organic phase was separated. The obtained organic phase was washed sequentially with 0.5 mol/L hydrochloric acid water, water, 5% aqueous sodium carbonate solution, and saturated aqueous sodium chloride solution/water (1/1 (v/v)). The washed organic phase was dried over anhydrous sodium sulfate, and the solvent was distilled off under reduced pressure to obtain compound aa07 as a crude product. To the obtained crude product, DMSO (5 mL) and ethyl acetate (20 mL) were added, and the obtained solution was filtered, and then ethyl acetate was distilled off to some extent under reduced pressure. The obtained residue was purified by reverse-phase medium pressure column chromatography (0.1% formic acid-containing acetonitrile/water) to obtain compound aa07 (Fmoc-Pro-Ile-pip) (2.41 g, 32%, 97% purity [peak area percentage]).

LCMS (ESI) m/z = 518 $(M+H)^+$
Retention time: 0.94 minutes (analysis condition: SQDFA05)

The synthesis method of compound aa08 (Fmoc-Asp(OAl)-Pro-Ile-pip) will be described in detail. To a solution of compound aa07 (Fmoc-Pro-Ile-pip)(2.41 g, 4.66 mmol) in DMF (23 mL), 1,8-diazabicyclo[5.4.0]-7-undecene (DBU, 0.702 mL, 4.66 mmol) was added, and then the mixture was stirred at room temperature for 10 minutes. To this reaction solution, triethylamine hydrochloride (0.641 g, 4.66 mmol), N,N-diisopropylethylamine (DIPEA, 0.813 mL, 4.66 mmol), Fmoc-Asp(OAl)-OH (CAS: 146982-24-3) (2.41 g, 4.66 mmol), EDCI·HCl (1.25 g, 6.52 mmol), HOAT (0.760 g, 5.59 mmol) were sequentially added, and the mixture was stirred at room temperature for 1 hour and 30 minutes. To this reaction solution, ethyl acetate (10 v/w) and 0.5 mol/L hydrochloric acid water (10 v/w) were added, and the organic phase was separated. The obtained organic phase was washed sequentially with 0.5 mol/L hydrochloric acid water, water, 5% aqueous sodium carbonate solution, and saturated aqueous sodium chloride solution/water (1/1 (v/v)). The washed organic phase was dried over anhydrous sodium sulfate, and the solvent was distilled off under reduced pressure to obtain compound aa08 as a crude product. The obtained crude product was dissolved in DMSO and purified by reverse-phase medium pressure column chromatography (0.1% formic acid-containing acetonitrile/water) to obtain compound aa08 (Fmoc-Asp(OAl)-Pro-Ile-pip) (1.71 g, 55%, 84% purity [peak area percentage]).

LCMS (ESI) m/z = 674 $(M+H)^+$
Retention time: 2.94 minutes (analysis condition: SQDA05 long)

The synthesis method of compound aa09 will be described in detail. To a solution of compound aa08 (Fmoc-Asp(OAl)-Pro-Ile-pip) (1.71 g, 84% purity) in dichloromethane (5.08 mL), tetrakis(triphenylphosphine) palladium(0) (CAS: 14221-01-3, tetrakis(triphenylphosphine) palladiumu(0), Pd $(PPhe_3)_4$, 0.294 g, 0.254 mmol) and phenylsilane (CAS: 694-53-1, phenylsilane, 0.313 mL, 2.54 mmol) were added, and the mixture was stirred at 0°C for 15 minutes. The solvent in the obtained reaction solution was distilled off under reduced pressure, and purification by reverse-phase medium pressure column chromatography (0.1% formic acid-containing acetonitrile/water) was performed to obtain compound aa09 (Fmoc-Asp-Pro-Ile-pip) (1.11 g, 69%, 99% purity [peak area percentage]).

LCMS (ESI) m/z = 633 $(M+H)^+$
Retention time: 0.80 minutes (analysis condition: SQDFA05)

Compound aa09-resin (Fmoc-Asp(O-Trt(2-Cl)-resin)-Pro-Ile-pip) was synthesized in accordance with the description of WO 2013/100132.

< General Synthesis Method of Peptide >

The peptide compounds used in Examples and Reference Examples were synthesized by solid phase synthesis. Examples of the solid phase synthesis method can include Amino Acids, 2018, 50, 39-68 or Solid phase peptide synthesis (published by Bachem Holding AG) [search date: December 20, 2022], Internet <URL: https://www.bachem.com/wpfd_file/solid-phase-peptide-synthesis/>. In Examples and Reference Examples, reference was made to the peptide synthesis method by Fmoc method described in WO 2013/100132 or WO 2018/225864. In Examples and Reference Examples, the extension of peptides was performed by the basic synthesis method of cyclic peptide shown in Figure 1. The basic synthesis method of cyclic peptide shown in Figure 1 comprises the following five-step processes:

1) an extension reaction of a peptide from the N-terminus of an amino acid by Fmoc method using a substance in which a carboxyl group of Asp side chain or a carboxyl group of peptide main chain was supported on 2-chlorotrityl resin (a peptide chain extension reaction using an Fmoc group-protected amino acid as a raw material),
2) an excising process of the peptide from the 2-chlorotrityl resin;
3) amide cyclization by condensation of the carboxyl group of the Asp side chain or the carboxyl group of the peptide main chain generated by the excising process off from the 2-chlorotrityl resin and the amino group at the N-terminus of the peptide chain (triangular unit)
4) optionally, deprotection of the protecting group of the side chain functional group contained in the peptide chain; and
5) purification of the compound by preparative HPLC. In Examples and Reference Examples, unless otherwise stated, the synthesis of cyclic peptides was performed by the basic synthesis method of cyclic peptide shown in Figure 1.

< Synthesis Method of Peptide Compound Containing N-Alkylamino Acid >

Peptide compounds containing N-alkylamino acids can be synthesized according to the "General synthesis method of peptide" described in Examples and Reference Examples using N-alkylamino acids protected by Fmoc groups shown in Tables 2 to 4 as raw materials.

< 1. Solid-Phase Synthesis of Peptide by Automatic Synthesizer >

Compound 1 was synthesized by the method described in International Publication No. WO 2013/100132 or WO 2018/225864. Detailed operating procedures of the peptide synthesizer (Multipep RS; CEM Corporation (formerly Intavis AG)) were in accordance with the manual attached to the synthesizer. The abbreviations, amino acid structural formulas, and formal names of each amino acid residue that constitutes Compound 1 (cyclic peptide) and Compound 6 (cyclic peptide) are shown in Table 5 below.

[Table 5]

| Abbreviation | Amino acid structural formula | Name | Abbreviation | Amino acid structural formula | Name |
|---|---|---|---|---|---|
| Val | 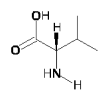 | (2S)-2-Amino-3-methyl-butanoic acid | MeGly | 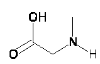 | 2-(Methylamino)acetic acid |
| Thr | 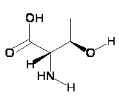 | (2S,3R)-2-Amino-3-hydroxybutanoic acid | MeAla | 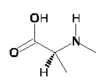 | (2S)-2-(Methylamino) propanoic acid |
| Ser(nPr) | 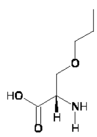 | (2S)-2-Amino-3-propoxypropanoic acid | Leu | 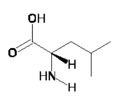 | (2S)-2-Amino-4-methyl-pentanoic acid |

(continued)

| Abbreviation | Amino acid structural formula | Name | Abbreviation | Amino acid structural formula | Name |
|---|---|---|---|---|---|
| Ser | 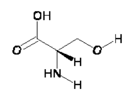 | (2S)-2-Amino-3-hydroxypropanoic acid | Ile | 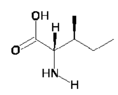 | (2S,3S)-2-Amino-3-methylpentanoic acid |
| Pro | 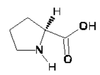 | (2S)-Pyrrolidine-2-carboxylic acid | D-Val | 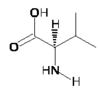 | (2R)-2-Amino-3-Methylbutanoic acid |
| Phe | 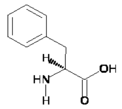 | (2S)-2-Amino-3-phenylpropanoic acid | D-MeAla | 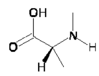 | (2R)-2-(Methylamino) propanoic acid |
| MeVal | 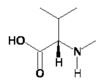 | (2S)-3-Methyl-2-(methylamino)butanoic acid | Cha | 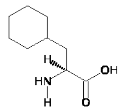 | (2S)-2-Amino-3-cyclohexylpropanoic acid |
| MePhe(4-F) | 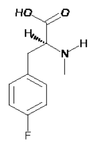 | (2S)-3-(4-Fluorophenyl)-2-(methylamino) propanoic acid | bMeAla | 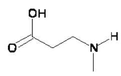 | 3-(Methylamino)propanoic acid |
| MePhe(34-F2) | 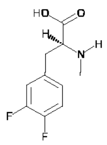 | (2S)-3-(3,4-Difluorophenyl)-2-(methylamino) propanoic acid | Ala(cPent) | 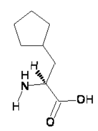 | (2S)-2-Amino-3-cyclopentylpropanoic acid |
| MePhe | 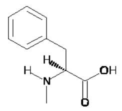 | (2S)-2-(Methylamino)-3-phenylpropanoic acid | Ala | 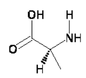 | (2S)-2-Aminopropanoic acid |
| MeLeu | 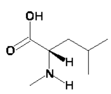 | (2S)-4-Methyl-2-(methylamino)pentanoic acid | Thr(THP) | 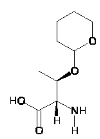 | (2S,3R)-2-Amino-3-(oxan-2-yloxy)butanoic acid |
| MeIle | 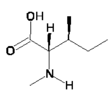 | (2S,3S)-3-Methyl-2-(methylamino) pentanoic acid | Ser(THP) | 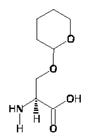 | (2S)-2-Amino-3-(oxan-2-yloxy)propanoic acid |

< 1-1. Peptide Extension Reaction from N-terminus of Amino Acids by Fmoc Method >

Fmoc-amino acid (concentration in solution 1: 0.3 to 0.6 mol/L) constituting a cyclic peptide that is a purification target, and HOAt, oxyma or HOOBt (concentration in solution 1: 0.375 mol/L), which are activators of the carboxyl group, were dissolved in NMP or NMP/DMF (1/1) to prepare solution 1. N,N'-diisopropylcarbodiimide (DIC) (10%v/v) was mixed with N,N-dimethylformamide (DMF) to prepare solution 2.

When Fmoc-Ser(THP)-OH or Fmoc-Thr(THP)-OH was used as the Fmoc-amino acid having a THP group in the side chain, solution 1 was prepared using oxyma as the activator of the carboxyl group, and Molecular Sieves 4A1/8 (FUJIFILM Wako Pure Chemical Corporation) or Molecular Sieves 4A1/16 (FUJIFILM Wako Pure Chemical Corporation) were added for peptide synthesis.

2-Chlorotrityl resin (100 mg) bound with a side chain carboxyl group of aspartic acid, the N-terminus of which was protected with an Fmoc group, was put in the reaction vessel with a filter, and set in the peptide synthesizer. To this resin

(100 mg), dichloromethane (DCM) (0.8 mL) was added, and the mixture was allowed to stand for about 1 hour to swell the resin. The solution was then discharged from the reaction vessel with a filter. Solution 1 and solution 2 were set in the peptide synthesizer, and automated synthesis with the peptide synthesizer was started.

< De-Fmoc Step >

To the reaction vessel with a filter containing the swollen resin, a solution of diazabicycloundecene (DBU) in DMF (2%v/v, 0.7 mL) was added to deprotect the Fmoc group at the N-terminus at room temperature. The reaction was performed for 4.5 minutes in deprotection at the first residue, and for 10 minutes in deprotection at the second and subsequent residues, and the solution was then discharged from the reaction vessel with a filter. DMF (0.7 mL) was added thereto to wash the resin and allowed to stand for 5 minutes, and the solution was discharged from the reaction vessel with a filter. This resin-washing step was repeated three more times to obtain a resin in which the N-terminal Fmoc group of the amino acid or peptide attached to the resin was removed to be an amino group.

< Extension Step >

Subsequently, solution 1 (0.3 mL per reaction vessel) and solution 2 (0.36 mL per reaction vessel) were mixed with a mixing vial of the synthesizer, then the mixture was added to the resin after the deprotection treatment, and the filtered reaction vessel was heated to 40°C. The condensation reaction between the amino group on the resin and the Fmoc-amino acid was performed for 2.5 hours. After the reaction, the solution was then discharged from the reaction vessel with a filter. Subsequently, the resin after the reaction was washed 3 times with DMF (0.7 mL). This condensation reaction of the Fmoc-amino acid subsequent to the deprotection reaction of the Fmoc group was set to one cycle, and this cycle was repeated to extend a peptide on the resin surface. After the extension of the last amino acid, the de-Fmoc step was not performed, and the resin was washed four times with DCM (1.0 mL per reaction vessel).

< Synthesis of Fmoc-Pro-MeAla-Cha-Leu-Pro-MePhe(34-F2)-Ala(cPent)-MePhe(4-F)-MeVal-Asp(O-Trt(2-Cl)-resin)-pyrro (Compound 1) >

[Formula 4]

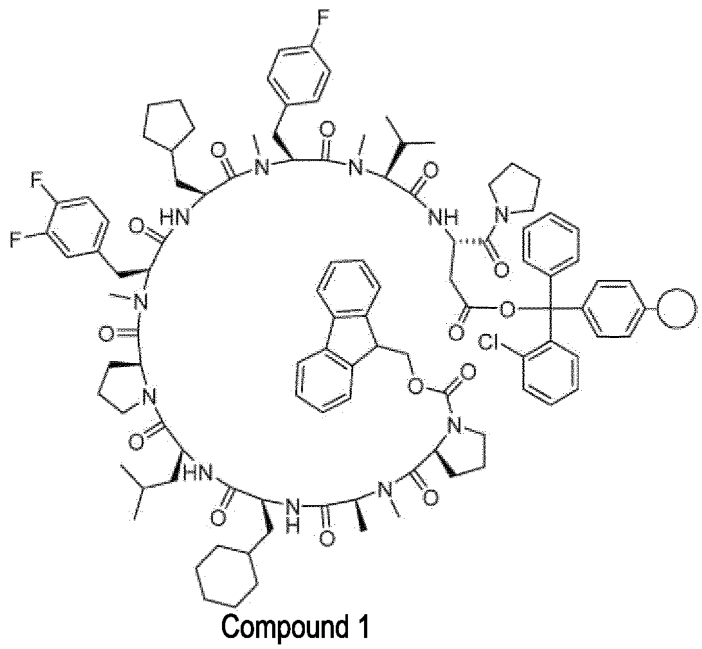

Compound 1

Fmoc-Asp(O-Trt(2-Cl)-resin)-pyrro (compound aa03-resin, 0.497 mmol/g, 100 mg, 48 reaction vessels with a filter each containing 0.0497 mmol of the same), Fmoc-MeVal-OH, Fmoc-MePhe(4-F)-OH, Fmoc-Ala(cPent)-OH, Fmoc-MePhe(34-F2)-OH, Fmoc-Pro-OH, Fmoc-Leu-OH, Fmoc-Cha-OH, Fmoc-MeAla-OH, and Fmoc-Pro-OH were used as raw materials. Compound 1 was prepared by the peptide extension reaction by Fmoc method described above.

< 1-2. Synthesis of Compound 6 (3S,6S,9S,12S,18S,22S,25S,28S,31S,34S,37S)-6-(cyclohexylmethyl)-31-(cyclopentylmethyl)-34-[(3,4-difluorophenyl)methyl]-28-[(4-fluorophenyl)methyl]-3-isobutyl-25-isopropyl-9,10,18,19,26,29,35-heptamethyl-22-(pyrrolidin-1-carbonyl)-1,4,7,10,16,19,23,26,29,32,35- undecazatricyclo[35.3.0.012,16]tetracontan-2,5,8,11,17,20,24,27,30,33,36-undecaone >

[Formula 5]

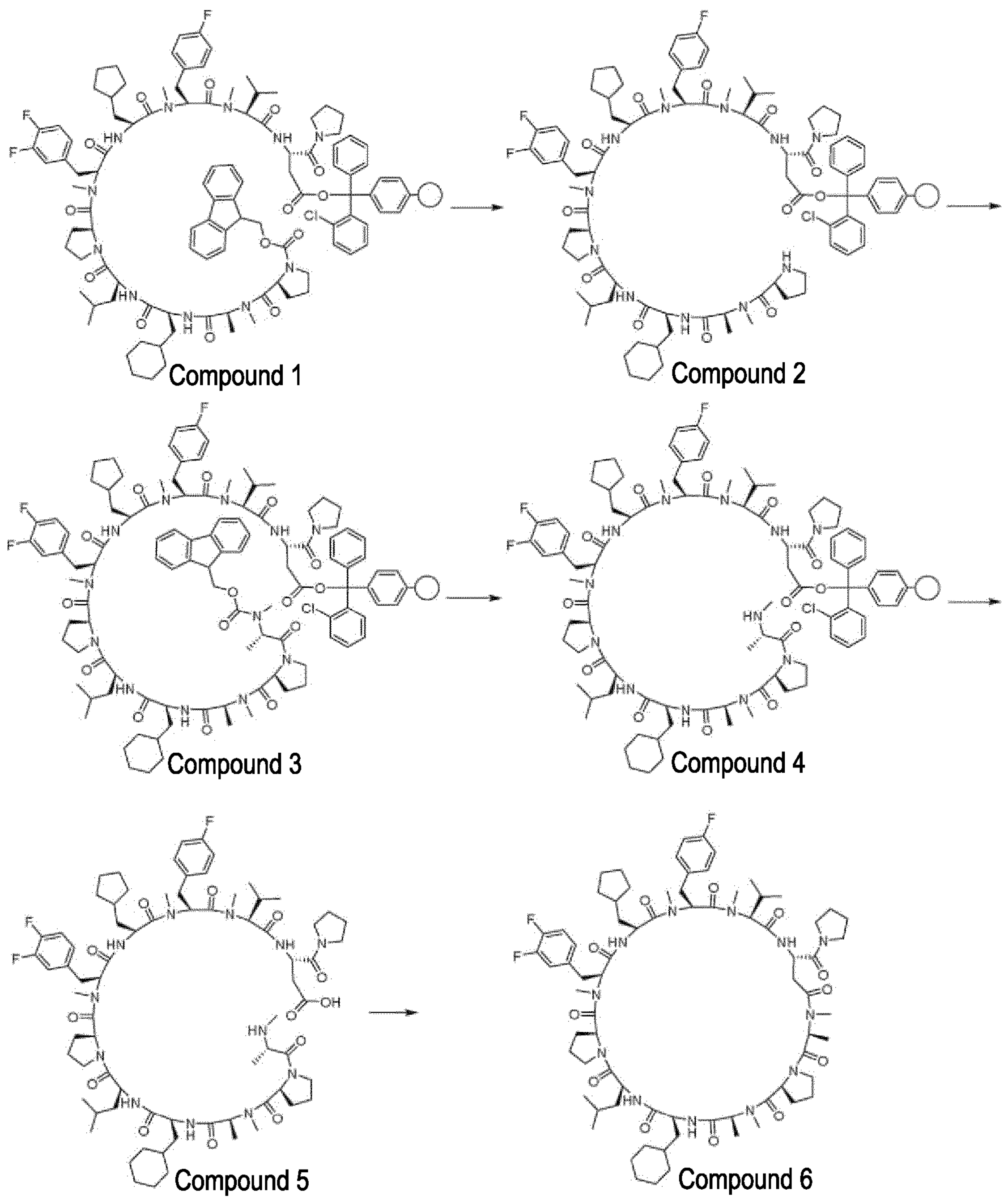

Compound 1

Compound 2

Compound 3

Compound 4

Compound 5

Compound 6

< De-Fmoc Step >

Fmoc-Pro-MeAla-Cha-Leu-Pro-MePhe(34-F2)-Ala(cPent)-MePhe(4-F)-MeVal-Asp(O-Trt(2-Cl)-resin)-pyrro (Compound 1) was washed by adding toluene (1 mL per reaction vessel), and allowed to stand for 5 minutes, and then the solution was discharged from the reaction vessel with a filter. This washing step of the resin was repeated twice more to

obtain a resin swollen with toluene.

A solution of diazabicycloundecene (DBU) in toluene (2%v/v, 0.7 mL per reaction vessel) was added to a solid phase reaction vessel containing the resin swollen with toluene to deprotect the N-terminal Fmoc group at room temperature. After the reaction for 10 minutes, the solution was discharged from the reaction vessel with a filter. Toluene (0.7 mL per reaction vessel) was added thereto to wash the resin and allowed to stand for 5 minutes, and the solution was discharged from the reaction vessel with a filter. The washing step of the resin with toluene was repeated once more, and then DCM (0.7 mL per reaction vessel) was added thereto to wash the resin and allowed to stand for 5 minutes, and the solution was discharged from the reaction vessel with a filter. The washing step of the resin with DCM was repeated once more to obtain H-Pro-MeAla-Cha-Leu-Pro-MePhe(34-F2)-Ala(cPent)-MePhe(4-F)-MeVal-Asp(O-Trt(2-Cl)-resin)-pyrro (Compound 2) in which the N-terminal Fmoc group of the peptide attached to the resin was removed to be an amino group.

< Extension Step >

To the resin obtained as described above, a solution obtained by mixing a solution of Fmoc-MeAla-OH (4 molar equivalents) in DCM (0.25 mL), a solution of [ethylcyano(hydroxyimino)acetato-*O*$^2$)tri 1-pyrrolidinylphosphonium hexafluorophosphate (PyOxim, 153433-21-7, 4 molar equivalents) in DCM (0.25 mL), and DIPEA (6 molar equivalents) and allowing the mixture to stand for about 1 to 2 minutes was added, and the mixture was shaken at room temperature for 3 hours. The number of molar equivalents was calculated based on a value obtained by multiplying the ratio of support of amino acid on the resin used as a raw material (mmol/g) by the amount of the resin used (usually 100 mg). The solution was discharged from the reaction vessel with a filter, and then the resin was washed 4 times with DMF (0.7 mL per reaction vessel) and 4 times with DCM (0.7 mL per reaction vessel) and dried to obtain Fmoc-MeAla-Pro-MeAla-Cha-Leu-Pro-MePhe(34-F2)-Ala(cPent)-MePhe(4-F)-MeVal-Asp(O-Trt(2-Cl)-resin)-pyrro (Compound 3). After completion of the peptide extension, a solution of diazabicycloundecene (DBU) in DMF (2%v/v, 0.7 mL per reaction vessel) was added to the resin and reacted for 15 minutes to perform a deprotection reaction of the Fmoc group, and then the solution was discharged from the reaction vessel with a filter. The obtained resin was washed 4 times with DMF (0.7 mL) and 4 times with DCM (0.7 mL) to obtain H-MeAla-Pro-MeAla-Cha-Leu-Pro-MePhe(34-F2)-Ala(cPent)-MePhe(4-F)-MeVal-Asp(O-Trt(2-Cl)-resin)-pyrro (Compound 4).

< Excision Step of Extended Peptide from Resin >

To the resin (Compound 4) obtained by the above method, 2,2,2-trifluoroethanol (TFE)/DCM (1/1, v/v, 2 mL) containing 0.75% (v/v) DIPEA was added, and a reaction was performed at room temperature for 2 hours to excise the peptide chain from the resin. After the reaction, the solution in the tube was recovered from the reaction vessel with a filter. The operation of adding 2,2,2-trifluoroethanol (TFE)/DCM (1/1, v/v, 1 mL) to the remaining resin and recovering the solution from the reaction vessel with a filter was performed twice. After all the obtained excision solutions were mixed and DMF (4 mL) or 1,2-dichloroethane (4 mL) was mixed, the solvent was distilled off under reduced pressure by a high throughput centrifugal evaporator (HT-12) manufactured by Genevac Ltd. to obtain H-MeAla-Pro-MeAla-Cha-Leu-Pro-MePhe(34-F2)-Ala(cPent)-MePhe(4-F)-MeVal-Asp-pyro (Compound 5).

< Cyclization Method of Excised Peptide >

The residue obtained by the above method was dissolved in a mixed solution of DMF (8 mL) and DCM (8 mL), and a DMF solution (0.5 M, 1.5 molar equivalents) of (1-cyano-2-ethoxy-2-oxoethylideneaminooxy)dimethylamino-morpholino-carbenium hexafluorophosphate (COMU) and DIPEA (1.8 molar equivalents) were added, and the mixture was stirred at room temperature for 30 minutes to perform a condensation cyclization reaction between the amino group at the N-terminus and the carboxyl group at the C-terminus. The number of molar equivalents was calculated based on a value obtained by multiplying the ratio of support of amino acid on the resin used as a raw material (mmol/g) by the amount of the resin used (usually 100 mg). The production of the cyclic peptide of interest was confirmed by LC/MS measurement (SQ Detector 2 manufactured by Waters Corporation), and then the reaction solution was subjected to a high throughput centrifugal evaporator (HT-12) manufactured by Genevac Ltd. to distill off the solvent under reduced pressure.

< Purification Method of Cyclic Peptide >

DMSO was added to the residue obtained by the above method (48 filtered reaction vessels), and the mixture was purified by reverse-phase medium pressure column chromatography (0.1% formic acid-containing acetonitrile/water) in one lot to obtain compound 6 (3S,6S,9S,12S,18S,22S,25S,28S,31S,34S,37S)-6-(cyclohexylmethyl)-31-(cyclopentylmethyl)-34-[(3,4-difluorophenyl)methyl]-28-[(4-fluorophenyl)methyl]-3-isobutyl-25-isopropyl-9,10,18,19,26,29,35-heptamethyl-22-(pyrrolidin-1-carbonyl)-1,4,7,10,16,19,23,26,29,32,35-undecazatricyclo[35.3.0.012,16]tetracontan-2,5,8,11,17,20,24,27,30,33,36-undecaone) (2.3 g, 68%, 84% purity [peak area percentage]). In the conditions of LC used for purity analysis (purity analysis condition A), isomer 1 and isomer 2, which are isomers of compound 6, were confirmed from the results of precision mass spectrometry before and after the retention time of compound 6 (around 11.1 minutes), which were retention times of around 10.8 minutes and around 11.4 minutes, respectively. From the analysis of the peak area of LC, it was also confirmed that the isomer 1 of compound 6 observed near the retention time of 10.8 minutes (relative retention time: 0.972) was 2.0% in peak area percentage, and the isomer 2 of compound 6 observed near the retention time of 11.4 minutes (relative retention time: 1.03) was 6.8% in peak area percentage. The isomer 1 and/or isomer 2 are presumed to be diastereomers of compound 6, based on the results of the synthesis process and precision mass spectrometry. The relative retention time refers to the retention time of each peak divided by the retention time of the target (in this case, compound 6).

[Formula 6]

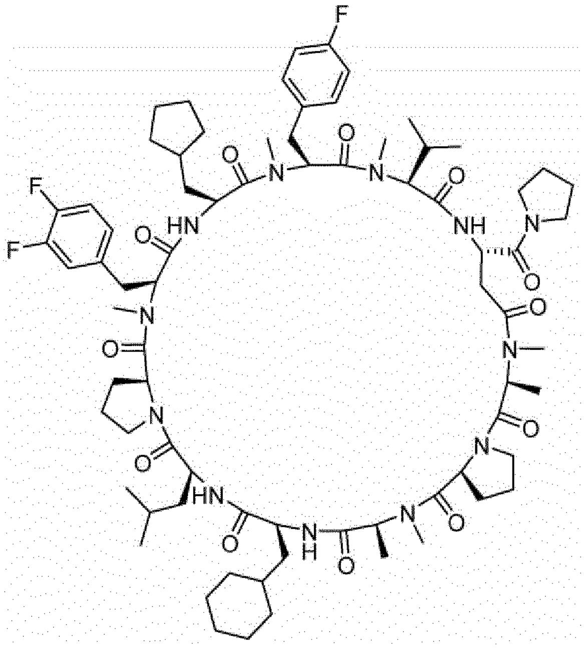

Recovered amount, Yield: 2.3 g, 68%
Purity: 84.0%
LCMS (ESI) m/z = 1426 $(M-H)^-$
Retention time: 0.85 minutes (analysis condition SQDAA50)

HPLC conditions for purity analysis (purity analysis condition A)

Column: ACQUITY UPLC Peptide CSH C18, 2.1 x 150 mm, 1.7 μm
Solvent: A) 0.1% FA in $H_2O$ B) 0.1% FA in $CH_3CN$
Gradient program: 50% → 100% B (15 minutes) → 100% B (20 minutes) → 50% B (20.1 minutes)
Column temperature: 50°C
Flow rate: 0.3 mL/min
Autosampler temperature: 20°C
Sample concentration: about 0.3 mg/mL in $CH_3CN$
Injection volume: 5 μL
UV detection: 220 nm
System: Nexera X2 (SHIMADZU CORPORATION)

< 1-3. Synthesis of Compound 9 (S)-1-((2S,5S,8S,11S,14S,17S,20S,23S)-14,20-dibenzyl-8-((S)-sec-butyl)-5,17-diisobutyl-11-isopropyl-2,7,13,19-tetramethyl-3,6,9,12,15,18,21,25-octaoxy-1,4,7,10,13,16,19,22-octaazacyclopentacosan-23-carbonyl)-N-((2S,3S)-3-methyl-1-oxo-1-(piperidin-1-yl) pentan-2-yl)pyrrolidin-2-carboxamide) >

Synthesis and purification were performed using compound aa09-resin (Fmoc-Asp(O-Trt(2-Cl)-resin)-Pro-Ile-pip) in the same manner as compound 6 as shown in Formula (7) below to obtain compound 9 (246 mg, 47%, 93.0% purity [peak area percentage]).

[Formula 7]

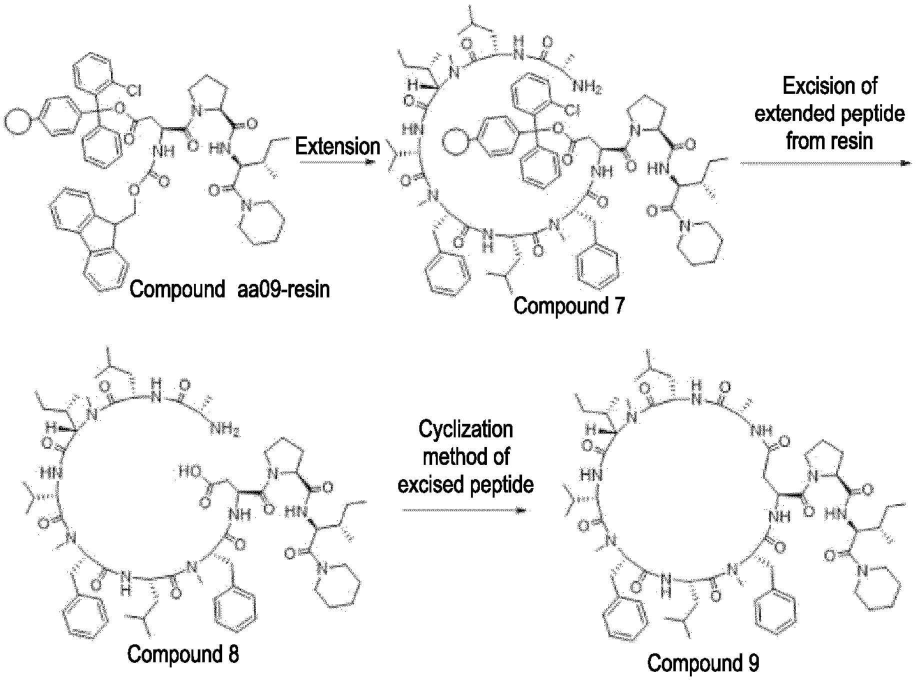


Compound aa09-resin

Compound 7

Compound 8

Compound 9

··· (7)

Recovered amount, Yield: 246 mg, 47%
Purity: 93.0%
LCMS (ESI) m/z = 1237 $(M-H)^-$
Retention time: 0.81 minutes (analysis condition SQDFA50)
HPLC conditions for purity analysis: Purity analysis condition C

< 1-4. Synthesis of compound 11 ((3S,6S,9S,12S,18S,21S,24S,27S,30R,34S)-6,27-dibenzyl-18-((R)-1-hydroxyethyl)-3,12,21,24-tetraisobutyl-30-isopropyl-4,7,13,16,22,28-hexamethyl-34-(piperidine-1-carbonyl)-9-(propoxymethyl)-1,4,7,10,13,16,19,22,25,28,31-undecaazacyclotetratriacontan-2,5,8, 11,14, 17,20,23,26,2932-undecaone) >

Compound 10 ((3S,6S,9S,12S,18S,21S,24S,27S,30R,34S)-6,27-dibenzyl-3,12,21,24-tetraisobutyl-30-isopropyl-4,7,13,16,22,28-hexamethyl-34-(piperidin-1-carbonyl)-9-(propoxymethyl)-18-((1R)-1-((tetrahydro-2H-pyran-2-yl) oxy) ethyl)-1,4,7,10,13,16,19,22,25,28,31-undecaazacyclotetratriacontan-2,5,8,11,14,17,20,23,26,29,32-undecaone) was synthesized using compound aa04-resin (Fmoc-Asp(O-Trt(2-Cl)-resin)-pip) in accordance with the description of WO 2021/090855.

< Deprotection of Protecting Group of Side Chain Functional Group of Cyclic Peptide >

In the sequence synthesized using Fmoc-protected amino acids having THP-protected hydroxyl groups in the side chain, for example, Fmoc-Thr(THP)-OH and Fmoc-Ser(THP)-OH, 4 mL of a solution of hydrogen sulfate tetramethylammonium (0.05 M) in 1,1,1,3,3,3-hexafluoroisopropyl alcohol (HFIP) (containing 2% (v/v) triisopropyl silane (TIPS), 1% (v/v) 1,2-dichloroethane) was added to the residue obtained above (14 reaction vessels). The residue was dissolved and allowed to stand at room temperature for 4 hours to deprotect the THP group. After confirming the completion of the reaction by LC/MS (SQ Detector 2 manufactured by Waters Corporation), diisopropylethylamine

(DIPEA) (70 μL) was added to the reaction solution (14 reaction vessels), and the solvent was distilled off under reduced pressure by a high throughput centrifugal evaporator (HT-12) manufactured by Genevac Ltd. to obtain compound 11 (372 mg, 44%, 92.3% purity [peak area percentage]).

[Formula 8]

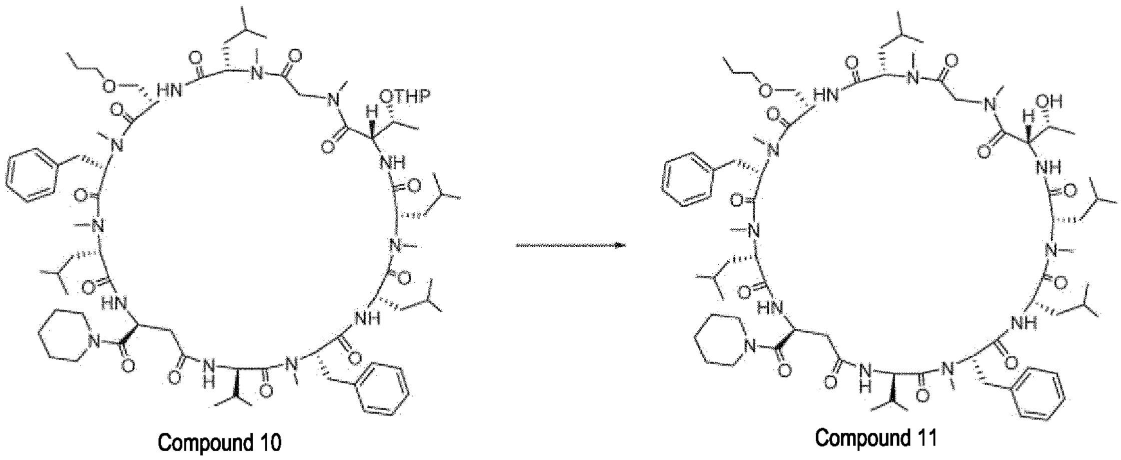

Compound 10 Compound 11

Recovered amount, Yield: 372 mg, 44%
Purity: 92.3%
LCMS (ESI) m/z = 1398 $(M-H)^-$
Retention time: 0.82 minutes (analysis condition SQDFA50)
HPLC conditions for purity analysis: Purity analysis condition C

To a cyclic peptide compound synthesized in accordance with the production method of compound 6 (14 reaction vessels per compound), 4 mL of a solution of hydrogen sulfate tetramethylammonium (0.05 M) in 1,1,1,3,3,3-hexafluoroisopropyl alcohol (HFIP) (containing 2% (v/v) triisopropyl silane (TIPS), 1% (v/v) 1,2-dichloroethane) was added (14 reaction vessels per compound). The residue was dissolved and allowed to stand at room temperature for 4 hours to deprotect the THP group. After confirming the completion of the reaction by LC/MS (SQ Detector 2 manufactured by Waters Corporation), diisopropylethylamine (DIPEA) (70 μL) was added to the reaction solution, and the solvent was distilled off under reduced pressure by a high throughput centrifugal evaporator (HT-12) manufactured by Genevac Ltd. The obtained residues were collected in one vessel for each compound, and each compound was purified by reverse-phase medium pressure column chromatography (0.1% formic acid-containing acetonitrile/water) to obtain compounds 12 to 15.

< 1-5. Synthesis of Compound 12 ((3S,6S,9S,12S,16S,19S,25S,28S,31S,36aS)-25,28-benzyl-19-((R)-1-hydroxyethyl)-3,31-diisobutyl-9-isopropyl-2,6,8,15,16,21,24,27,30-nonamethyl-12-(piperidine-1-carbonyl)tetracosahydropyrrolo[2,1-l][1,4,7,10,13,16,19,22,25,28,31]undecaazacyclotetratriacontin-1,4,7,10,14,17,20,23,26,29,32(11H)-undecanone) >

[Formula 9]

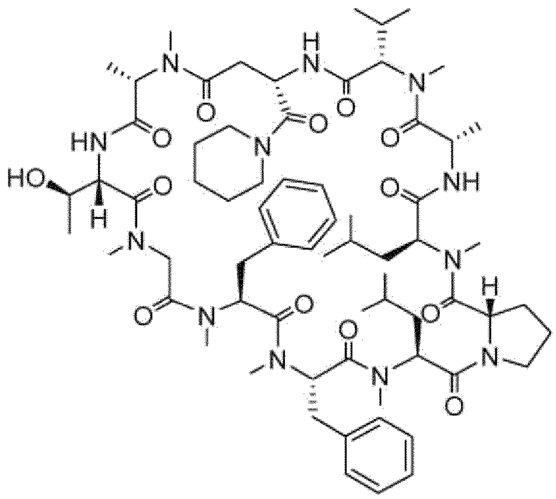

Compound 12

Recovered amount, Yield: 233 mg, 30%
Purity: 85.7%
LCMS (ESI) m/z = 1296 $(M-H)^-$
Retention time: 0.54 minutes (analysis condition SQDFA50)
HPLC conditions for purity analysis: Purity analysis condition C

< 1-6. Synthesis of Compound 13 ((3S,6S,9S,12S,15S,18S,21S,27S,30S,33S,41S)-6,9-dibenzyl-15-((S)-sec-butyl)-27-((R)-1-hydroxyethyl)-12,18,21,30,33-pentaisobutylbutyl-3,7,13,19,22,25,31,38-octamethyl-41-(piperidine-1-carbonyl)-1,4,7,10,13,16,19,22,25,28,31,34,38-tridecaazacyclohentetracontan-2,5,8,11,14,17,20,23,26,29,32,35,39-tridecaone) >

[Formula 10]

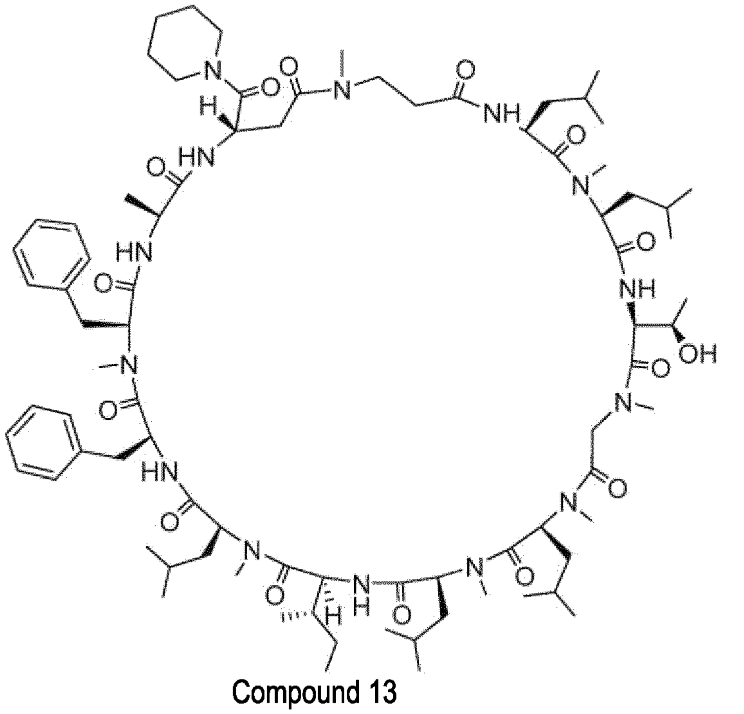

Compound 13

Recovered amount, Yield: 388 mg, 42%
Purity: 92.3%
LCMS (ESI) m/z = 1552 $(M-H)^-$
Retention time: 0.74 minutes (analysis condition SQDFA50)
HPLC conditions for purity analysis: Purity analysis condition C

< 1-7. Synthesis of Compound 14 ((3S,6S,9S,12S,15S,18S,21S,24S,27S,30S,33S,41S)-6,24-dibenzyl-33-((S)-sec-butyl)-21-((R)-1-hydroxymethyl)-3,9.12.18,30-pentaisobutyl-4,10,13,15,16,19,25,27,28,31,38-undecamethyl-41-(piperidine-1-carbonyl)-1,4,7,10,13,16,19,22,25,28,31,34,38-tridecaazacyclohentetracontan-2,5,8,11,14,17,20,23,26,29,32,35,39-tridecaone) >

[Formula 11]

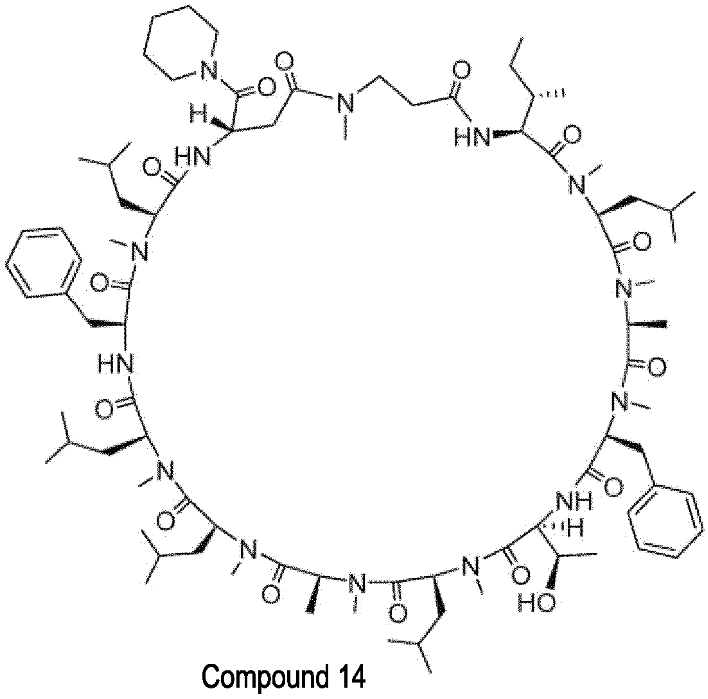

Compound 14

Recovered amount, Yield: 203 mg, 21%
Purity: 84.3%
LCMS (ESI) m/z = 1594 (M-H)[-]
Retention time: 0.81 minutes (analysis condition SQDFA50)
HPLC conditions for purity analysis: Purity analysis condition C

< 1-8. Synthesis of Compound 15 ((3S,6S,9S,12S,15S,18S,21S,24S,27S,30S,33S,36S,39R,43S)-21,36-dibenzyl-27-((S)-sec-butyl)-6-((R)-1-hydroxyethyl)-9-(hydroxymethyl)-3,12,18,24,30-pentaisobutyl-4,15,16,19,25,31,33,34,37,39,40-undecamethyl-43-(piperidine-1-carbonyl)-1,4,7,10,13,16,19,22,25,28,31,34,37,40-tetradecaazacyclotritetracontan-2,5,8,11,14,17,20,23,26,29,32,35,38,41-tetradecaon) >

[Formula 12]

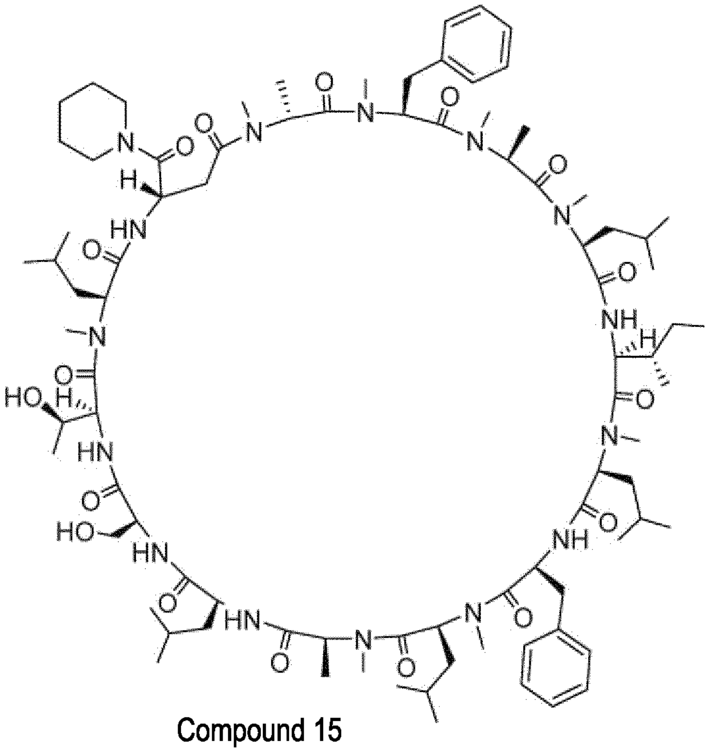

Compound 15

Recovered amount, Yield: 388 mg, 42%
Purity: 89.5%
LCMS (ESI) m/z = 1667 $(M-H)^-$
Retention time: 0.74 minutes (analysis condition SQDFA50)
HPLC conditions for purity analysis: Purity analysis condition C

[Reference Example 2: Search for Metal Salts Capable of Composite Formation]

< Basic Operation >

The $^1$H-NMR of each of a solution obtained by dissolving compound 6 in acetonitrile-d3 (deuteroacetonitrile, $CD_3CN$, CAS: 2206-26-0) (concentration of the cyclic peptide: 1.4 mM, no metal salt addition experiment) and a solution obtained by mixing a metal salt shown in Table 6 below with the cyclic peptide so that the metal salt is 1, 3, or 6 molar equivalents to the cyclic peptide (concentration of the cyclic peptide: 1.4 mM, metal salt addition experiment) was measured at 278 K or 298 K. The results of comparing the obtained $^1$H-NMR spectra are summarized in Table 6.

[Table 6]

| Run | Metal salt added | Added amount/molar equivalent | Suggestion of chelation by $^1$H-NMR |
|---|---|---|---|
| **1** | **$Mg(ClO_4)_2$** | 1 | No initial peak component, occurrence of new peak component, composite formation |
| | | **3** | No initial peak component, occurrence of new peak component, composite formation |
| | | **6** | No initial peak component, occurrence of new peak component, composite formation |

(continued)

| Run | Metal salt added | Added amount/molar equivalent | Suggestion of chelation by $^1$H-NMR |
|---|---|---|---|
| 2 | $MgI_2$ | 1 | No initial peak component, occurrence of new peak component, composite formation |
| | | 3 | No initial peak component, occurrence of new peak component, composite formation |
| | | 6 | No initial peak component, further occurrence of new peak component, composite formation |
| 3 | $Mg(OTf)_2$ | 1 | No initial peak component, occurrence of new peak component, composite formation |
| | | 3 | No initial peak component, occurrence of new peak component, composite formation |
| | | 6 | No initial peak component, occurrence of new peak component, composite formation |
| 4 | $CaI_2$ | 1 | No initial peak component, occurrence of new peak component, composite formation |
| | | 3 | No initial peak component, further occurrence of new peak component, composite formation |
| | | 6 | No initial peak component, change in peak intensity ratio, composite formation |
| 5 | $Sc(OTf)_3$ | 1 | No initial peak component, occurrence of new peak component, composite formation |
| | | 3 | No initial peak component, further occurrence of new peak component, composite formation |
| | | 6 | No initial peak component, change in peak intensity ratio, composite formation |
| 6 | AgOTf | 1 | No change in peak component, no composite formation |
| | | 3 | No change in peak component, no composite formation |
| | | 6 | No change in peak component, no composite formation |

The shape of the $^1$H-NMR spectrum reflects the interaction between the compound and the metal and the changes in the conformation state that accompany with the interaction. The formation of a composite was determined by comparing the peak components observed in the $^1$H-NMR spectrum obtained under no metal salt addition to the peak components observed in the $^1$H-NMR spectrum obtained when the metal salt was added. In other words, the peak component obtained under no metal salt addition was used as the initial peak component to compare the peak component observed in the $^1$H-NMR spectrum when the metal salt was added, and it was determined that the composite was formed when a new peak for the cyclic peptide occurred other than the initial peak components, when a change in the peak intensity ratio occurred, or when the peak component observed in the addition of the metal salt was sharpened and/or broadened compared to the initial peak component (Figures 2 to 7). The formal names and CAS numbers of the metal salts used herein are shown in Table 7.

[Table 7]

| Metal salt | Name | CAS No. |
|---|---|---|
| LiI | Lithium iodide | 10377-51-2 |
| $LiClO_4$ | Lithium perchlorate | 7791-03-9 |

(continued)

| Metal salt | Name | CAS No. |
|---|---|---|
| KI | Potassium iodide | 7681-11-0 |
| $KBF_4$ | Potassium tetrafluoroborate | 14075-53-7 |
| $KPF_6$ | Potassium hexafluorophosphate | 17084-13-8 |
| $BaI_2$ | Barium iodide | 13718-50-8 |
| $Ba(ClO_4)_2$ | Barium perchlorate | 13465-95-7 |
| $MgBr_2$ | Magnesium bromide | 7789-48-2 |
| $Mg(ClO_4)_2$ (83% by mass) | Magnesium perchlorate (83%) | 64010-42-0 |
| $MgI_2$ | Magnesium iodide | 10377-58-9 |
| $Mg(OTf)_2$ | Magnesium trifluoromethanesulfonate | 60871-83-2 |
| $MgSO_4$ | Magnesium sulfate | 7487-88-9 |
| $Mg(OAc)_2 \cdot 4H_2O$ | Magnesium acetate·tetrahydrate | 16674-78-5 |
| $MgCl_2$ | Magnesium chloride | 142-72-3 |
| $MgF_2$ | Magnesium fluoride | 7783-40-6 |
| $CaI_2$ | Calcium iodide | 10102-68-8 |
| $CaBr_2$ | Calcium bromide | 7789-41-5 |
| $Sc(OTf)_3$ | Scandium(III) trifluoromethanesulfonate | 144026-79-9 |
| $Sm(OTf)_3$ | Samarium(III) trifluoromethanesulfonate | 52093-28-4 |
| $Ce(OTf)_3$ | Cerium(III) trifluoromethanesulfonate | 76089-77-5 |
| $Yb(OTf)_3$ | Ytterbium(III) trifluoromethanesulfonate | 54761-04-5 |
| $Zn(OTf)_2$ | Zinc(II) trifluoromethanesulfonate | 54010-75-2 |
| $Mn(OTf)_2$ | Manganese bis(trifluoromethanesulfonate) | 55120-76-8 |
| $In(OTf)_3$ | Indium(III) trifluoromethanesulfonate | 128008-30-0 |
| $Bi(OTf)_3$ | Bismuth tris(trifluoromethanesulfonate) | 88189-03-1 |
| AgOTf | Silver trifluoromethanesulfonate | 2923-28-6 |

[Reference Example 3: Confirmation of Solvent Capable of Composite Formation]

< Basic Operation >

The $^1$H-NMR of each of a solution obtained by dissolving compound 6 in various deuterated solvents shown in Table 8 (concentration of the cyclic peptide: 1.4 mM, no metal salt addition experiment) and a solution obtained by adding about 1 molar equivalent of magnesium perchlorate to the cyclic peptide and dissolving in various deuterated solvents shown in Table 8 (concentration of the cyclic peptide: 1.4 mM, no metal salt addition experiment) was measured at 298 K to obtain $^1$H-NMR spectra.

In Table 8, the $^1$H-NMR spectra of compound 6 under no magnesium perchlorate addition and the $^1$H-NMR spectra of compound 6 when magnesium perchlorate was added to compound 6 by about 1 molar equivalent were compared, and when the composite formation in the solution was suggested, such as when the peak component for the cyclic peptide under no magnesium perchlorate addition disappeared and a new peak component for the cyclic peptide occurred, when a change in the peak intensity ratio occurred, or when the peak component under magnesium perchlorate addition was sharpened, broadened or the like compared to the peak components under no magnesium perchlorate addition, it was described as "formed". In particular, 2,2,2-trifluoroethanol-d3, acetonitrile-d3, and acetone-d6 showed significant peak component changes. The $^1$H-NMR spectra of compound 6 under no magnesium perchlorate addition and the $^1$H-NMR spectra of compound 6 when magnesium perchlorate was added to compound 6 by about 1 molar equivalent were compared, and when the composite formation was not suggested, such as when no peak component for the cyclic peptide occurred other than the peak components under no magnesium perchlorate addition, when a change in the peak

intensity ratio did not occur, or when the peak component under magnesium perchlorate addition was neither sharpened nor broadened compared to the peak components under no magnesium perchlorate addition, it was described as "not formed".

[Table 8]

| Run | CAS No. | Solvent name | Solvent | Composite formation |
|---|---|---|---|---|
| 1 | 811-98-3 | Methanol-d4 | $CD_3OD$ | Not formed |
| 2 | 1516-08-1 | Ethanol-d6 | $CD_3CD_2OD$ | Not formed |
| 3 | 22739-76-0 | 2-Propanol-d8 | $(D_3C)_2CDOD$ | Formed |
| 4 | 53001-22-2 | tert-Butanol-d10 | $(D_3C)_3COD$ | Formed |
| 5 | 77253-67-9 | 2,2,2-Trifluoroethanol-d3 | $CF_3CD_2OD$ | Formed |
| 6 | 2206-27-1 | Dimethyl sulfoxide-d6 | DMSO-d6 | Not formed |
| 7 | 2206-26-0 | Acetonitrile-d3 | $CD_3CN$ | Formed |
| 8 | 4472-41-7 | N,N-dimethylformamide-d4 | DMF-d7 | Not formed |
| 9 | 1693-74-9 | Tetrahydrofuran-d8 | THF-d8 | Formed |
| 10 | 2037-26-5 | Toluene-d8 | Toluene-dB | Formed |
| 11 | 17647-74-4 | 1,4-Dioxane-d8 | 1,4-dioxane-d8 | Formed |
| 12 | 666-52-4 | Acetone-d6 | Acetone-d6 | Formed |
| 13 | 1665-00-5 | Dichloromethane-d2 | DCM-d2 | Formed |

Runs 2, 6, 8 to 13 of Table 8

0.333 mL (Compound 6, 1.01 mg, 0.705 μmol) of a solution of compound 6 (30.24 mg) in acetonitrile ($CH_3CN$, CAS: 75-05-8, 10 mL) was dispensed into vessels and concentrated to dryness. Then, 0.100 mL ($Mg(ClO_4)_2$, 0.188 mg, 0.840 μmol) of a solution of magnesium perchlorate ($Mg(ClO_4)_2$, 4.52 mg, 83 w%) in acetonitrile (2.0 mL) was added to each vessel, and concentrated to dryness. The resultant was dissolved in 0.5 mL of deuterated solvents shown in Table 8 (toluene-d8, ethanol-d6, tetrahydrofuran-d8, acetone-d6, dichloromethane-d2, N,N-dimethylformamide-d4, dimethylsulfoxide-d6, 1,4-dioxane-d8), and $^1$H-NMR was measured at 298 K.

Run 1 of Table 8

0.028 mL (compound 6, 0.999 mg, 0.700 μmol) of a solution of compound 6 (2.78 mg) in methanol-d4 (0.0779 mL) was mixed with 0.0135 mL ($Mg(ClO_4)_2$, 0129 mg, 0.580 μmol) of a solution of magnesium perchlorate ($Mg(ClO_4)_2$, 2.31 mg, 83 w%) in methanol-d4 (0.2 mL). Methanol-d4 (0.4585 mL) was then added, and $^1$H-NMR was measured at 298 K.

Runs 3 to 5 of Table 8

0.010 mL (compound 6, 1.00 mg, 0.700 μmol) of a solution of compound 6 (6.69 mg) in acetonitrile ($CH_3CN$, 0.0669 mL) and 0.0355 mL ($Mg(ClO_4)_2$, 0.160 mg, 0.717 μmol) of a solution of magnesium perchlorate ($Mg(ClO_4)_2$, 10.9 mg, 83 w%) in $CH_3CN$ (2.0 mL) were added to each vessel and concentrated to dryness. The resultant was dissolved in 0.5 mL of deuterated solvents shown in Table 8 (2-propanol-d8, tert-butanol-d10, 2,2,2-trifluoroethanol-d3) and $^1$H-NMR was measured at 298 K.

Run 7 of Table 8

0.028 mL (compound 6, 1.00 mg, 0.700 μmol) of a solution of compound 6 (13.04 mg) in acetonitrile-d3 (0.365 mL), 0.0253 mL ($Mg(ClO_4)_2$, 0.129 mg, 0.580 μmol) of a solution of magnesium perchlorate ($Mg(ClO_4)_2$, 9.25 mg, 83 w%) in acetonitrile-d3 (1.5 mL), and 0.4467 mL of acetonitrile-d3 were mixed, and $^1$H-NMR was measured at 298 K.

[Example 1: Solvent Range Confirmation]

[Example 1-1: Confirmation of Solvent Range Usable for Composite Formation]

< Basic Operation >

Compound 6 (about 10 mg) was mixed with about 1 molar equivalent of magnesium perchlorate and dissolved in the various solvents shown in Table 9 below. Hexane was added directly to the solution, or the solvent was distilled off under reduced pressure or lyophilization, and dichloromethane and hexane were added, and then the mixture was stirred at room temperature according to the following. The resulting solid and supernatant were centrifuged, the solvents were distilled off under reduced pressure or nitrogen spraying, the supernatant and solid after vacuum-drying each were re-dissolved in acetonitrile, and HPLC of each was measured. Table 9 shows the peak area percentage of compound 6, the purity calculated based on the peak area value, the recovery rate, the ratios of isomer 1 and isomer 2 to compound 6, and the purification efficiency.

The sample for HPLC measurement of Example 1-1 was prepared to be about 0.3 mg/mL based on the initial concentration. In Example 1-1, HPLC of the prepared sample was measured under the same conditions as the purity confirmation conditions for Compound 6 in Reference Example 1-2 (purity analysis condition A). Unless otherwise mentioned, the purity and the recovery rate were calculated based on the peak area percentage and peak area (measured at 220 nm) of HPLC measurements under the above conditions on about 0.3 mg/mL accurately prepared preparation. The ratios of isomer 1 and isomer 2 to compound 6 and the purification efficiency were also calculated. Compound 6 used as a raw material was a lot synthesized in Reference Example 1-2, and its purity was 84% based on the peak area percentage under the conditions of HPLC used in the purity analysis. It was thus determined that there was a purification effect when the purity of compound 6 after this operation was improved from 84%. In addition, even when the purity of compound 6 after this operation was not improved from 84%, it was determined that there was a purification effect for those in which the purification efficiency of the isomer of compound 6 was a positive value.

Recovery Rate Calculation Method 1

For example, assuming that the experiment was performed using about *a* mg of raw material, the solid and the solution were centrifuged, the solvent was distilled off by reduced pressure or nitrogen spraying, and (150 *b*/*a*) μL of the solution obtained by adding *b* mL of acetonitrile to the vacuum-dried supernatant and solid was sampled, and then a sample was prepared so that the entire solution was 0.5 mL of 50%v/v aqueous acetonitrile solution. When HPLC of each of the prepared sample and the accurately prepared 0.30 mg/mL preparation are measured and the peak areas (220 nm) of the targets are set to *c* and *d*, respectively, the recovery rate is expressed by the following expression. Considering the case where the actual weighing value (e mg) does not match the assumed weight of *a* mg, a correction was performed using the weighing value.

[Expression 1]

$$[c \div \{(a \div b) \times (150b / a) \div 1000 \div 0.5\}] \div \{d \div 0.3\} \times 100 \times (a \div e)\ (\%)$$

$$\Rightarrow \frac{c \times a}{d \times e} \times 100\ (\%)$$

Purification Efficiency Calculation Method

The ratios of isomer 1 or isomer 2 to compound 6 and the purification efficiency to confirm how much isomer 1 and isomer 2 were separated from compound 6 with respect to compound 6 were calculated by the following expressions.

- Ratio of isomer 1 to compound 6 x 100:

(Peak area of isomer 1) / (Peak area of compound 6) x 100

- Ratio of isomer 2 to compound 6 x 100:

(Peak area of isomer 2) / (Peak area of compound 6) x 100

- Purification efficiency of isomer 1 (%):

{1 - (Ratio of compound 6 to isomer 1) / (Ratio of compound 6 of preparation to isomer 1)} × 100

- Purification efficiency of isomer 2 (%):

{1 - (Ratio of compound 6 to isomer 2) / (Ratio of compound 6 of preparation to isomer 2)} × 100

In Example 1-1, the recovery rate was calculated by the above-described recovery rate calculation method 1 using Labsolution manufactured by SHIMADZU CORPORATION as the HPLC analysis software (*a* = 10). The purification efficiency of the isomers was calculated by the calculation method of the purification efficiency described above.

[Table 9]

| Run | Solvent | Observation | Supernatant | | | | | | Solid | | | | | |
|---|---|---|---|---|---|---|---|---|---|---|---|---|---|---|
| | | | Purity | Recovery rate | Isomer 1 | | Isomer 2 | | Purity | Recovery rate | Isomer 1 | | Isomer 2 | |
| | | | | | Ratio of isomer 1 to compound 6 ×100 | Purification efficiency (%) | Ratio of isomer 2 to compound 6 ×100 | Purification efficiency (%) | | | Ratio of isomer 1 to compound 6 ×100 | Purification efficiency (%) | Ratio of isomer 2 to compound 6 ×100 | Purification efficiency (%) |
| 1 | Dimethylformamide | Candy-like solid precipitate | 72% | 32% | 5.9 | -124 | 17.1 | -123 | 94% | 75% | 0.7 | 72 | 2.7 | 64 |
| 2 | Dimethylacetamide | Candy-like solid precipitate | 66% | 14% | 5.7 | -118 | 26.0 | -240 | 92% | 88% | 1.5 | 44 | 3.8 | 50 |
| 3 | Dimethyl sulfoxide | Candy-like solid precipitate | 83% | 81% | 2.8 | -8 | 8.4 | -10 | 91% | 22% | 0.8 | 68 | 3.0 | 61 |
| 4 | Methanol | Solid precipitate | 75% | 6% | 1.6 | 39 | 24.0 | -214 | 85% | 97% | 2.4 | 7 | 6.7 | 12 |
| 5 | Ethanol | Solid precipitate | 85% | 8% | 2.3 | 10 | 10.6 | -38 | 84% | 98% | 2.5 | 5 | 7.2 | 5 |
| 6 | Dichloromethane | Solid precipitate | 71% | 9% | 2.0 | 22 | 27.2 | -256 | 87% | 90% | 2.3 | 11 | 5.3 | 30 |

Run 1 of Table 9: Dimethylformamide

To compound 6 (10.1 mg), a 35 mM dimethylformamide solution (0.200 mL) prepared by diluting a dimethylformamide solution (0.325 mL) of $Mg(ClO_4)_2$ (6.5 mg) was added, and the mixture was lyophilized. To the obtained solid, dichloromethane (0.66 mL) was added, and the mixture was stirred for 3 days. Hexane (0.33 mL) was added and the mixture was stirred for an additional two days, and the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of supernatant and solid was sampled, and a sample was prepared so that the entire solution was 0.5 mL of a 50%v/v aqueous acetonitrile solution. As a result of confirming the purity and the recovery rate by HPLC, it was found that the purity and the recovery rate of the solid were 94% and 75%, respectively, and the purity and the recovery rate of the supernatant were 72% and 32%, respectively. With these operations, it was confirmed that the precipitated solid was purified. The purification efficiency of isomers 1 and 2 to compound 6 was -124% and -123% in the supernatant, respectively, and 72% and 64% in the solid, respectively. It is considered that the solid exhibited good purification effects because compound 6 precipitated in priority over isomers 1 and 2 under metal addition conditions.

Calculation of Recovery Rate of Run 1

After HPLC of the preparation of 0.30 mg/mL solution was measured, the peak area of the target (2950511, 220 nm) was measured. HPLC of the prepared 50%v/v aqueous acetonitrile solution was measured, and the peak area of the target (supernatant: 962751, solid: 2240371) was measured, and the recovery rate was calculated according to the following method.

Supernatant:

$$(962751 \text{ x } 10) / (2950511 \text{ x } 10.1) \text{ x } 100 \approx 32\%$$

Solid:

$$(2240371 \text{ x } 10) / (2950511 \text{ x } 10.1) \text{ x } 100 \approx 75\%$$

Calculation of Purification Efficiency of Run 1

After HPLC of the preparation of 0.30 mg/mL solution was measured, the peak area of the target (2950511, 220 nm) was measured. The peak areas of the isomers (isomer 1: 77125, isomer 2: 225732) were measured in the same manner. HPLC of the prepared 50%v/v aqueous acetonitrile solution was measured, and the peak area of the target (supernatant: 962751, solid: 2240371), the peak area of isomer 1 (supernatant: 56402, solid: 16355), and the peak area of isomer 2 (supernatant: 164256, solid: 61383) were measured, and the purification efficiency was calculated according to the following methods.

Ratio of isomer ratio 1 in supernatant:

$$56402 / 962751 \text{ x } 100 \approx 5.9\%$$

Ratio of isomer ratio 2 in supernatant:

$$164256 / 962751 \text{ x } 100 \approx 17.1\%$$

Ratio of isomer ratio 1 in solid:

$$16355 / 2240371 \text{ x } 100 \approx 0.7\%$$

Ratio of isomer ratio 2 in solid:

$$61383 / 2240371 \text{ x } 100 \approx 2.7\%$$

Purification efficiency of isomer ratio 1 in supernatant:

$$\{1 - (56402 / 962751) / (77125 / 2950511)\} \times 100 \approx -124\%$$

Purification efficiency of isomer ratio 2 in supernatant:

$$\{1 - (164256 / 962751) / (225732 / 2950511)\} \times 100 \approx -123\%$$

Purification efficiency of isomer ratio 1 in solid:

$$\{1 - (16355 / 2240371) / (77125 / 2950511)\} \times 100 \approx 72\%$$

Purification efficiency of isomer ratio 2 in solid:

$$\{1 - (61383 / 2240371) / (225732 / 2950511)\} \times 100 \approx 64\%$$

Run 2 of Table 9: Dimethylacetamide

To compound 6 (10.0 mg), a 35 mM dimethylacetamide solution (0.200 mL) prepared by diluting a dimethylacetamide solution (0.265 mL) of $Mg(ClO_4)_2$ (5.3 mg) was added, and the mixture was lyophilized. To the obtained solid, dichloromethane (0.66 mL) was added, and the mixture was stirred for 3 days. Hexane (0.33 mL) was added and the mixture was stirred for an additional two days, and the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 3 of Table 9: Dimethylsulfoxide

To compound 6 (10.0 mg), a 35 mM dimethylsulfoxide solution (0.200 mL) prepared by diluting a dimethylsulfoxide solution (0.260 mL) of $Mg(ClO_4)_2$ (5.2 mg) was added, and the mixture was lyophilized. To the obtained solid, dichloromethane (0.66 mL) and hexane (0.33 mL) were sequentially added, and the mixture was stirred for 4 days, and the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 4 of Table 9: Methanol

To compound 6 (10.0 mg), a 35 mM methanol solution (0.200 mL) prepared by diluting a methanol solution (0.305 mL) of $Mg(ClO_4)_2$ (6.1 mg) was added, and the mixture was concentrated and vacuum-dried. To the obtained solid, dichloromethane (0.66 mL) was added, and the mixture was stirred for 3 days. Hexane (0.33 mL) was added and the mixture was stirred for an additional two days, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 5 of Table 9: Ethanol

To compound 6 (9.9 mg), a 35 mM ethanol solution (0.200 mL) prepared by diluting an ethanol solution (0.410 mL) of $Mg(ClO_4)_2$ (8.2 mg) was added, and the mixture was concentrated and vacuum-dried. To the obtained solid, dichloromethane (0.66 mL) was added, and the mixture was stirred for 3 days. Hexane (0.33 mL) was added and the mixture was stirred for an additional two days, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the supernatant was purified.

Run 6 of Table 9: Dichloromethane

To a mixture of compound 6 (10.1 mg) and $Mg(ClO_4)_2$ (2.14 mg, 83 w%), dichloromethane (0.66 mL) was added, and the mixture was stirred for 3 days. Hexane (0.33 mL) was added and the mixture was stirred for an additional two days, and the resulting solid and solution were centrifuged. After the solvent was distilled off, the vacuum-dried supernatant and solid were re-dissolved in acetonitrile (1.0 mL). 15 µL of the obtained acetonitrile solution (1.0 mL) of the supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

From the above results, it was found that, in the composite formation of magnesium perchlorate with the cyclic peptide, in addition to the solvent with which the composite formation was observed in Reference Example 3, a solvent with which the composite formation was not observed can also be used in the purification method of the cyclic peptide.

[Example 1-2: Confirmation of Solvent Range Capable of Purification]

< Basic Operation >

Compound 6 (about 15 mg or about 10 mg) was mixed with about 1 molar equivalent of magnesium perchlorate and dissolved in acetonitrile. The solvent was distilled off under reduced pressure, the various solvents shown in Table 10 below were added, and the mixture was stirred at room temperature according to the following. The supernatant and the resulting solid were centrifuged, and HPLC of each of the supernatant and vacuum-dried solid were measured. The purity and the recovery rate were determined based on the peak area percentage and peak area value of compound 6 and shown in Table 10.

[Table 10]

| Run | Solvent (ratio,volume) | Purity of supernat ant | Recovery rate (superna tant) | Purity of solid/pre cipitate | Recovery rate (solid/pre cipitate) |
|---|---|---|---|---|---|
| 1 | Dichloromethane/Hexane(2:1,1.5 mL) | 68% | 14% | 90% | 82% |
| 2 | Acetone/Hexane(1:1,2 mL) | 69% | 22% | 92% | 68% |
| 3 | 1,4-Dioxane(1 mL) | 72% | 15% | 90% | 76% |
| 4 | Dichloromethane/Heptane(1:2,3 mL) | 84% | 22% | 86% | 44% |
| 5 | Dichloromethane/Toluene(1:2,3 mL) | 83% | 37% | 87% | 52% |
| 6 | Acetonitrile/Toluene(1:29,3 mL) | 83% | 27% | 86% | 53% |
| 7 | Ethyl acetate(0.66 mL) | 70% | 9% | 88% | 81% |
| 8 | Ethanol/Heptane(3:1, 2.66 mL) | 75% | 28% | 90% | 69% |
| 9 | Butanol/Heptane(1:1,1 mL) | 69% | 21% | 92% | 78% |
| 10 | Acetonitrile/Water(1:2,1 mL) | 82% | 14% | 86% | 87% |
| 11 | Acetonitrile/tert-Butyl methyl ether(4:13,1.13 mL) | 81% | 55% | 91% | 51% |
| 12 | Tetrahydrofuran/Heptane(3:1,1 mL) | 71% | 16% | 92% | 81% |
| 13 | Isopropyl acetate/Heptane(4:1,1.65 mL) | 73% | 3% | 85% | 95% |
| 14 | Methyl ethyl ketone/Heptane(3:2,1.65 mL) | 72% | 18% | 88% | 85% |
| 15 | Methanol/Toluene(1:29,1 mL) | 89% | 61% | 79% | 43% |
| 16 | Dimethyl sulfoxide/tert-Butyl methyl ether(9:91,1 mL) | 85% | 94% | 92% | 11% |
| 17 | Dimethylformamide/tert-Butyl methyl ether(1:8,0.9 mL) | 83% | 105% | 68% | 3% |

(continued)

| Run | Solvent (ratio,volume) | Purity of supernat ant | Recovery rate (superna tant) | Purity of solid/pre cipitate | Recovery rate (solid/pre cipitate) |
|---|---|---|---|---|---|
| 18 | Benzyl alcohol/tert-Butyl methyl ether(3:7,1 mL) | 53% | 20% | 97% | 98% |

The samples for HPLC measurement of Examples 1-2 and 2-1 were prepared to be about 0.3 mg/mL based on the initial concentration. In Examples 1-2 and 2-1, HPLC of the prepared sample was measured under the same conditions as the purity confirmation conditions for Compound 6 in Reference Example 1-2 (purity analysis condition A). Unless otherwise mentioned, the recovery rate was calculated with correction based on the peak area (measured at 220 nm) of HPLC measurements under the above conditions on about 0.3 mg/mL accurately prepared preparation. Compound 6 used as a raw material was a lot synthesized in Reference Example 1-2, and its purity was 84% based on the peak area percentage under the conditions of HPLC used in the purity analysis. It was thus determined that there was a purification effect when the purity was improved from 84%.

For Runs 7 to 18, HPLC was measured under purity analysis condition A. The purity and the recovery rate were calculated based on the peak area percentage and peak area (measured at 220 nm) of HPLC measurements under purity analysis condition A on about 0.3 mg/mL accurately prepared preparation. The ratios of isomer 1 and isomer 2 to compound 6 and the purification efficiency were also calculated. For Runs 7 to 18, even when the purity of compound 6 after this operation was not improved from 84%, it was determined that there was a purification effect for those in which the purification efficiency of the isomer of compound 6 was a positive value.

Recovery Rate Calculation Method 2

For example, assuming that the experiment was performed using *a* mg of raw material and the amount of solution used was b mL, when measuring the HPLC of the supernatant, first, the supernatant (b/100) mL was sampled, and then a sample was prepared by adding acetonitrile so that the entire solution was 0.5 mL. Separately, *d* mg of the raw material was weighed and a preparation was prepared with a 10 mL measuring flask. When HPLC of each of the prepared sample and preparation are measured and the peak areas (220 nm) of the targets are set to c and e, respectively, the recovery rate is expressed by the following expression.

[Expression 2]

$$[c \div \{(a \div b) \times (b / 100) \div 0.5\}] \div \{e \div (d \div 10)\} \times 100\ (\%)$$

$$\Rightarrow \frac{5 \times c \times d}{a \times e} \times 100\ (\%)$$

Recovery Rate Calculation Method 3

For example, assuming that the experiment was performed using *a* mg of raw material, the supernatant and the solid were centrifuged, the solvent contained in the supernatant or the solid was distilled off under reduced pressure and dried, and then 0.5 mL (or 1.0 mL) of acetonitrile was added. 5 μL (or 10 μL) of the obtained solution was mixed with 495 μL (or 490 μL) of acetonitrile to prepare a sample. Separately, *d* mg of the raw material was weighed and a preparation was prepared with a 10 mL measuring flask. When HPLC of each of the prepared sample and preparation are measured and the peak areas (220 nm) of the targets are set to *f* and *e*, respectively, the recovery rate is expressed by the following expression.

[Expression 3]

$$[f \div \{(a \div 0.5) \div 100\}] \div \{e \div (d \div 10)\} \times 100\ (\%)$$

$$\Rightarrow \frac{5 \times f \times d}{a \times e} \times 100\ (\%)$$

In Runs 2 to 6, the recovery rate was calculated by the above-described recovery rate calculation method 3 using ACD/Spectrus manufactured by Advanced Chemistry Development, Inc. as the HPLC analysis software. In Runs 7 to 18, the recovery rate was calculated by the above-described recovery rate calculation method 1 using Labsolution manufactured by SHIMADZU CORPORATION as the HPLC analysis software (*a* = 10). The purification efficiency of the isomers was calculated by the calculation method of the purification efficiency described above.

Run 1 of Table 10: Dichloromethane/Hexane

To the solid obtained after concentrating a solution (1.0 mL) of compound 6 (15.00 mg) and $Mg(ClO_4)_2$ (3.03 mg, 83 w%) in $CH_3CN$, dichloromethane (1 mL) and hexane (0.5 mL) were added, and the mixture was stirred overnight. The resulting solid and solution were centrifuged. The solution was dried for 6 hours after concentration, acetonitrile (0.5 mL) was added, and the purity and the recovery rate were confirmed by HPLC. As a result, it was found that the purity was 68% and the recovery rate was 14% from the peak area percentage. The obtained solid was washed with a mixed solution of dichloromethane (1 mL) and hexane (0.5 mL), then centrifuged, and the obtained solid was vacuum-dried overnight to obtain 13.64 mg of compound 6-$Mg(ClO_4)_2$ composite as a solid. As a result of confirming the purity and the recovery rate of the obtained composite by HPLC, it was found that the purity was 90% and the recovery rate was 82%. With these operations, it was confirmed that the precipitated solid was purified.

Calculation of Recovery Rate of Dichloromethane/Hexane-Based Solid

HPLC of the preparation of 0.297 mg/mL solution was measured, and then the peak area of the target (51555, 220 nm) was measured.
3.00 mg of 13.64 mg of the obtained solid was dissolved in 10 mL of acetonitrile. HPLC was then measured, the peak area of the target (46818) was measured, and the recovery rate was calculated according to the following method.

$$(13.64/0.300) \text{ x } 46818 / \{(15/0.297) \text{ x } 51555\} \text{ x } 100 \approx 82\%$$

Run 2 of Table 10: Acetone/Hexane

To the solid obtained after concentrating a solution (1.0 mL) of compound 6 (15.00 mg) and $Mg(ClO_4)_2$ (3.01 mg, 83 w%) in $CH_3CN$, acetone (1 mL) and hexane (1 mL) were added, and the mixture was stirred overnight. The resulting solid and solution were centrifuged. The solution was dried for 6 hours after concentration, acetonitrile (0.5 mL) was added, and the purity and the recovery rate were confirmed by HPLC. As a result, it was found that the purity was 69% and the recovery rate was 22% from the peak area percentage. The obtained solid was dried for 6 hours and dissolved in acetonitrile (0.5 mL), and the purity and the recovery rate was confirmed by HPLC, and it was found that the purity was 92% and the recovery rate was 68%. With these operations, it was confirmed that the precipitated solid was purified.

Run 3 of Table 10: 1,4-Dioxane

To the solid obtained after concentrating a solution (1.0 mL) of compound 6 (14.98 mg) and $Mg(ClO_4)_2$ (2.96 mg, 83 w%) in $CH_3CN$, 1,4-dioxane (1 mL) was added, and the mixture was stirred overnight. The resulting solid and solution were centrifuged. The solution was dried for 6 hours after concentration, acetonitrile (0.5 mL) was added, and the purity and the recovery rate were confirmed by HPLC. As a result, it was found that the purity was 72% and the recovery rate was 15% from the peak area percentage. The obtained solid was dried for 6 hours and dissolved in acetonitrile (0.5 mL), and the purity and the recovery rate was confirmed by HPLC, and it was found that the purity was 90% and the recovery rate was 76%. With these operations, it was confirmed that the precipitated solid was purified.

Runs 4, 5, and 6 of Table 10

A solution (5.0 mL) of compound 6 (75.12 mg) and $Mg(ClO_4)_2$ (14.68 mg, 83 w%) in $CH_3CN$ was dispensed in 1 mL portions, and each was concentrated. To the obtained solid, each solvent shown in Runs 4 to 6 below was added, and the mixture was stirred over the weekend. The resulting solid and solution were centrifuged. The solution was vacuum-dried after concentration, acetonitrile (0.5 mL) was added, and the purity and the recovery rate were confirmed by HPLC.
The obtained solid was dissolved in acetonitrile (0.5 mL) after vacuum-drying, and the purity and the recovery rate were confirmed by HPLC. Note that a sample for the purity and the recovery rate measurement was prepared by diluting 10 μL of each acetonitrile solution of solution and solid prepared after concentration and vacuum-drying with 990 μL of acetonitrile. As a result, it was confirmed that both solid phases were purified.

Run 7 of Table 10: Ethyl Acetate

To compound 6 (9.9 mg), a 35 mM acetonitrile solution (0.200 mL) prepared by diluting an acetonitrile solution (0.414 mL) of $Mg(ClO_4)_2$ (20.7 mg) was added, and the mixture was concentrated and vacuum-dried. To the obtained solid, ethyl acetate (0.66 mL) was added, and the mixture was stirred for 3 days. The resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Calculation of Recovery Rate of Run 7

After HPLC of the preparation of 0.30 mg/mL solution was measured, the peak area of the target (2950511, 220 nm) was measured. HPLC of the prepared 50%v/v aqueous acetonitrile solution was measured, and the peak area of the target (supernatant: 255084, solid: 2369897) was measured, and the recovery rate was calculated according to the following method.

Supernatant:

$$(255084 \text{ x } 10)/(2950511 \text{ x } 9.9) \text{ x } 100 \approx 9\%$$

Solid:

$$(2369897 \text{ x } 10)/(2950511 \text{ x } 9.9) \text{ x } 100 \approx 81\%$$

Run 8 of Table 10: Ethanol/Heptane

To compound 6 (10.0 mg), a 35 mM acetonitrile solution (0.200 mL) prepared by diluting an acetonitrile solution (0.414 mL) of $Mg(ClO_4)_2$ (20.7 mg) was added, and the mixture was concentrated and vacuum-dried. To the obtained solid, ethanol (0.66 mL) was added, and the mixture was stirred for 3 days. Heptane (1.0 mL) was added and the mixture was stirred overnight, and then heptane (1.0 mL) was further added and the mixture was stirred for 2 days, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 9 of Table 10: Butanol/Heptane

A 35 mM acetonitrile solution (0.200 mL) prepared by dispensing and diluting 0.100 mL of an acetonitrile solution (0.925 mL) of compound 6 (92.5 mg) and 0.038 mL of an acetonitrile solution (0.500 mL) of $Mg(ClO_4)_2$ (25.0 mg) was concentrated and vacuum-dried. To the obtained solid, butanol (0.5 mL) and heptane (0.5 mL) were added, and the mixture was stirred for 2 days. Heptane (0.25 mL) was further added and the mixture was stirred for 6 hours, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Runs 10 and 12 of Table 10

35 mM acetonitrile solutions (each 0.200 mL) prepared by dispensing and diluting each 0.100 mL of an acetonitrile solution (0.925 mL) of compound 6 (92.5 mg) and each 0.038 mL of an acetonitrile solution (0.500 mL) of $Mg(ClO_4)_2$ (25.0 mg) were concentrated and vacuum-dried. To the obtained solids, each solvent shown in Runs 10 and 12 of Table 10 was added, and the mixture was stirred for 3 days. The resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 11 of Table 10: Acetonitrile/tert-Butyl Methyl Ether

A 35 mM acetonitrile solution (0.200 mL) prepared by dispensing and diluting 0.100 mL of an acetonitrile solution (0.925 mL) of compound 6 (92.5 mg) and 0.038 mL of an acetonitrile solution (0.500 mL) of $Mg(ClO_4)_2$ (25.0 mg) was concentrated and vacuum-dried. To the obtained solid, acetonitrile (0.13 mL) and tert-butyl methyl ether (0.87 mL) were added, and the mixture was stirred for 2 days. Acetonitrile (0.13 mL) was further added and the mixture was stirred for 6 hours, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 13 of Table 10: Isopropyl acetate/Heptane

A 35 mM acetonitrile solution (0.200 mL) prepared by dispensing and diluting 0.100 mL of an acetonitrile solution (0.925 mL) of compound 6 (92.5 mg) and 0.038 mL of an acetonitrile solution (0.500 mL) of $Mg(ClO_4)_2$ (25.0 mg) was concentrated and vacuum-dried. To the obtained solid, isopropyl acetate (0.66 mL) and heptane (0.33 mL) were added, and the mixture was stirred for 2 days. Isopropyl acetate (0.66 mL) was further added and the mixture was stirred for 6 hours, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 14 of Table 10: Methyl ethyl ketone/Heptane

A 35 mM acetonitrile solution (0.200 mL) prepared by dispensing and diluting 0.100 mL of an acetonitrile solution (0.925 mL) of compound 6 (92.5 mg) and 0.038 mL of an acetonitrile solution (0.500 mL) of $Mg(ClO_4)_2$ (25.0 mg) was concentrated and vacuum-dried. To the obtained solid, methyl ethyl ketone (0.66 mL) and heptane (0.66 mL) were added, and the mixture was stirred for 2 days. Methyl ethyl ketone (0.33 mL) was further added and the mixture was stirred for 6 hours, and the resulting solid and solution were centrifuged. After the solvent was distilled off, the vacuum-dried supernatant and solid were re-dissolved in acetonitrile (1.0 mL). 15 μL of the obtained acetonitrile solution (1.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 15 of Table 10: Methanol/Toluene

A 35 mM acetonitrile solution (0.200 mL) prepared by dispensing and diluting 0.100 mL of an acetonitrile solution (0.925 mL) of compound 6 (92.5 mg) and 0.038 mL of an acetonitrile solution (0.500 mL) of $Mg(ClO_4)_2$ (25.0 mg) was concentrated and vacuum-dried. To the obtained solid, methanol (33.3 μL) and toluene (966.7 μL) were added and the mixture was stirred for 3 days, then the resulting oil-like precipitate and solution were centrifuged. After the solvent was distilled off, the vacuum-dried supernatant and precipitate were re-dissolved in acetonitrile (1.0 mL). 15 μL of the obtained acetonitrile solution (1.0 mL) of the supernatant and precipitate was sampled, and a sample was prepared so that the entire solution was 0.5 mL of a 50%v/v aqueous acetonitrile solution. As the results of confirming the purity and the recovery rate by HPLC, it was confirmed that the supernatant was purified.

Run 16 of Table 10: Dimethyl Sulfoxide/tert-Butyl Methyl Ether

A 35 mM acetonitrile solution (0.200 mL) prepared by dispensing and diluting 0.100 mL of an acetonitrile solution (0.485 mL) of compound 6 (48.5 mg) and 0.094 mL of an acetonitrile solution (0.265 mL) of $Mg(ClO_4)_2$ (5.3 mg) was concentrated and vacuum-dried. To the obtained solid, dimethyl sulfoxide (90 μL) and tert-butyl methyl ether (910 μL) were added and the mixture was stirred for 3 days, and then the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of supernatant and solid was sampled, and a sample was prepared so that the entire solution was 0.5 mL of a 50%v/v aqueous acetonitrile solution. As the results of confirming the purity and the recovery rate by HPLC, it was confirmed that the solid was purified.

Run 17 of Table 10: Dimethylformamide/tert-Butyl Methyl Ether

To compound 6 (10.1 mg), a 35 mM acetonitrile solution (0.200 mL) prepared by diluting an acetonitrile solution (0.475 mL) of $Mg(ClO_4)_2$ (9.5 mg) was added, and the mixture was concentrated and vacuum-dried. To the obtained solid, dimethylformamide (0.1 mL) and tert-butyl methyl ether (0.4 mL) were added and the mixture was stirred for 3 days, then tert-butyl methyl ether (0.4 mL) was further added and the mixture was stirred overnight, and then the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of supernatant and solid was sampled, and a sample was prepared so that the entire solution was 0.5 mL of a 50%v/v aqueous acetonitrile solution. As a result of confirming the purity and the recovery rate by HPLC, there was no significant improvement in purity, but the purification efficiency of isomer 1 was 40% and the purification efficiency of isomer 2 was 13% with respect to compound 6 in the solid, it was thus determined that the solid was purified.

Run 18 of Table 10: Benzyl Alcohol/tert-Butyl Methyl Ether

To compound 6 (10.1 mg), a 35 mM acetonitrile solution (0.200 mL) prepared by diluting an acetonitrile solution (0.475 mL) of $Mg(ClO_4)_2$ (9.5 mg) was added, and the mixture was concentrated and vacuum-dried. To the obtained solid, benzyl alcohol (0.3 mL) and tert-butyl methyl ether (0.7 mL) were added and the mixture was stirred for 4 days, and then the resulting solid and solution were centrifuged. After the solvent was distilled off, the vacuum-dried supernatant and solid were re-dissolved in acetonitrile (3.0 mL). 45 μL of the obtained acetonitrile solution (3.0 mL) of the supernatant and solid, was sampled, and a sample was prepared so that the entire solution was 0.5 mL of a 50%v/v aqueous acetonitrile solution. As the results of confirming the purity and the recovery rate by HPLC, it was confirmed that the solid was purified.

From the above results, it was found that, in the composite formation of magnesium perchlorate with the cyclic peptide, in addition to the solvent with which the composite formation was observed in Reference Example 3, a solvent with which the composite formation was not observed can also be used in the purification of the cyclic peptide.

[Example 2-1: Preparation of Composite and Confirmation of Purification Effect]

< Basic Operation >

Compound 6 (about 15 mg or about 10 mg) was mixed with about 1 molar equivalent of each of the various metal salts shown in Table 11 below and dissolved in acetonitrile, ethanol, or an aqueous ethanol solution. The solvent was then distilled off under reduced pressure, and the various solvents shown in Table 11 below were added, and the mixture was stirred at room temperature according to the following, and then the supernatant and the resulting solid were centrifuged. The HPLC of each of the supernatant and the vacuum-dried solid were measured under purity analysis condition A, and the purity and the recovery rate were determined based on the peak area percentage and peak area value of compound 6 as shown in Table 12. HPLC analysis was performed using ACD/Spectrus manufactured by Advanced Chemistry Development, Inc. (Runs 1 to 26) or Labsolution manufactured by SHIMADZU CORPORATION (Runs 27 to 31). The recovery rate was calculated by the recovery rate calculation method 1, 2, or 3 of Example 1. Compound 6 used as a raw material was a lot synthesized in Reference Example 1-2, and its purity was 84% based on the peak area percentage under the condition of HPLC used in the purity analysis (Purity analysis condition A). It was thus determined that there was a purification effect when the purity was improved from 84% based on the analysis of HPLC measured under the same conditions. In addition, even when the purity of compound 6 after this operation was not improved from 84%, it was determined that there was a purification effect for those in which the purification efficiency of the isomer of compound 6 was a positive value. The mass and molar equivalents of the metal salts actually used and the mass of compound 6 were shown in Table 13.

[Table 11]

| Run | Metal salt | Observation | First solvent | Second solvent |
|---|---|---|---|---|
| 1 | none | No precipitate | $CH_3CN$ | DCM/Hexane : 1 mL/2 mL |
| 2 | LiI | Solid precipitate | $CH_3CN$ | DCM/Hexane : 1 mL/2 mL |
| 3 | $LiClO_4$ | Solid precipitate | $CH_3CN$ | DCM/Hexane : 1 mL/2 mL |
| 4 | KI | Crystal precipitate | $CH_3CN$ | $CH_3CN$ : 0.2 mL |
| 5 | $KBF_4$ | Added metal salt precipitate | $CH_3CN$ | DCM/Hexane : 1 mL/2 mL |
| 6 | $KPF_6$ | Added metal salt precipitate | $CH_3CN$ | DCM/Hexane : 1 mL/2 mL |

(continued)

| Run | Metal salt | Observation | First solvent | Second solvent |
|---|---|---|---|---|
| 7 | $BaI_2$ | Solid precipitate | $CH_3CN$ | DCM/Hexane : 1 mL/2 mL |
| 8 | $Ba(ClO_4)_2$ | Solid precipitate | $CH_3CN$ | DCM/Hexane : 1 mL/2 mL |
| 9 | $MgBr_2$ | Crystal precipitate | $CH_3CN$ | DCM/Hexane : 1 mL/1 mL |
| 10 | $Mg(ClO_4)_2$ | Solid precipitate | $CH_3CN$ | DCM/Hexane : 1 mL/0.5 mL |
| 11 | $MgI_2$ | Crystal and solid precipitate | $CH_3CN$ | DCM/Hexane : 1 mL/1 mL |
| 12 | $Mg(OTf)_2$ | Solid precipitate | $CH_3CN$ | DCM/Hexane : 1 mL/1 mL |
| 13 | $MgSO_4$ | Metal salt not dissolved in $CH_3CN$ | $CH_3CN$ | DCM/Hexane : 1 mL/1 mL |
| 14 | $Mg(OAc)_2$-$4H_2O$ | Metal salt not dissolved in $CH_3CN$ | $CH_3CN$ | DCM/Hexane : 1 mL/1 mL |
| 15 | $MgCl_2$ | Metal salt not dissolved in $CH_3CN$ | $CH_3CN$ | DCM/Hexane : 1 mL/1 mL |
| 16 | $MgF_2$ | Metal salt not dissolved in $CH_3CN$ | $CH_3CN$ | DCM/Hexane : 1 mL/1 mL |
| 17 | $CaBr_2$ | Solid precipitate | $CH_3CN$ | DCM/Hexane : 1 mL/2 mL |
| 18 | $Sc(OTf)_3$ | Solid precipitate | $CH_3CN$ | DCM/Hexane : 1 mL/1 mL |
| 19 | $Sm(OTf)_3$ | Solid precipitate | $CH_3CN$ | DCM/Hexane : 1 mL/1 mL |
| 20 | $Ce(OTf)_3$ | Solid precipitate | $CH_3CN$ | DCM/Hexane : 1 mL/2 mL |
| 21 | $Yb(OTf)_3$ | Solid precipitate | $CH_3CN$ | DCM/Hexane : 1 mL/2 mL |
| 22 | $Zn(OTf)_2$ | Solid precipitate | $CH_3CN$ | DCM/Hexane : 1 mL/2 mL |
| 23 | $Mn(OTf)_2$ | Solid precipitate | $CH_3CN$ | DCM/Hexane : 1 mL/1 mL |
| 24 | $In(OTf)_3$ | Compound 6 racemization | $CH_3CN$ | DCM/Hexane : 1 mL/2 mL |
| 25 | $Bi(OTf)_3$ | Compound 6 degradation | $CH_3CN$ | - |
| 26 | AgOTf | No precipitate | $CH_3CN$ | DCM/Hexane : 1 mL/0.6 mL |
| 27 | $MgSO_4$-$7H_2O$ | Solid precipitate | $EtOH/H_2O$ : 3/2 | THF/Heptane : 0.2 mL/0.8 mL |
| 28 | $Mg(OAc)_2$-$4H_2O$ | Solid precipitate | EtOH | THF/Heptane : 0.2 mL/0.8 mL |
| 29 | $MgCl_2$-$6H_2O$ | Solid precipitate | EtOH | THF/Heptane : 0.4 mL/0.4 mL |
| 30 | $Mp(OAc)_2$-$4H_2O$ | Oil-like precipitate | EtOH | $CH_3CN/H_2O$ : 0.3 mL/0.6 mL |
| 31 | $MgCl_2$-$6H_2O$ | Oil-like precipitate | EtOH | $CH_3CN/H_2O$ : 0.3 mL/0.6 mL |

[Table 12]

| Run | Supernatant | | | | | | Solid/precipitate | | | | | |
|---|---|---|---|---|---|---|---|---|---|---|---|---|
| | Purity | Recovery rate | Isomer 1 | | Isomer 2 | | Purity | Recovery rate | Isomer 1 | | Isomer 2 | |
| | | | Ratio of isomer 1 to compound 6 ×100 | Purification efficiency (%) | Ratio of isomer 2 to compound 6 ×100 | Purification efficiency (%) | | | Ratio of isomer 1 to compound 6 ×100 | Purification efficiency (%) | Ratio of isomer 2 to compound 6 ×100 | Purification efficiency (%) |
| 1 | 84% | - | 2.5 | - | 8.2 | - | - | - | - | - | - | - |
| 2 | 88% | 78% | 1 | 60 | 6.3 | 23 | 61% | 7% | 14.2 | -468 | 19.8 | -142 |
| 3 | 91% | 60% | 0.7 | 73 | 4.7 | 43 | 74% | 28% | 5.2 | -108 | 13.3 | -63 |
| 4 | 82% | 43% | 2.8 | -12 | 9 | -10 | 90% | 57% | 1.6 | 38 | 5 | 39 |
| 5 | 84% | 84% | 2.4 | 2 | 8 | 1 | - | <1% | - | - | - | - |
| 6 | 84% | 83% | 2.4 | 4 | 7.8 | 5 | - | <1% | - | - | - | - |
| 7 | 85% | 29% | 1.4 | 45 | 10.4 | -28 | 85% | 54% | 2.5 | -2 | 6.3 | 23 |
| 8 | 84% | 23% | 1.3 | 50 | 12.1 | -48 | 86% | 65% | 2.5 | -2 | 6 | 27 |
| 9 | 72% | 34% | 5.4 | -116 | 17.5 | -115 | 98% | 43% | 0.3 | 88 | 1.5 | 82 |
| 10 | 63% | 9% | 4.4 | -78 | 27.8 | -242 | 90% | 68% | 1.8 | 28 | 3.7 | 55 |
| 11 | 60% | 12% | 7.3 | -191 | 36.1 | -343 | 90% | 73% | 1.5 | 40 | 3.3 | 60 |
| 12 | 82% | 46% | 1.8 | 26 | 12.1 | -49 | 89% | 41% | 2.5 | 2 | 3.3 | 59 |
| 13 | 84% | 82% | 2.4 | 5 | 7.7 | 6 | - | <1% | - | - | - | - |
| 14 | 84% | 84% | 2.5 | 0 | 8.3 | -2 | - | <1% | - | - | - | - |
| 15 | 84% | 81% | 2.4 | 4 | 8.1 | 1 | - | <1% | - | - | - | - |
| 16 | 84% | 82% | 2.4 | 3 | 8 | 1 | - | <1% | - | - | - | - |
| 17 | 86% | 45% | 1 | 59 | 9.7 | -19 | 84% | 42% | 3.4 | -35 | 6.1 | 25 |
| 18 | 84% | 42% | 2.4 | 4 | 9.6 | -17 | 87% | 40% | 2 | 19 | 5.3 | 34 |
| 19 | 84% | 50% | 2.2 | 14 | 10.5 | -28 | 87% | 33% | 2.2 | 12 | 3.8 | 53 |
| 20 | 69% | 12% | 3.8 | -52 | 32.1 | -293 | 88% | 63% | 2 | 20 | 3.4 | 58 |
| 21 | 87% | 32% | 1 | 58 | 9 | -10 | 85% | 54% | 2.9 | -15 | 6.6 | 18 |
| 22 | 79% | 21% | 0.9 | 64 | 13.3 | -63 | 86% | 58% | 2.2 | 10 | 6.3 | 23 |

(continued)

| Run | Supernatant | | | | | | Solid/precipitate | | | | | |
|---|---|---|---|---|---|---|---|---|---|---|---|---|
| | Purity | Recovery rate | Isomer 1 | | Isomer 2 | | Purity | Recovery rate | Isomer 1 | | Isomer 2 | |
| | | | Ratio of isomer 1 to compound 6 ×100 | Purification efficiency (%) | Ratio of isomer 2 to compound 6 ×100 | Purification efficiency (%) | | | Ratio of isomer 1 to compound 6 ×100 | Purification efficiency (%) | Ratio of isomer 2 to compound 6 ×100 | Purification efficiency (%) |
| 23 | 77% | 32% | 3.1 | -22 | 15.6 | -91 | 90% | 49% | 1.8 | 27 | 3.6 | 56 |
| 24 | 89% | 38% | 1.7 | 31 | 6.2 | 24 | 76% | 42% | 3.3 | -32 | 9.7 | -19 |
| 25 | - | - | - | - | - | - | - | - | - | - | - | - |
| 26 | 84% | 89% | 2.3 | 8 | 8.2 | 0 | - | <1% | - | - | - | - |
| 27 | 86% | 92% | 2.3 | 13 | 7.4 | 3 | 69 | 8 | 5.3 | -102 | 6.9 | 10 |
| 28 | 87% | 92% | 2.3 | 12 | 7.5 | 2 | 72 | 6 | 4.7 | -80 | 6.9 | 10 |
| 29 | 84% | 91% | 2.5 | 5 | 7.5 | 2 | 72 | 9 | 2.3 | 12 | 7 | 8 |
| 30 | 81% | 9% | 2.1 | 20 | 5.5 | 28 | 88 | 50 | 2 | 24 | 7.1 | 7 |
| 31 | 82% | 20% | 2.4 | 7 | 5.8 | 24 | 86 | 79 | 2.5 | 5 | 7.6 | 1 |

[Table 13]

| Run | Metal used | Mass of metal/mg | Equivalent of metal /molar equivalent | Compound 6 used/mg |
|---|---|---|---|---|
| 2 | LiI | 1.44 | 1.03 | 15.0 |
| 3 | $LiClO_4$ | 1.38 | 1.24 | 15.0 |
| 4 | KI | 1.82 | 1.04 | 15.0 |
| 5 | $KBF_4$ | 1.36 | 1.02 | 15.1 |
| 6 | $KPF_6$ | 2.45 | 1.26 | 15.0 |
| 7 | $BaI_2$ | 4.17 | 1.01 | 15.0 |
| 8 | $Ba(ClO_4)_2$ | 3.68 | 1.04 | 15.0 |
| 9 | $MgBr_2$ | 2.20 | 1.14 | 15.0 |
| 10 | $Mg(ClO_4)_2$ (83w%) | 3.03 | 1.07 | 15.0 |
| 11 | $MgI_2$ | 3.16 | 1.08 | 15.1 |
| 12 | $Mg(OTf)_2$ | 3.55 | 1.04 | 15.1 |
| 13 | $MgSO_4$ | 1.28 | 1.01 | 15.0 |
| 14 | $Mg(OAc)_2$-$4H_2O$ | 2.43 | 1.08 | 15.0 |
| 15 | $MgCl_2$ | 1.12 | 1.12 | 15.0 |
| 16 | $MgF_2$ | 0.77 | 1.18 | 15.0 |
| 17 | $CaBr_2$ | 2.14 | 1.02 | 15.0 |
| 18 | $Sc(OTf)_3$ | 5.18 | 1.00 | 15.0 |
| 19 | $Sm(OTf)_3$ | 6.28 | 1.00 | 15.1 |
| 20 | $Ce(OTf)_3$ | 6.25 | 1.01 | 15.1 |
| 21 | $Yb(OTf)_3$ | 6.73 | 1.03 | 15.0 |
| 22 | $Zn(OTf)_2$ | 4.13 | 1.08 | 15.1 |
| 23 | $Mn(OTf)_2$ | 3.85 | 1.04 | 15.0 |
| 24 | $In(OTf)_3$ | 6.14 | 1.04 | 15.1 |
| 25 | $Bi(OTf)_3$ | 7.07 | 1.03 | 15.0 |
| 26 | AgOTf | 2.65 | 0.98 | 15.0 |
| 27 | $MgSO_4$-$7H_2O$ | 1.73 | 1.00 | 10.0 |
| 28 | $Mg(OAc)_2$-$4H_2O$ | 1.50 | 1.00 | 10.0 |
| 29 | $MgCl_2$-$6H_2O$ | 1.42 | 1.00 | 10.0 |
| 30 | $Mg(OAc)_2$-$4H_2O$ | 1.50 | 10.0 | 10.0 |
| 31 | $MgCl_2$-$6H_2O$ | 1.42 | 1.01 | 9.9 |

The mass of the inorganic salts of Runs 27 to 31 was calculated from the volume of the solution obtained by preparing an acetonitrile solution of each inorganic salt, dispensing, and mixing with compound 6.

The following experiments were performed on the metal salts shown in Run 10 of Table 11.

To the solid obtained after concentrating a solution (1.0 mL) of compound 6 (15.00 mg) and $Mg(ClO_4)_2$ (3.03 mg, 83 w%) in $CH_3CN$, dichloromethane (1 mL) and hexane (0.5 mL) were added, and the mixture was stirred overnight. The resulting solid and solution were centrifuged. The solution was dried for 6 hours after concentration, acetonitrile (0.5 mL) was added, and the purity and the recovery rate were confirmed by HPLC. As a result, it was found that the purity was 68% and the recovery rate was 14% from the peak area percentage. The obtained solid was washed with a mixed solution of dichloromethane (1 mL) and hexane (0.5 mL), then centrifuged, and the obtained solid was vacuum-dried overnight to obtain 13.64 mg of compound 6-$Mg(ClO_4)_2$ composite as a solid. As a result of confirming the purity and the recovery rate of the obtained composite by HPLC, it was found that the purity was 90% and the recovery rate was 82%. With these operations, it was confirmed that the precipitated solid was purified.

Calculation of Recovery Rate

After HPLC of the preparation of 0.297 mg/mL solution was measured, the peak area of the target (51555, 220 nm) was measured.
3.00 mg of 13.64 mg of the obtained solid was dissolved in 10 mL of acetonitrile. HPLC was then measured, the peak area of the target (46818) was measured, and the recovery rate was calculated according to the following method.

$$(13.64/0.300) \times 46818 / \{(15/0.297) \times 51555\} \times 100 \approx 82\%$$

The following experiments were performed on the metal salts shown in Run 26 of Table 11.
To a solid obtained by vacuum-drying a solution of compound 6 (15.0 mg) and AgOTf (2.65 mg, 0.98 molar equivalents) in $CH_3CN$ (0.5 mL), dichloromethane (1 mL) and hexane (0.6 mL, divided into a total of six portions of 0.1 mL each for every 2 to 5 hours) were added. No precipitate occurred, thus the purity and the recovery rate of the solution (supernatant) were confirmed by HPLC (calculation method 2).

Calculation of Recovery Rate

HPLC was measured before and after adding hexane during the above experiment, the peak area of the target (before adding hexane: 55176, after adding hexane: 49150) was measured, and the recovery rate was calculated according to the following method.

$$(49150 / 55176) \times 100 \approx 89\%$$

The following experiments were performed on the metal salts shown in Runs 11, 12, 13, 18, 19, and 23 of Table 11.
To the solid obtained after concentrating a $CH_3CN$ solution (0.2 mL) or suspension (0.2 mL) of compound 6 (about 15 mg) and each of various metal salts (about 1 molar equivalent), dichloromethane (1 mL) and hexane (1 mL) were added and the mixture was stirred for 4.5 hours, and the resulting solid and solution were centrifuged. After decanting the supernatant, the obtained solid was dissolved in 0.5 mL of $CH_3CN$ after vacuum-drying, and the purity and the recovery rate were confirmed by HPLC (calculation method 3).
The following experiments were performed on the metal salts shown in Runs 9, 14, 15, and 16 of Table 11.
To the solid obtained after leaving a $CH_3CN$ solution (0.4 mL) or suspension (0.4 mL) of compound 6 (about 15 mg) and each of various metal salts (about 1 molar equivalent) in an open state overnight and then vacuum-drying, dichloromethane (1 mL) and hexane (1 mL) were added and the mixture was stirred for 5 hours, and the resulting solid and solution were centrifuged. After decanting the supernatant, the obtained solid was dissolved in 1.0 mL of $CH_3CN$ after vacuum-drying, and the purity and the recovery rate were confirmed by HPLC.
The following experiments were performed on the metal salts shown in Runs 2, 3, 7, 8, and 17 of Table 11.
To the solid obtained after leaving a $CH_3CN$ solution (0.4 mL) of compound 6 (about 15 mg) and each of various metal salts (about 1 molar equivalent) in an open state overnight and then vacuum-drying, dichloromethane (1 mL) and hexane (1 mL) were added and the mixture was stirred for 5 hours, then hexane (1 mL) was further added and the mixture was stirred for 19 hours, and the resulting solid and solution were centrifuged. After decanting the supernatant, the obtained solid was dissolved in 1.0 mL of $CH_3CN$ after vacuum-drying, and the purity and the recovery rate were confirmed by HPLC (calculation method 3).
The following experiments were performed on the metal salts shown in Runs 21, 24, and 25 of Table 11.
To the solid obtained after vacuum-drying a $CH_3CN$ solution (0.2 mL) of compound 6 (about 15 mg) and each of various metal salts (about 1 molar equivalent), dichloromethane (1 mL) and hexane (1 mL) were added and the mixture was stirred overnight, then hexane (1 mL) was further added and the mixture was stirred for 5 hours, and the resulting solid and solution were centrifuged. After decanting the supernatant, the obtained solid was dissolved in 1.0 mL of $CH_3CN$ after vacuum-drying, and the purity and the recovery rate were confirmed by HPLC (calculation method 3).
The following experiments were performed on the metal salts shown in Runs 5, 6, 20, and 22 of Table 11.
To the solid obtained after vacuum-drying a $CH_3CN$ solution (0.1 mL) of compound 6 (about 15 mg) and each of various metal salts (about 1 molar equivalent), dichloromethane (1 mL) and hexane (1 mL) were added and the mixture was stirred for 17 hours, then hexane (1 mL) was further added and the mixture was stirred for 6 hours, and the resulting solid and solution were centrifuged. After decanting the supernatant, the obtained solid was dissolved in 1.0 mL of $CH_3CN$ after vacuum-drying, and the purity and the recovery rate were confirmed by HPLC (calculation method 3).
The following experiments were performed on the metal salts shown in Run 4 of Table 11.
A solution (0.2 mL) of compound 6 (15.02 mg) and KI (1.82 mg, 1.04 molar equivalents) in $CH_3CN$ was stirred with ultrasound and the resulting solid and solution were centrifuged. After decanting the supernatant, the obtained solid was

dissolved in 1.0 mL of $CH_3CN$ after vacuum-drying, and the purity and the recovery rate were confirmed by HPLC. For the supernatant, the solvent was distilled off under reduced pressure, and then the supernatant was vacuum-dried and dissolved in 1.0 mL of $CH_3CN$, and the purity and the recovery rate were confirmed by HPLC (calculation method 3).

The following experiments were performed on the metal salts shown in Run 27 of Table 11. To compound 6 (10.0 mg), a solution (0.200 mL) prepared by dispensing 0.087 mL of a solution of $MgSO_4$-$7H_2O$ (8.9 mg) in 60% ethanol (0.445 mL) and diluting with 0.113 mL of 60% ethanol was added, and the mixture was concentrated and vacuum-dried. To the obtained solid, tetrahydrofuran (0.2 mL) and heptane (0.8 mL) were added and the mixture was stirred for 3 days, and then the resulting solid and solution were centrifuged. After the solvent was distilled off, the vacuum-dried supernatant and solid were re-dissolved in a 60% aqueous ethanol solution (1.0 mL). 15 μL of the obtained 60% aqueous ethanol solution (1.0 mL) of supernatant and solid was sampled, the solvent was distilled off, and a sample was prepared so that the entire solution was 0.5 mL of a 50%v/v aqueous acetonitrile solution. The purity and the recovery rate were confirmed by HPLC.

The following experiments were performed on the metal salts shown in Run 28 of Table 11. A solution (0.200 mL) prepared by dispensing 0.100 mL of an ethanol solution (0.485 mL) of compound 6 (48.5 mg) and 0.075 mL of an ethanol solution (0.515 mL) of $Mg(OAc)_2$-$4H_2O$ (10.3 mg) and then diluting with 0.025 mL of ethanol was concentrated and vacuum-dried. To the obtained solid, tetrahydrofuran (0.2 mL) and heptane (0.8 mL) were added and the mixture was stirred for 3 days, and then the resulting solid and solution were centrifuged. After the solvent was distilled off, the vacuum-dried supernatant and solid were re-dissolved in ethanol (1.0 mL). 15 μL of the obtained ethanol solution (1.0 mL) of the supernatant and solid was sampled, the solvent was distilled off, and a sample was prepared so that the entire solution was 0.5 mL of a 50%v/v aqueous acetonitrile solution. The purity and the recovery rate were confirmed by HPLC.

The following experiments were performed on the metal salts shown in Run 29 of Table 11. A solution (0.200 mL) prepared by dispensing 0.100 mL of an ethanol solution (0.485 mL) of compound 6 (48.5 mg) and 0.071 mL of an ethanol solution (0.330 mL) of $MgCl_2$-$6H_2O$ (6.6 mg) and then diluting with 0.029 mL of ethanol was concentrated and vacuum-dried. To the obtained solid, tetrahydrofuran (0.4 mL) and heptane (0.4 mL) were added and the mixture was stirred for 3 days, and then the resulting solid and solution were centrifuged. After the solvent was distilled off, the vacuum-dried supernatant and solid were re-dissolved in ethanol (1.0 mL). 15 μL of the obtained ethanol solution (1.0 mL) of the supernatant and solid was sampled, the solvent was distilled off, and a sample was prepared so that the entire solution was 0.5 mL of a 50%v/v aqueous acetonitrile solution. The purity and the recovery rate were confirmed by HPLC.

The following experiments were performed on the metal salts shown in Run 30 of Table 11. To compound 6 (10.0 mg), a solution (0.200 mL) prepared by dispensing 0.050 mL of a solution of $Mg(OAc)_2$-$4H_2O$ (13.5 mg) in aqueous ethanol solution (0.450 mL) and diluting with 0.150 mL of ethanol was added, and the mixture was concentrated and vacuum-dried. To the obtained solid, acetonitrile (0.3 mL) and water (0.6 mL) were added and the mixture was stirred for 4 days, then the resulting oil-like precipitate and solution were centrifuged. After the solvent was distilled off, the vacuum-dried supernatant and precipitate were re-dissolved in ethanol (1.0 mL). 15 μL of the obtained ethanol solution (1.0 mL) of the supernatant and precipitate was sampled, the solvent was distilled off, and a sample was prepared so that the entire solution was 0.5 mL of a 50%v/v aqueous acetonitrile solution. The purity and the recovery rate were confirmed by HPLC.

The following experiments were performed on the metal salts shown in Run 31 of Table 11. To compound 6 (9.9 mg), a 35 mM aqueous ethanol solution (0.200 mL) prepared by diluting an aqueous ethanol solution (0.475 mL) of $MgCl_2$-$6H_2O$ (9.5 mg) was added, and the mixture was concentrated and vacuum-dried. To the obtained solid, acetonitrile (0.3 mL) and water (0.6 mL) were added and the mixture was stirred for 4 days, then the resulting oil-like precipitate and solution were centrifuged. After the solvent was distilled off, the vacuum-dried supernatant and precipitate were re-dissolved in ethanol (1.0 mL). 15 μL of the obtained ethanol solution (1.0 mL) of the supernatant and precipitate was sampled, the solvent was distilled off, and a sample was prepared so that the entire solution was 0.5 mL of a 50%v/v aqueous acetonitrile solution. The purity and the recovery rate were confirmed by HPLC.

Table 11 shows the purity and the recovery rate of the supernatant and solid, and the ratios of isomer 1 or isomer 2 to compound 6 and the purification efficiency to confirm how much isomer 1 and isomer 2 were separated from compound 6 with respect to compound 6 calculated by the following expressions.

- Ratio of isomer 1 to compound 6 x 100:

$$(\text{Peak area of isomer 1}) / (\text{Peak area of compound 6}) \times 100$$

- Ratio of isomer 2 to compound 6 x 100:

$$(\text{Peak area of isomer 2}) / (\text{Peak area of compound 6}) \times 100$$

- Purification efficiency of isomer 1 (%):

{1 - (Ratio of isomer 1 to compound 6)/(Ratio of isomer 1 to compound 6 in preparation)} × 100

- Purification efficiency of isomer 2 (%):

{1 - (Ratio of isomer 2 to compound 6)/(Ratio of isomer 2 to compound 6 in preparation)} × 100

For Run 29, the ratio and purification efficiency were calculated for an individual impurity. The ratio of the impurity (relative retention time 0.71, molecular weight 1797.06, estimated from LC-MS/MS as linear analogue byproduct peptide) to compound 6 and the purification efficiency to confirm how much the impurity was separated from compound 6 with respect to compound 6 were calculated by the following expressions.

- Ratio of impurity to compound 6 x 100:

(Peak area of impurity) / (Peak area of compound 6) x 100

- Purification efficiency of impurity (%):

{1 - (Ratio of impurity to compound 6)/ (Ratio of impurity 1 to compound 6 of reference)} × 100

It was found that, when lithium iodide (LiI, Run 2, purity of supernatant 88%, recovery rate 78%), lithium perchlorate ($LiClO_4$, Run 3, purity of supernatant 91%, recovery rate 60%), or ytterbium(III) trifluoromethanesulfonate ($Yb(OTf)_3$, Run 21, purity of supernatant 87%, recovery rate 32%) was used, the purity of supernatant was 3% or more higher than the purity of the preparation of 84%. In particular, when lithium iodide and lithium perchlorate were used, the purification efficiency of isomer 1 was 60% and 73%, respectively, and the purification efficiency of isomer 2 was 23% and 43%, respectively, and a certain amount of both isomers was successfully removed. On the other hand, when ytterbium(III) trifluoromethanesulfonate was used, the purification efficiency of isomer 2 was lowered to -10%, but the purification efficiency of isomer 1 was high, that was 58%, resulting the improvement of purity to 87%.

It was found, when indium trifluoromethanesulfonate ($In(OTf)_3$, Run 24, purity of supernatant 89%, recovery rate 37%) was used, the purity of the supernatant was found to be 5% higher than the purity of the preparation 84%, but compound 6, isomer 1, and isomer 2 each were isomerized, and the proportion of each in the preparation and the proportion of each after the purification operation changed.

It was found that, when potassium iodide (KI, Run 4, purity of solid 90%, recovery rate 57%), magnesium bromide ($MgBr_2$, Run 9, purity of solid 98%, recovery rate 43%), magnesium perchlorate ($Mg(ClO_4)_2$, Run 10, purity of solid 90%, recovery rate 82%), magnesium iodide ($MgI_2$, Run 11, purity of solid 90%, recovery rate 73%), magnesium trifluoromethanesulfonate ($Mg(OTf)_2$, Run 12, purity of solid 89%, recovery rate 41%), scandium(III) trifluoromethanesulfonate ($Sc(OTf)_3$, Run 18, purity of solid 87%, recovery rate 40%), samarium(III) trifluoromethanesulfonate ($Sm(OTf)_3$, Run 19, purity of solid 87%, recovery rate 33%), cerium trifluoromethanesulfonate ($Ce(OTf)_3$, Run 20, purity of solid 88%, recovery rate 63%), or manganese bis(trifluoromethanesulfonate) ($Mn(OTf)_2$, Run 23, purity of solid 90%, recovery rate 50%) was used, the purity of the precipitated solid was 3% or more higher than the purity of the preparation of 84%. In particular, when magnesium bromide was used, the purification efficiencies of isomer 1 and isomer 2 were high, that were 88% and 82%, respectively, and the purity was 98%. On the other hand, when magnesium trifluoromethanesulfonate and samarium(III) trifluoromethanesulfonate were used, the purification efficiencies of isomer 1 in each case were low, that were 2% and 12%, respectively, but the purification efficiencies of isomer 2 were 59% and 53%, respectively, resulting the improvements of purity to 89% or 87%, respectively. For other metal salts, it was found that the purification efficiency of isomer 1 was 19% or more and the purification efficiency of isomer 2 was 34% or more, which shows that both isomers could be removed with balance (Runs 4, 10, 11, 18, 20, and 23).

When potassium tetrafluoroborate ($KBF_4$, Run 5) or potassium hexafluorophosphate ($KPF_6$, Run 6) was used, the metal salt used when dichloromethane and hexane were added precipitated, and no solid containing compound 6 was obtained. In addition, there was no change in the purity of the supernatant and the purity of the preparation. It is considered that this is because, during the experiment process, metal salts precipitated, and compound 6 and the metal were unable to form a composite.

For magnesium sulfate ($MgSO_4$, Run 13), magnesium acetate tetrahydrate ($Mg(OAc)_2 \cdot 4H_2O$, Run 14), magnesium chloride ($MgCl_2$, Run 15), and magnesium fluoride ($MgF_2$, Run 16), when compound 6 and each metal salt were mixed and then acetonitrile was added, the metal salt was not dissolved and a suspension was generated, and as a result of continuing the experiment as it was, no solid containing compound 6 was obtained. In addition, there was no change in the purity of the supernatant and the purity of the preparation. It is considered that this is because the metal salt did not

dissolve in acetonitrile, and compound 6 and the metal were unable to form a composite.

On the other hand, as a result of using ethanol (or 60% ethanol water) as a solvent for composite formation in Runs 27 to 31, the purification effect could be confirmed in the supernatants of Runs 27 and 28 and in the solids of Runs 30 and 31. Although there was no significant improvement in purity in Run 29, the purification effect was confirmed by paying attention to the peak of relative retention time 0.71 as shown in Figure 8 and calculating the purification efficiency, resulting that the purification efficiency was -131% in the solid and 100% (not observed) in the supernatant. It is considered that the purification effect was observed in Runs 27 to 31 because both the metal salt and compound 6 (or impurity) were dissolved in ethanol (or 60% ethanol water) and mixed to be capable of forming a composite.

It was found that, when barium iodide ($BaI_2$, Run 7, purity of solid 85%, recovery rate 54%), barium perchlorate ($Ba(ClO_4)_2$, Run 8, purity of solid 86%, recovery rate 65%), or zinc(II) trifluoromethanesulfonate ($Zn(OTf)_2$, Run 22, purity of solid 86%, recovery rate 58%) were used, the purity of the precipitated solid was improved by 1 to 2% compared to the purity of the preparation, but the purification efficiency of isomer 2 was 23 to 27%. On the other hand, the purification efficiency of isomer 1 was 10% or less for all of them. From these results, it was confirmed that isomer 2 can be removed to some extent when these metal salts are used.

It was found that, when calcium bromide ($CaBr_2$, Run 17, purity of supernatant 86%, recovery rate 45%) was used, the purity of the supernatant was improved by 2% compared to the purity of the preparation, but the purification efficiency of isomer 1 was 59%. On the other hand, the purification efficiency of isomer 2 was -19%, which hindered the improvement of purity. From these results, it was confirmed that isomer 1 can be removed to some extent when these metal salts are used.

When bismuth tris(trifluoromethanesulfonate) ($Bi(OTf)_3$, Run 25) was used, compound 6 was decomposed, thus the purification effect could not be confirmed.

When silver trifluoromethanesulfonate (AgOTf, Run 26) was used, no solid precipitation was observed, and there was no change in the purity of the supernatant and the purity of the preparation. It is considered from the spectral analysis of $^1$H-NMR in Reference Example 2 that the purity of the supernatant was not increased because compound 6 and silver trifluoromethanesulfonate do not form a composite. It is also considered that the same results as Run 26 can be obtained when the experiment is performed in the same manner but under no metal addition, since compound 6 and silver trifluoromethanesulfonate do not form a composite.

<u>Calculation Based on Peak Area of Preparation (Recovery Rate Calculation Method 3)</u>

Calculation of Solid Recovery Rates of Runs 11, 12, 13, 18, 19, and 23 (examples)

After HPLC of the preparation of 0.306 mg/mL solution was measured, the peak area of the target (60127) was measured. 4 μL of the solid acetonitrile solution obtained in the above experiment was diluted with 396 μL of acetonitrile and measured by HPLC, and then the peak area of the target was measured, and the recovery rate of the target in the supernatant or solid was calculated based on the area of the preparation.

$MgI_2$:

$$(42893 / 15.05 \text{ x } 5) / (60127 / 3.06) \text{ x } 100 \approx 73\%$$

$Mg(OTf)_2$:

$$(24183 / 15.07 \text{ x } 5) / (60127 / 3.06) \text{ x } 100 \approx 41\%$$

$MgSO_4$:

$$(257 / 15.04 \text{ x } 5) / (60127 / 3.06) \text{ x } 100 \approx 0\%$$

$Sc(OTf)_3$:

$$(23815 / 14.99 \text{ x } 5) / (60127 / 3.06) \text{ x } 100 \approx 40\%$$

$Sm(OTf)_3$:

$$(19507 / 15.05 \times 5) / (60127 / 3.06) \text{ x } 100 \approx 33\%$$

$Mn(OTf)_2$:

$$(29747 / 15.00 \text{ x } 5) / (60127 / 3.06) \text{ x } 100 \approx 50\%$$

Calculation of Recovery Rate of Run 27

In Runs 27 to 31, the recovery rates of supernatant and solid were calculated by the above-described recovery rate calculation method 1. The calculation of the supernatant and solid recovery rate of Run 27 will be described as an example.
After HPLC of the preparation of 0.30 mg/mL solution was measured, the peak area of the target (2950511, 220 nm) was measured. HPLC of the prepared 50%v/v aqueous acetonitrile solution was measured, and the peak area of the target (supernatant: 2704577, solid: 234383) was measured, and the recovery rate was calculated according to the following method.

Supernatant:

$$(2704577 \text{ x } 10)/(2950511 \text{ x } 10.0) \text{ x } 100 \approx 92\%$$

Solid:

$$(234383 \text{ x } 10)/(2950511 \text{ x } 10.0) \text{ x } 100 \approx 8\%$$

From the above results, it was confirmed that, depending on the type of metal salt that forms a composite with the cyclic peptide, there is a case that leads or not to a purification effect on the cyclic peptide that is a purification target.

From the above, it was confirmed that the relative solubility of the composite and an impurity other than the composite can be changed by composite formation of the cyclic peptide that is a purification target with a metal ion, and that the purification of the cyclic peptide of the target can be carried out by utilizing the difference in their solubilities. It was also found that this method, in particular, can remove a certain amount of isomers of cyclic peptide that is a purification target, which are generally considered to be difficult to separate.

[Example 2-2: Confirmation of Dependence on Experimental Conditionsl

< Basic Operation >

Compound 6 was mixed with about 1 molar equivalent of magnesium perchlorate and dissolved in acetonitrile. The solvent was distilled off under reduced pressure, dichloromethane and hexane were added, and the mixture was stirred at room temperature according to the following. The resulting solid and liquid were centrifuged and the supernatant was sampled for HPLC measurement. The purity and the recovery rate calculated based on the values of peak area percentage and peak area of each compound are shown in Tables 14 and 15.

The sample for HPLC measurement was prepared to be about 0.3 mg/mL based on the initial concentration. In Example 2-2, HPLC was measured under purity analysis condition B using ACD/Spectrus manufactured by Advanced Chemistry Development, Inc. as the HPLC analysis software, and the purity and the recovery rate were calculated based on the peak area percentage and peak area at the stage where only dichloromethane was added in each experimental operation. It should be noted that, since the near 100% total recovery rate of solid and supernatant and the purity improvement of solid have already been confirmed in the similar experiment performed on Run 1 in Table 8, the solid recovery experiment was omitted in this experiment, and it was determined that there was a purification effect when the purity of the supernatant decreased from the reference value.

Recovery Rate Calculation Method 4

For example, assuming that the experiment was performed using *a* mg of raw material and the amount of solution used was b mL, when measuring the HPLC of the supernatant, the solid and the solution were centrifuged, and after sampling the supernatant (150 *b*/*a*) μL, the solvent was distilled off, and a sample was prepared so that the entire solution was 0.5 mL of a 50%v/v aqueous acetonitrile solution. When HPLC of each of the prepared samples is measured, and the

peak areas (220 nm) of the reference sample and the target after solvent addition are set to c and d, respectively, the recovery rate is expressed by the following expression.

[Expression 4]

$$[c \div \{(a \div b) \times (150b / a) \div 1000 \div 0.5\}] \div \{d \div 0.3\} \times 100(\%)$$

$$\Rightarrow \frac{c}{d} \times 100\ (\%)$$

[Example 2-2-1: Confirmation on Time Dependence]

[Table 14]

| Run | | 0 min | 1 min | 10 min | 30 min | 1 hr | 2 hrs | 6 hrs | 2 days |
|---|---|---|---|---|---|---|---|---|---|
| 1 | Recovery rate of supernatant (%) | 100% | 34% | 21% | 17% | 17% | 16% | 16% | 18% |
| | Purity of supernatant (%) | 80% | 73% | 66% | 61% | 59% | 61% | 59% | 59% |
| 2 | Recovery rate of supernatant (%) | 100% | 46% | 39% | 36% | 31% | 35% | 34% | 37% |
| | Purity of supernatant (%) | 80% | 73% | 71% | 70% | 69% | 70% | 70% | 71% |
| 3 | Recovery rate of supernatant (%) | 100% | 61% | 53% | 24% | 13% | 13% | 13% | 14% |
| | Purity of supernatant (%) | 81% | 78% | 76% | 69% | 59% | 60% | 60% | 61% |

Runs 1 to 3 of Table 14

To compound 6 (Run 1: 15.0 mg, Run 2: 15.1 mg, Run 3: 15.1 mg), a 53 mM acetonitrile solution (each 0.200 mL) prepared by dispensing an acetonitrile solution (0.423 mL) of $Mg(ClO_4)_2$ (12.7 mg) (Run 1: 0.094 mL, Run 2: 0.095 mL, Run 3: 0.095 mL) and diluting with acetonitrile was added, and the mixture was concentrated and vacuum-dried. To the obtained solid, dichloromethane (1.0 mL) was added, and the mixture was stirred for 15 minutes, then 10 μL was sampled from the solution. Hexane (0.5 mL) was added to each solution and the mixture was stirred for 1 minute, then the resulting solid and liquid were quickly centrifuged, and 15 μL was sampled from the supernatant. After stirring again and 10 minutes after the addition of hexane, the solution was centrifuged again and the supernatant (15 μL) was sampled. Similarly, centrifugation of each solution and sampling of the supernatant (15 μL) were performed at a stage 30 minutes, 1 hour, 2 hours, 6 hours, and 2 days after the addition of hexane. For the sampled supernatant of each stage, the solvent was distilled off, and a sample was prepared so that the entire solution was 50 μL of a 50%v/v aqueous acetonitrile solution. As the results of confirming the purity and the recovery rate by HPLC, the purity of the supernatant decreased at any of the times and a purification effect was observed. In addition, since the purity and the recovery rate were almost unchanged 1 hour after the addition of hexane, it is considered that the approximate of equilibrium is about 1 hour.

Calculation Example of Supernatant Recovery Rate

In Run 1, the peak area of the target at the stage of adding only dichloromethane (3152623360, 225 nm) and the peak area of the target at 1 hour after the addition of hexane (523598976, 225 nm) were measured. The recovery rate was calculated according to the following method.

$$5253598976 / 3125623360 \times 100 \approx 17\%$$

From the above results, it was confirmed that there is a purification effect of the cyclic peptide on the composite of magnesium perchlorate with the cyclic peptide in any cases from immediately after to after allowing sufficient time after the second solvent addition. It was also confirmed that the purification effect becomes constant after a certain period of time.

[Example 2-2-2: Confirmation on Concentration Dependence]

[Table 15]

| Run | Amount of compound 6 | Solvent amount | | Properties | Purity of supernatant | Recovery rate (supernatant) |
|---|---|---|---|---|---|---|
| | | Dichloromethane | Hexane | | | |
| 1 | 1.5 mg | 1.0 mL | None | Solution | - | - |
| | | 1.0 mL | 0.5 mL | Solution | - | - |
| 2 | 1.5 mg | 1.0 mL | None | Solution | 81% | - |
| | | 0.75 mL | 0.75 mL | Solid precipitate | 78% | 51% |
| 3 | 7.5 mg | 1.0 mL | None | Solution | 81% | - |
| | | 1.0 mL | 0.5 mL | Solid precipitate | 68% | 29% |
| 4 | 15 mg | 1.0 mL | None | Solution | 80% | - |
| | | 1.0 mL | 0.5 mL | Solid precipitate | 62% | 21% |
| 5 | 45 mg | 1.0 mL | None | Solution | 82% | - |
| | | 1.0 mL | 0.5 mL | Solid precipitate | 59% | 9% |

Run 1 of Table 15

To compound 6 (Run 1: 1.5 mg), a 5.3 mM acetonitrile solution (0.200 mL) prepared by diluting 0.0084 mL of an acetonitrile solution (0.550 mL) of $Mg(ClO_4)_2$ (16.7 mg) with 0.1916 mL of acetonitrile was added, and the mixture was concentrated and vacuum-dried. To the obtained solid, dichloromethane (1.0 mL) was added and the mixture was stirred for 15 minutes, then hexane (0.5 mL) was added and the mixture was stirred for 7 hours, but no precipitation occurred.

Run 2 of Table 15

To compound 6 (Run 2: 1.5 mg), a 5.3 mM acetonitrile solution (0.200 mL) prepared by diluting 0.0084 mL of an acetonitrile solution (0.550 mL) of $Mg(ClO_4)_2$ (16.7 mg) with 0.1916 mL of acetonitrile was added, and the mixture was concentrated and vacuum-dried. To the obtained solid, dichloromethane (1.0 mL) was added, and the mixture was stirred for 15 minutes, then 10 μL was sampled from the solution. After the solvent was distilled off and the resultant was vacuum-dried, dichloromethane (0.75 mL) was added to redissolve, and hexane (0.75 mL) was added, and the mixture was stirred overnight, then the resulting solid and liquid were centrifuged, and 15 μL was sampled from the supernatant. For the sampled supernatant of each stage, the solvent was distilled off, and a sample was prepared so that the entire solution was 50 μL of a 50%v/v aqueous acetonitrile solution. As the results of confirming the purity and the recovery rate by HPLC, the purity of the supernatant decreased and a purification effect was observed.

Run 3 of Table 15

To compound 6 (Run 3: 7.5 mg), a 26 mM acetonitrile solution (0.200 mL) prepared by diluting 0.047 mL of an acetonitrile solution (0.550 mL) of $Mg(ClO_4)_2$ (16.7 mg) with 0.153 mL of acetonitrile was added, and the mixture was concentrated and vacuum-dried. To the obtained solid, dichloromethane (1.0 mL) was added, and the mixture was stirred for 15 minutes, then 10 μL was sampled from each solution. Hexane (0.5 mL) was added to each solution and the mixture was stirred for 7 hours, then centrifugation was performed, and 15 μL was sampled from the supernatant of each solution. For the sampled supernatant of each stage, the solvent was distilled off, and a sample was prepared so that the entire solution was 250 μL, 1.5 mL of a 50%v/v aqueous acetonitrile solution to match the concentrations. As the results of confirming the purity and the recovery rate by HPLC, the purity of the supernatant decreased in any of the cases and a purification effect was observed.

Run 4 of Table 15

To compound 6 (15.1 mg), a 53 mM acetonitrile solution (0.200 mL) prepared by diluting 0.143 mL of an acetonitrile solution (0.387 mL) of $Mg(ClO_4)_2$ (7.7 mg) with 0.057 mL of acetonitrile was added, and the mixture was concentrated and vacuum-dried. To the obtained solid, dichloromethane (1.0 mL) was added, and the mixture was stirred for 15 minutes, then 10 μL was sampled from the solution. Hexane (0.5 mL) was added and the mixture was stirred

overnight, then the resulting solid and liquid were centrifuged, and 15 μL was sampled from the supernatant. For the sampled supernatant of each stage, the solvent was distilled off, and a sample was prepared so that the entire solution was 0.5 mL of a 50%v/v aqueous acetonitrile solution. As a result of confirming the purity and the recovery rate of the supernatant by HPLC, it was found that the purity of the reference was 80%, while the purity of the supernatant was 62% and the recovery rate was 21%, it was indicated that there was a purification effect.

Run 5 of Table 15

To compound 6 (Run 5: 45.0 mg), a 63 mM acetonitrile solution (0.500 mL) prepared by diluting 0.283 mL of an acetonitrile solution (0.550 mL) of $Mg(ClO_4)_2$ (16.7 mg) with 0.217 mL of acetonitrile was added, and the mixture was concentrated and vacuum-dried. To the obtained solid, dichloromethane (1.0 mL) was added, and the mixture was stirred for 15 minutes, then 10 μL was sampled from each solution. Hexane (0.5 mL) was added to each solution and the mixture was stirred for 7 hours, then centrifugation was performed, and 15 μL was sampled from the supernatant of each solution. For the sampled supernatant of each stage, the solvent was distilled off, and a sample was prepared so that the entire solution was 250μL, 1.5 mL of a 50%v/v aqueous acetonitrile solution to match the concentrations. As the results of confirming the purity and the recovery rate by HPLC, the purity of the supernatant decreased in any of the cases and a purification effect was observed.

It is considered that the precipitation did not occur in Run 1 because the amount of the cyclic peptide was smaller than that of Runs 3 to 5, and the amount of composite of magnesium perchlorate with the cyclic peptide produced was smaller. On the other hand, it is considered that the precipitation occurred in Run 2 because, although the amount and concentration of the cyclic peptide were the same as those of Run 1 (concentration: 1 mg/mL), the solubility of composite of the magnesium perchlorate with the cyclic peptide produced was lower than that of Run 1 since the ratio of hexane, which is a poor solvent, was higher than that of Run 1.

[Example 2-3: Example Using Cyclic Peptides Having Different Numbers of Amino Acid Residues in Cyclic Moiety]

[Example 2-3-1: Confirmation of Substrate Generality of Purification Effect using $Mg(ClO_4)_2$ and Dichloromethane/Hexane]

< Basic Operation >

Compounds 9, 11, 12 to 15 (each about 10 mg) having different numbers of amino acid residues in the cyclic moiety of the cyclic peptide each was mixed with about 1 molar equivalent of magnesium perchlorate and dissolved in acetonitrile. The solvent was distilled off under reduced pressure, dichloromethane and hexane were added, and the mixture was stirred at room temperature according to the following. The resulting solid and liquid were centrifuged, the solvents were distilled off, and vacuum-dried supernatant and solid were re-dissolved in acetonitrile, and HPLC of each were measured. HPLC analysis was performed using ACD/Spectrus manufactured by Advanced Chemistry Development, Inc. The purity and the recovery rate calculated based on the values of peak area percentage and peak area (measured at 225 nm) of each compound are shown in Table 16 and Table 17. The compounds used as raw materials were the compounds synthesized in Reference Examples 1 to 3 to 8. Based on the analysis of HPLC measured under the same conditions (purity analysis condition C) as the HPLC used in the above purity analysis, it was determined that there was a purification effect when the purity was improved. In addition, even when the purity was not improved from the value before the operation, attention was paid to individual impurities, and it was determined that there was a purification effect for those in which the purification efficiency was a positive value.

The sample for HPLC measurement was prepared to be about 0.3 mg/mL based on the initial concentration. In Example 2-3, HPLC was measured under purity analysis condition C. In this experiment, the purity and the recovery rate were calculated based on the peak area percentage and peak area at the stage where dichloromethane (0.66 mL) was added in each experimental operation. The recovery rate was calculated by the recovery rate calculation method 4 described above. For some, attention was paid to individual impurities, and the ratio and purification efficiency were calculated on the impurities.

[Table 16]

| Run | Compound | Metal addition | Supernatant | | | | | Solid | | | | |
|---|---|---|---|---|---|---|---|---|---|---|---|---|
| | | | Recovery rate | Impurity 1/ Compound 9 x100 | Purification efficiency | Impurity 2/ Compound 9 x100 | Purification efficiency | Recovery rate | Impurity 1/ Compound 9 x100 | Purification efficiency | Impurity 2/ Compound 9 x100 | Purification efficiency |
| 1 | Compound 9 | None | 91% | 1.00 | 2% | 0.73 | -7% | - | - | - | - | - |
| 2 | | $Mg(ClO_4)_2$ | 35% | 0.47 | 62% | 0.30 | 67% | 60% | 1.76 | -42% | 1.28 | -42% |

For compound 9, the ratio and purification efficiency were calculated for individual two impurities. The ratio of impurity 1 (relative retention time 0.88, molecular weight 2604.69, estimated from LC-MS/MS as linear dimer analogue byproduct peptide) or impurity 2 (relative retention time 0.89, molecular weight 2618.71, estimated from LC-MS/MS as linear dimer analogue byproduct peptide) to compound 9 and the purification efficiency to confirm how much impurity 1 and impurity 2 were separated from compound 9 with respect to compound 9 were calculated by the following expressions.

- Ratio of impurity 1 to compound 9 x 100:

$$(\text{Peak area of impurity 1}) / (\text{Peak area of compound 9}) \times 100$$

- Ratio of impurity 2 to compound 9 x 100:

$$(\text{Peak area of impurity 2}) / (\text{Peak area of compound 9}) \times 100$$

- Purification efficiency of impurity 1 (%):

{1 - (Ratio of impurity 1 to compound 9)/(Ratio of impurity 1 to compound 9 of reference)} × 100

- Purification efficiency of impurity 2 (%):

{1 - (Ratio of impurity 2 to compound 9)/(Ratio of impurity 2 to compound 9 of reference)} × 100

Run 1 of Table 16

To compound 9 (10.0 mg), acetonitrile (0.2 mL) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours, and then centrifugation was performed, but no precipitation occurred. 20 μL was sampled from the supernatant. A sample was prepared so that the entire solution was 0.5 mL of a 50% v/v aqueous acetonitrile solution.

Run 2 of Table 16

To compound 9 (10.2 mg), 0.217 mL of a separately prepared acetonitrile solution (0.99 mL) of $Mg(ClO_4)_2$ (9.9 mg) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours. After centrifugation, solid was precipitated, and 20 μL was sampled from the supernatant. The solid and liquid were centrifuged, the solvent was distilled off, then the vacuum-dried solid was re-dissolved in acetonitrile (1.0 mL), and 15 μL was sampled from the solution. A sample was prepared so that the entire solution was 0.5 mL of a 50%v/v aqueous acetonitrile solution. As the result of confirming the purity and the recovery rate by HPLC, it was 92%, and there was no improvement over the initial purity of 92%. On the other hand, when the purification efficiencies of impurity 1 (relative retention time 0.88) and impurity 2 (0.89) were calculated as shown in Figure 9, it was shown that the purification efficiencies of impurity 1 and impurity 2 with respect to compound 9 in the supernatant were 67% and 62%, respectively, thus it was determined that the supernatant was purified.

[Table 17]

| Run | Compound | Metal addition | Solvent amount | | Initial purity | Supernatant | | Solid | |
|---|---|---|---|---|---|---|---|---|---|
| | | | DCM | Hexane | | Purity | Recovery rate | Purity | Recovery rate |
| 1 | Compound 12 | None | 0.66 mL | 0.66 mL | 86% | 84% | 101% | - | - |
| 2 | | $Mg(ClO_4)_2$ | 0.66 mL | 0.66 mL | 83% | 93% | 48% | 75% | 48% |
| 3 | Compound 14 | None | 0.66 mL | 0.66 mL | 84% | 85% | 91% | - | - |
| 4 | | $Mg(ClO_4)_2$ | 0.66 mL | 0.66 mL | 84% | 93% | 61% | 72% | 36% |

(continued)

| Run | Compound | Metal addition | Solvent amount | | Initial purity | Supernatant | | Solid | |
|---|---|---|---|---|---|---|---|---|---|
| | | | DCM | Hexane | | Purity | Recovery rate | Purity | Recovery rate |
| 5 | Compound 11 | None | 0.66 mL | 0.66 mL | 92% | 93% | 95% | - | - |
| 6 | | $Mg(ClO_4)_2$ | 0.66 mL | 0.66 mL | 92% | 96% | 75% | 84% | 28% |
| 7 | Compound 13 | None | 0.66 mL | 0.66 mL | 92% | 92% | 94% | - | - |
| 8 | | $Mg(ClO_4)_2$ | 0.66 mL | 0.66 mL | 92% | 96% | 69% | 85% | 43% |
| 9 | Compound 15 | None | 0.66 mL | 1.32 mL | 90% | 90% | 95% | - | - |
| 10 | | $Mg(ClO_4)_2$ | 0.66 mL | 1.32 mL | 90% | 95% | 82% | 76% | 22% |

Run 1 of Table 17

To compound 12 (9.8 mg), acetonitrile (0.2 mL) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours, and then centrifugation was performed, but no precipitation occurred. 20 μL was sampled from the supernatant. A sample was prepared so that the entire solution was 0.5 mL of a 50%v/v aqueous acetonitrile solution.

Run 2 of Table 17

To compound 12 (10.1 mg), 0.207 mL of a separately prepared acetonitrile solution (0.99 mL) of $Mg(ClO_4)_2$ (9.9 mg) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours. After centrifugation, solid was precipitated, and 20 μL was sampled from the supernatant. The solid and liquid were centrifuged, the solvents were distilled off, then the vacuum-dried solids were re-dissolved in acetonitrile (1.0 mL), and 15 μL was sampled from each solution. Samples were prepared so that the entire solution was 0.5 mL of a 50%v/v aqueous acetonitrile solution. As the results of confirming the purity and the recovery rate by HPLC, it was confirmed that any of the supernatants were purified.

Run 3 of Table 17

To compound 14 (10.0 mg), acetonitrile (0.2 mL) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours, and then centrifugation was performed, but no precipitation occurred. 20 μL was sampled from the supernatant. A sample was prepared so that the entire solution was 0.5 mL of a 50%v/v aqueous acetonitrile solution.

Run 4 of Table 17

To compound 14 (10.0 mg), 0.169 mL of a separately prepared acetonitrile solution (0.99 mL) of $Mg(ClO_4)_2$ (9.9 mg) was added, and the obtained solution was concentrated and vacuum-dried. Then, to the obtained solid, dichloromethane (0.66 mL) was added, and the mixture was stirred for 15 minutes, then 10 μL was sampled. Hexane (0.33 mL) was added and the mixture was stirred overnight, then hexane (0.33 mL) was further added and the mixture was stirred for 3.5 hours. After centrifugation, solid was precipitated, and 20 μL was sampled from the supernatant. The solid and liquid were centrifuged, the solvents were distilled off, then the vacuum-dried solids were re-dissolved in acetonitrile (1.0 mL), and 15 μL was sampled from each solution. Samples were prepared so that the entire solution was 0.5 mL of a 50%v/v aqueous acetonitrile solution. As the results of confirming the purity and the recovery rate by HPLC, it was confirmed that any of the supernatants were purified.

Run 5 of Table 17

An acetonitrile solution (0.200 mL) of compound 11 (9.9 mg) was concentrated and vacuum-dried. Then, to the obtained solid, dichloromethane (0.66 mL) was added, and the mixture was stirred for 15 minutes, then 10 μL was sampled. Hexane (0.66 mL) was added and the mixture was stirred for 4.5 hours, and then centrifugation was performed, but no precipitation occurred. 20 μL was sampled from the supernatant. For each sampled solution, the solvent was distilled off, and a sample was prepared so that the entire solution was 0.5 mL of a 50% v/v aqueous acetonitrile solution.

Run 6 of Table 17

To compound 11 (10.1 mg), a 36 mM acetonitrile solution (0.200 mL) prepared by diluting an acetonitrile solution (0.380 mL) of $Mg(ClO_4)_2$ (3.8 mg) was added, and the mixture was concentrated and vacuum-dried. Then, to the obtained solid, dichloromethane (0.66 mL) was added, and the mixture was stirred for 15 minutes, then 10 μL was sampled. Hexane (0.66 mL) was added and the mixture was stirred for 4.5 hours. After centrifugation, solid was precipitated, and 20 μL was sampled from the supernatant. The solid and liquid were centrifuged, the solvents were distilled off, then the vacuum-dried solids were re-dissolved in acetonitrile (1.0 mL), and 15 μL was sampled from each solution. For each sampled solution, the solvent was distilled off, and a sample was prepared so that the entire solution was 0.5 mL of a 50%v/v aqueous acetonitrile solution. As the results of confirming the purity and the recovery rate by HPLC, it was confirmed that the supernatant was purified.

Run 7 of Table 17

An acetonitrile solution (0.200 mL) of compound 13 (9.9 mg) was concentrated and vacuum-dried. Then, to the obtained solid, dichloromethane (0.66 mL) was added, and the mixture was stirred for 15 minutes, then 10 μL was sampled. Hexane (0.66 mL) was added and the mixture was stirred for 4.5 hours, and then centrifugation was performed, but no precipitation occurred. 20 μL was sampled from the supernatant. For each sampled solution, the solvent was distilled off, and a sample was prepared so that the entire solution was 0.5 mL of a 50%v/v aqueous acetonitrile solution.

Run 8 of Table 17

To compound 13 (9.9 mg), a 32 mM acetonitrile solution (0.200 mL) prepared by diluting an acetonitrile solution (0.410 mL) of $Mg(ClO_4)_2$ (4.1 mg) was added, and the mixture was concentrated and vacuum-dried. To the obtained solid, dichloromethane (0.66 mL) was added, and the mixture was stirred for 15 minutes, then 10 μL was sampled. Hexane (0.66 mL) was added and the mixture was stirred for 4.5 hours. After centrifugation, solid was precipitated, and 20 μL was sampled from the supernatant. The solid and liquid were centrifuged, the solvents were distilled off, then the vacuum-dried solids were re-dissolved in acetonitrile (1.0 mL), and 15 μL was sampled from each solution. For each sampled solution, the solvent was distilled off, and a sample was prepared so that the entire solution was 0.5 mL of a 50%v/v aqueous acetonitrile solution. As the results of confirming the purity and the recovery rate by HPLC, it was confirmed that the supernatant was purified.

Run 9 of Table 17

An acetonitrile solution (0.200 mL) of compound 15 (9.9 mg) was concentrated and vacuum-dried. Then, to the obtained solid, dichloromethane (0.66 mL) was added, and the mixture was stirred for 15 minutes, then 10 μL was sampled. Hexane (1.32 mL) was added and the mixture was stirred for 4.5 hours, and then centrifugation was performed, but no precipitation occurred. 30 μL was sampled from the supernatant. For each sampled solution, the solvent was distilled off, and a sample was prepared so that the entire solution was 0.5 mL of a 50%v/v aqueous acetonitrile solution.

Run 10 of Table 17

To compound 15 (9.9 mg), a 30 mM acetonitrile solution (0.200 mL) prepared by diluting an acetonitrile solution (0.470 mL) of $Mg(ClO_4)_2$ (4.7 mg) was added, and the mixture was concentrated and vacuum-dried. To the obtained solid, dichloromethane (0.66 mL) was added, and the mixture was stirred for 15 minutes, then 10 μL was sampled. Hexane (1.32 mL) was added and the mixture was stirred for 4.5 hours. After centrifugation, solid was precipitated, and 30 μL was sampled from the supernatant. The solid and liquid were centrifuged, the solvent was distilled off, then the vacuum-dried solid was re-dissolved in acetonitrile (1.0 mL), and 15 μL was sampled from each solution. For each sampled solution, the solvent was distilled off, and a sample was prepared so that the entire solution was 0.5 mL of a 50%v/v aqueous acetonitrile solution. As the results of confirming the purity and the recovery rate by HPLC, it was confirmed that the supernatant was

purified.

From the above, it was found that the purification method of the cyclic peptide of the present invention can also be applied to cyclic peptides having different numbers of amino acid residues in the cyclic moiety and cyclic peptides having branched chains.

[Example 2-3-2: Confirmation of Substrate Generality using $MgI_2$ and Various Solvents]

< Basic Operation >

Each compound shown in Table 18 below (about 10 mg), which are cyclic peptides having different numbers of amino acid residues in the cyclic moiety, was mixed with about 1 molar equivalent of magnesium iodide and dissolved in acetonitrile. The solvent was distilled off under reduced pressure, the various solvents shown in Table 18 below were added, and the mixture was stirred at room temperature according to the following. The resulting solid and liquid were centrifuged, the solvents were distilled off, and vacuum-dried supernatant and solid were re-dissolved in acetonitrile, and HPLC of each was measured under purity analysis condition C. HPLC analysis was performed using ACD/Spectrus manufactured by Advanced Chemistry Development, Inc. The purity and the recovery rate calculated based on the values of peak area percentage and peak area (measured at 225 nm) of each compound are shown in Table 18.

The sample for HPLC measurement was prepared to be about 0.3 mg/mL based on the initial concentration. In Example 2-3, HPLC was measured under purity analysis condition C. In this experiment, the purity and the recovery rate were calculated based on the peak area percentage and peak area at the stage where dichloromethane (0.66 mL) was added in Example 2-3-1. The recovery rate was calculated by the recovery rate calculation method 4 described above.

[Table 18]

| Run | Compound | Compound weight | Added $MgI_2$ solution amount (10 mg/mL) | Solvent (ratio,volume) | Initial purity | Supernatant | | Solid | |
|---|---|---|---|---|---|---|---|---|---|
| | | | | | | Purity | Recovery rate | Purity | Recovery rate |
| 1 | Compound 11 | 10.1 mg | 0.205 mL | Isopropyl acetate/-Heptane (15:1,0.8 mL) | 92% | 96% | 49% | 89% | 51% |
| 2 | Compound 15 | 10.1 mg | 0.172 mL | Isopropyl acetate/-Heptane (15:1,0.8 mL) | 90% | 96% | 61% | 80% | 37% |
| 3 | Compound 12 | 10.3 mg | 0.225 mL | Methyl ethyl ketone/Heptane (5:2,1.4 mL) | 86% | 88% | 59% | 79% | 44% |
| 4 | Compound 15 | 10.1 mg | 0.172 mL | Methyl ethyl ketone/Heptane (5:2,1.4 mL) | 90% | 92% | 49% | 89% | 43% |
| 5 | Compound 11 | 10.2 mg | 0.207 mL | Acetonitrile/TBME (3:2,1.0 mL) | 92% | 87% | 52% | 98% | 47% |
| 6 | Compound 15 | 10.2 mg | 0.173 mL | Acetonitrile/TBME (3:2,1.0 mL) | 90% | 95% | 66% | 84% | 33% |

Runs 1 to 6 of Table 18

To about 10 mg of each compound, 1 molar equivalent of a separately prepared acetonitrile solution (2.62 mL) of $MgI_2$ (26.2 mg) was added, and the obtained solution was concentrated and vacuum-dried. Then, to the obtained solid, each solvent shown in Runs 1 to 6 of Table 18 was added, and the mixture was stirred overnight. The resulting solid and solution were centrifuged, and the solvent was distilled off, then the vacuum-dried supernatant and solid were re-dissolved in acetonitrile (1.0 mL). 15 $\mu$L of the obtained acetonitrile solution (1.0 mL) of supernatant and solid was sampled, and a sample was prepared so that the entire solution was 0.5 mL of a 50%v/v aqueous acetonitrile solution. The purity and the

recovery rate were confirmed by HPLC. In Runs 1, 2, 3, 4 and 6, the supernatant was confirmed that the solid was purified. In Run 5, it was confirmed that the solid was purified.

From the above, it was found that the purification method of the cyclic peptide of the present invention can be carried out with various metal salts and solvents for cyclic peptides having different numbers of amino acid residues in the cyclic moiety and cyclic peptides having branched chains.

[Example 3: Extraction of Cyclic Peptides]

[Example 3-1: Study on Separation of Cyclic Peptide and Metal Salt]

< Experimental Operation 1 >

A solution of compound 6 (30.0 mg, 84% purity [peak area percentage]) and $Mg(ClO_4)_2$ (5.88 mg) in $CH_3CN$ (1.0 mL) was concentrated under reduced pressure, and the resulting solid was dissolved in 2 mL of dichloromethane, and 1 mL of hexane was further added, and the mixture was stirred for 3 hours. The obtained suspension was centrifuged and separated into solids and liquids, then the solid was added to a mixed solution of dichloromethane-hexane (2 mL/1 mL) and the mixture was stirred for an additional 3 hours. The obtained suspension was centrifuged and dried to obtain a solid (43.07 mg, 93% purity [peak area percentage]).

< Experimental Operation 2 >

To 1.13 mg of the solid obtained in Experimental operation 1, deuteroacetonitrile ($CD_3CN$, 0.6 mL) was added, and 0.5 mL of the resulting solution was used to measure $^1H$-NMR (Figure 10-a).

< Experimental Operation 3 >

To 1.02 mg of the solid obtained in Experimental operation 1, 1 mL of dichloromethane was added, and the mixture was left to dry overnight. Deuteroacetonitrile ($CD_3CN$, 0.5 mL) was added, and $^1H$-NMR was measured (Figure 10-b).

< Experimental Operation 4 >

To 1.14 mg of the solid obtained in Experimental operation 1, 1 mL of dichloromethane was added, and then 1 mL of water was added to perform a liquid-separation operation. The obtained organic phase and aqueous phase were separated, and 1 mL of water was added to the organic phase, and the liquid-separation operation was performed twice more. As a result of drying the obtained organic phase, 0.72 mg of solid was obtained. To the obtained solid, deuteroacetonitrile ($CD_3CN$, 0.5 mL) was added, and $^1H$-NMR was measured (Figure 10-c). For comparison, $^1H$-NMR spectra of raw material compound 6 (84% purity [peak area percentage]) were shown in Figure 10-d.

As a result of comparing the $^1H$-NMR spectra shown in Figure 10-a and Figure 10-d, $^1H$-NMR spectrum of the obtained solid (Figure 10-a) was different from the $^1H$-NMR spectrum of the raw material (Figure 10-d), thus it was confirmed that the obtained solid was a composite. As a result of comparing the $^1H$-NMR spectra shown in Figure 10-a and Figure 10-b, both showed the same spectrum, thus it was confirmed that the composite obtained in Experimental operation 1 was not decomposed even with the addition of dichloromethane.

As a result of comparing the $^1H$-NMR spectra shown in Figure 10-a, Figure 10-c, and Figure 10-d, the NMR spectrum obtained after the liquid-separation operation (Figure 10-c) are different from the solid $^1H$-NMR spectrum obtained in Experimental operation 1 (Figure 10-a) and are consistent with the main peaks of the $^1H$-NMR spectra of the raw material (Figure 10-d), thus it was confirmed that the composite was decomposed by the liquids-separation operation to obtain compound 6 under no metal addition.

From the above, it was found that $Mg(ClO_4)_2$ can be removed by subjecting the composite obtained in Experimental operation 1 to the liquid-separation operation using water and dichloromethane.

[Example 3-2: Extraction of Cyclic Peptides]

< Experimental Operation 1 (Extraction of Composite and Purification) >

A solution of compound 6 (15.17 mg, 84% purity [peak area percentage]) and $Mg(ClO_4)_2$ (2.95 mg) in $CH_3CN$ (0.5 mL) was concentrated under reduced pressure, and the resulting solid was dissolved in 1 mL of dichloromethane, and 0.6 mL of hexane was further added, and the mixture was stirred for 2 hours. The obtained suspension was centrifuged and

separated into solids and liquids, then the solid was added to a mixed solution of dichloromethane-hexane (1 mL/0.6 mL) and the mixture was stirred for an additional 30 minutes. The obtained suspension was centrifuged and dried to obtain a solid (13.16 mg, 88% purity).

< Experimental Operation 2 (Extraction of Compound 6 from Composite) >

To the solid obtained in Experimental operation 1, dichloromethane (1 mL) and water (1 mL) were added to perform a liquid-separation operation. The aqueous phase and the organic phase were separated, and water (2 mL) was added to the organic phase to perform a liquid-separation operation. This operation was repeated one more time. The aqueous phase and the organic phase were separated, and then water (2 mL) and acetonitrile (0.05 mL) were added to the organic phase to perform a liquid-separation operation. The aqueous phase and the organic phase were separated, and water (2 mL) was added to the organic phase to perform a liquid-separation operation. The aqueous phase and the organic phase were separated, and dichloromethane (0.5 mL) and water (1 mL) were added to the organic phase to perform a liquid-separation operation. The aqueous phase and the organic phase were separated, dichloromethane (1 mL) was added to the aqueous phase to perform a liquid-separation operation, and the organic phase and the aqueous phase were separated. The same operation was performed twice more. The obtained organic phase was collected and the solvent was distilled off under reduced pressure to obtain a solid (10.57 mg, purity 88%, recovery rate 73%).

The recovery rate was calculated as follows, and $^1$H-NMR was measured to confirm that the obtained solid was compound 6, not a composite.

$$\text{Calculation of recovery rate: } 10.57 \times 0.88 / (15.17 \times 0.84) \times 100 = 73\%$$

Hereinafter, Figures 2 to 10 will be described in detail. Figure 2 is a diagram of $^1$H-NMR spectra obtained by measuring $^1$H-NMR of each of a solution obtained by dissolving compound 6 in acetonitrile-d3 ($CD_3CN$) (concentration of the cyclic peptide: 1.4 mM) and a solution obtained by adding magnesium perchlorate ($Mg(ClO_4)_2$) to the cyclic peptide so that magnesium perchlorate is 1, 3, or 6 molar equivalents to the cyclic peptide (concentration of the cyclic peptide: 1.4 mM, metal salt addition experiment) was measured at 298 K.

Figure 2-a) is a diagram of the $^1$H-NMR spectrum of compound 6 in acetonitrile-d3 ($CD_3CN$) solution (1.4 mM) under no magnesium perchlorate ($Mg(ClO_4)_2$) addition.

Figure 2-b) is a diagram of the $^1$H-NMR spectrum of compound 6 in acetonitrile-d3 ($CD_3CN$) solution (1.4 mM) when 1 molar equivalent of magnesium perchlorate ($Mg(ClO_4)_2$) was added to compound 6.

Figure 2-c) is a diagram of the $^1$H-NMR spectrum of compound 6 in acetonitrile-d3 ($CD_3CN$) solution (1.4 mM) when 3 molar equivalents of magnesium perchlorate ($Mg(ClO_4)_2$) was added to compound 6.

Figure 2-d) is a diagram of the $^1$H-NMR spectrum of compound 6 in acetonitrile-d3 ($CD_3CN$) solution (1.4 mM) when 6 molar equivalents of magnesium perchlorate ($Mg(ClO_4)_2$) was added to compound 6.

Figure 3 is a diagram of $^1$H-NMR spectra obtained by measuring $^1$H-NMR of each of a solution obtained by dissolving compound 6 in acetonitrile-d3 ($CD_3CN$) (concentration of the cyclic peptide: 1.4 mM) and a solution obtained by adding magnesium iodide ($MgI_2$) to the cyclic peptide so that magnesium iodide is 1, 3, or 6 molar equivalents to the cyclic peptide (concentration of the cyclic peptide: 1.4 mM, metal salt addition experiment) was measured at 298 K.

Figure 3-a) is a diagram of the $^1$H-NMR spectrum of compound 6 in acetonitrile-d3 ($CD_3CN$) solution (1.4 mM) under no magnesium iodide ($MgI_2$) addition.

Figure 3-b) is a diagram of the $^1$H-NMR spectrum of compound 6 in acetonitrile-d3 ($CD_3CN$) solution (1.4 mM) when 1 molar equivalent of magnesium iodide ($MgI_2$) was added to compound 6.

Figure 3-c) is a diagram of the $^1$H-NMR spectrum of compound 6 in acetonitrile-d3 ($CD_3CN$) solution (1.4 mM) when 3 molar equivalents of magnesium iodide ($MgI_2$) was added to compound 6.

Figure 3-d) is a diagram of the $^1$H-NMR spectrum of compound 6 in acetonitrile-d3 ($CD_3CN$) solution (1.4 mM) when 6 molar equivalents of magnesium iodide ($MgI_2$) was added to compound 6.

Figure 4 is a diagram of $^1$H-NMR spectra obtained by measuring $^1$H-NMR of each of a solution obtained by dissolving compound 6 in acetonitrile-d3 ($CD_3CN$) (concentration of the cyclic peptide: 1.4 mM) and a solution obtained by adding magnesium trifluorosulfonate ($Mg(OTf)_2$) to the cyclic peptide so that magnesium trifluorosulfonate is 1, 3, or 6 molar equivalents to the cyclic peptide (concentration of the cyclic peptide: 1.4 mM, metal salt addition experiment) was measured at 298 K.

Figure 4-a) is a diagram of the $^1$H-NMR spectrum of compound 6 in acetonitrile-d3 ($CD_3CN$) solution (1.4 mM) under no magnesium trifluorosulfonate ($Mg(OTf)_2$) addition.

Figure 4-b) is a diagram of the $^1$H-NMR spectrum of compound 6 in acetonitrile-d3 ($CD_3CN$) solution (1.4 mM) when 1 molar equivalent of magnesium trifluorosulfonate ($Mg(OTf)_2$) was added to compound 6.

Figure 4-c) is a diagram of the $^1$H-NMR spectrum of compound 6 in acetonitrile-d3 ($CD_3CN$) solution (1.4 mM)

when 3 molar equivalents of magnesium trifluorosulfonate ($Mg(OTf)_2$) was added to compound 6.

Figure 4-d) is a diagram of the $^1H$-NMR spectrum of compound 6 in acetonitrile-d3 ($CD_3CN$) solution (1.4 mM) when 6 molar equivalents of magnesium trifluorosulfonate ($Mg(OTf)_2$) was added to compound 6.

Figure 5 is a diagram of $^1H$-NMR spectra obtained by measuring $^1H$-NMR of each of a solution obtained by dissolving compound 6 in acetonitrile-d3 ($CD_3CN$) (concentration of the cyclic peptide: 1.4 mM) and a solution obtained by adding calcium iodide ($CaI_2$) to the cyclic peptide so that calcium iodide is 1, 3, or 6 molar equivalents to the cyclic peptide (concentration of the cyclic peptide: 1.4 mM, metal salt addition experiment) was measured at 298 K.

Figure 5-a) is a diagram of the $^1H$-NMR spectrum of compound 6 in acetonitrile-d3 ($CD_3CN$) solution (1.4 mM) under no calcium iodide ($CaI_2$) addition.

Figure 5-b) is a diagram of the $^1H$-NMR spectrum of compound 6 in acetonitrile-d3 ($CD_3CN$) solution (1.4 mM) when 1 molar equivalent of calcium iodide ($CaI_2$) was added to compound 6.

Figure 5-c) is a diagram of the $^1H$-NMR spectrum of compound 6 in acetonitrile-d3 ($CD_3CN$) solution (1.4 mM) when 3 molar equivalents of calcium iodide ($CaI_2$) was added to compound 6.

Figure 5-d) is a diagram of the $^1H$-NMR spectrum of compound 6 in acetonitrile-d3 ($CD_3CN$) solution (1.4 mM) when 6 molar equivalents of calcium iodide ($CaI_2$) was added to compound 6.

Figure 6 is a diagram of $^1H$-NMR spectra obtained by measuring $^1H$-NMR of each of a solution obtained by dissolving compound 6 in acetonitrile-d3 ($CD_3CN$) (concentration of the cyclic peptide: 1.4 mM) and a solution obtained by adding scandium(III) trifluorosulfonate ($Sc(OTf)_3$) to the cyclic peptide so that scandium(III) trifluorosulfonate is 1, 3, or 6 molar equivalents to the cyclic peptide (concentration of the cyclic peptide: 1.4 mM, metal salt addition experiment) was measured at 298 K.

Figure 6-a) is a diagram of the $^1H$-NMR spectrum of compound 6 in acetonitrile-d3 ($CD_3CN$) solution (1.4 mM) under no scandium(III) trifluorosulfonate ($Sc(OTf)_3$) addition.

Figure 6-b) is a diagram of the $^1H$-NMR spectrum of compound 6 in acetonitrile-d3 ($CD_3CN$) solution (1.4 mM) when 1 molar equivalent of scandium(III) trifluorosulfonate ($Sc(OTf)_3$) was added to compound 6.

Figure 6-c) is a diagram of the $^1H$-NMR spectrum of compound 6 in acetonitrile-d3 ($CD_3CN$) solution (1.4 mM) when 3 molar equivalents of scandium(III) trifluorosulfonate ($Sc(OTf)_3$) was added to compound 6.

Figure 6-d) is a diagram of the $^1H$-NMR spectrum of compound 6 in acetonitrile-d3 ($CD_3CN$) solution (1.4 mM) when 6 molar equivalents of scandium(III) trifluorosulfonate ($Sc(OTf)_3$) was added to compound 6.

Figure 7 is a diagram of $^1H$-NMR spectra obtained by measuring $^1H$-NMR of each of a solution obtained by dissolving compound 6 in acetonitrile-d3 ($CD_3CN$) (concentration of the cyclic peptide: 1.4 mM) and a solution obtained by adding silver trifluorosulfonate (AgOTf) to the cyclic peptide so that silver trifluorosulfonate is 1, 3, or 6 molar equivalents to the cyclic peptide (concentration of the cyclic peptide: 1.4 mM, metal salt addition experiment) was measured at 298 K.

Figure 7-a) is a diagram of the $^1H$-NMR spectrum of compound 6 in acetonitrile-d3 ($CD_3CN$) solution (1.4 mM) under no silver trifluorosulfonate (AgOTf) addition.

Figure 7-b) is a diagram of the $^1H$-NMR spectrum of compound 6 in acetonitrile-d3 ($CD_3CN$) solution (1.4 mM) when 1 molar equivalent of silver trifluorosulfonate (AgOTf) was added to compound 6.

Figure 7-c) is a diagram of the $^1H$-NMR spectrum of compound 6 in acetonitrile-d3 ($CD_3CN$) solution (1.4 mM) when 3 molar equivalents of silver trifluorosulfonate (AgOTf) was added to compound 6.

Figure 7-d) is a diagram of the $^1H$-NMR spectrum of compound 6 in acetonitrile-d3 ($CD_3CN$) solution (1.4 mM) when 6 molar equivalents of silver trifluorosulfonate (AgOTf) was added to compound 6.

Figure 8 is a diagram showing an LC chart measured in RUN 29 of Example 2-1.

Figure 9 is a diagram showing an LC chart measured in RUN 2 of Example 2-3-1.

Figure 10 is a diagram of the $^1H$-NMR spectrum obtained by dissolving each solid obtained in each experimental operation (experimental operations 1 to 4) of Examples 3-1 in $CD_3CN$ and measuring $^1H$-NMR and the $^1H$-NMR spectrum obtained by dissolving compound 6 (84% purity [peak area percentage]) in $CD_3CN$ and measuring $^1H$-NMR.

Figure 10-a) is a diagram of the $^1H$-NMR spectrum obtained by adding deuteroacetonitrile ($CD_3CN$, 0.6 mL) to 1.13 mg of the solid obtained in Experimental operation 1, and measuring $^1H$-NMR using 0.5 mL of the resulting solution.

Figure 10-b) is a diagram of the $^1H$-NMR spectrum obtained by adding 1 mL of dichloromethane to 1.02 mg of the solid obtained in Experimental operation 1, leaving the mixture to dry overnight, adding deuteroacetonitrile ($CD_3CN$, 0.5 mL), and measuring H-NMR.

Figure 10-c) is a diagram of the $^1H$-NMR spectrum obtained by adding 1 mL of dichloromethane to 1.14 mg of the solid obtained in Experimental operation 1, then subjecting to a liquid-separation operation in accordance with Experimental operation 3, adding deuteroacetonitrile ($CD_3CN$, 0.5 mL) to the obtained solid, and measuring H-NMR.

Figure 10-d) is a diagram of the $^1H$-NMR spectrum of compound 6 (84% purity [peak area percentage]) in deuteroacetonitrile ($CD_3CN$) under no metal salt addition.

## Claims

1. A purification method of a cyclic peptide,

    the purification method comprising the following separation step (i) or (ii):

    (i) separating a cyclic peptide that is a purification target from a mixture containing the cyclic peptide that is a purification target as a composite with a metal atom or a metal ion, or
    (ii) separating a cyclic peptide that is a purification target or a peptide that is an impurity from a mixture containing the cyclic peptide that is a purification target and the peptide that is an impurity as a composite with a metal atom or a metal ion, wherein

    the metal atom is at least one selected from the group consisting of an alkali metal atom, an alkaline earth metal atom, a transition metal atom, a poor metal atom, and a rare earth metal atom, and
    the metal ion is at least one selected from the group consisting of an alkali metal ion, an alkaline earth metal ion, a transition metal ion, a poor metal ion, and a rare earth metal ion.

2. The purification method according to claim 1, comprising a step (1) of mixing the mixture containing the cyclic peptide that is a purification target or the mixture containing the cyclic peptide that is a purification target and the peptide that is an impurity with the metal atom or the metal ion as a pre-step of the separation step.

3. The purification method according to claim 2, wherein the step (1) is performed in a first solvent.

4. A purification method of a cyclic peptide, comprising

    a step (1)' of mixing, in a first solvent, a mixture containing a cyclic peptide that is a purification target or a mixture containing a cyclic peptide that is a purification target and a peptide that is an impurity with a metal atom or a metal ion, wherein
    the metal atom is at least one selected from the group consisting of an alkali metal atom, an alkaline earth metal atom, a transition metal atom, a poor metal atom, and a rare earth metal atom, and
    the metal ion is at least one selected from the group consisting of an alkali metal ion, an alkaline earth metal ion, a transition metal ion, a poor metal ion, and a rare earth metal ion.

5. The purification method according to claim 4, wherein in the step (1)', a composite of the cyclic peptide that is a purification target or the peptide that is an impurity with the metal atom or the metal ion is formed in the first solvent.

6. The purification method according to any one of claims 3 to 5, further comprising a step (2) of mixing a mixture obtained in the step (1) or the step (1)' with a second solvent, wherein
    the first solvent and the second solvent are different solvents from each other.

7. The purification method according to claim 6, comprising a step (2)' of removing at least a portion of the first solvent as a pre-step of the step (2).

8. The purification method according to claim 6 or 7, comprising a step (3) of separating a composite of the cyclic peptide that is a purification target or the peptide that is an impurity with the metal atom or the metal ion from a mixed solution of a mixture obtained in the step (1) or the step (1)' and the second solvent.

9. The purification method according to any one of claims 3 to 8, wherein the first solvent is at least one selected from the group consisting of an alcohol-based solvent, a nitrile-based solvent, a benzene-based solvent, an ether-based solvent, a ketone-based solvent, a halogen-based solvent, an ester-based solvent, a sulfoxide-based solvent, and an amide-based solvent.

10. A purification method of a cyclic peptide, comprising

    a step (4) of mixing a composite of a cyclic peptide that is a purification target or a peptide that is an impurity with a metal atom or a metal ion and a second solvent, wherein
    the metal atom is at least one selected from the group consisting of an alkali metal atom, an alkaline earth metal atom, a transition metal atom, a poor metal atom, and a rare earth metal atom, and

the metal ion is at least one selected from the group consisting of an alkali metal ion, an alkaline earth metal ion, a transition metal ion, a poor metal ion, and a rare earth metal ion.

**11.** The purification method according to claim 10, further comprising a step (5) of separating the composite from a mixed solution of the composite and the second solvent.

**12.** The purification method according to any one of claims 6 to 8, 10, and 11, wherein the second solvent is at least one selected from the group consisting of an ether-based solvent, a benzene-based solvent, a ketone-based solvent, a halogen-based solvent, an ester-based solvent, a nitrile-based solvent, a hydrocarbon-based solvent, an alcohol-based solvent, a sulfoxide-based solvent, and an amide-based solvent.

**13.** A production method of a cyclic peptide, comprising the purification method according to any one of claims 1 to 12.

**14.** The production method according to claim 13, further comprising a step of obtaining the cyclic peptide that is a purification target by a liquid phase synthesis method.

**15.** The production method according to claim 13, further comprising a step of obtaining the cyclic peptide that is a purification target by a solid phase synthesis method.

*Fig.1*

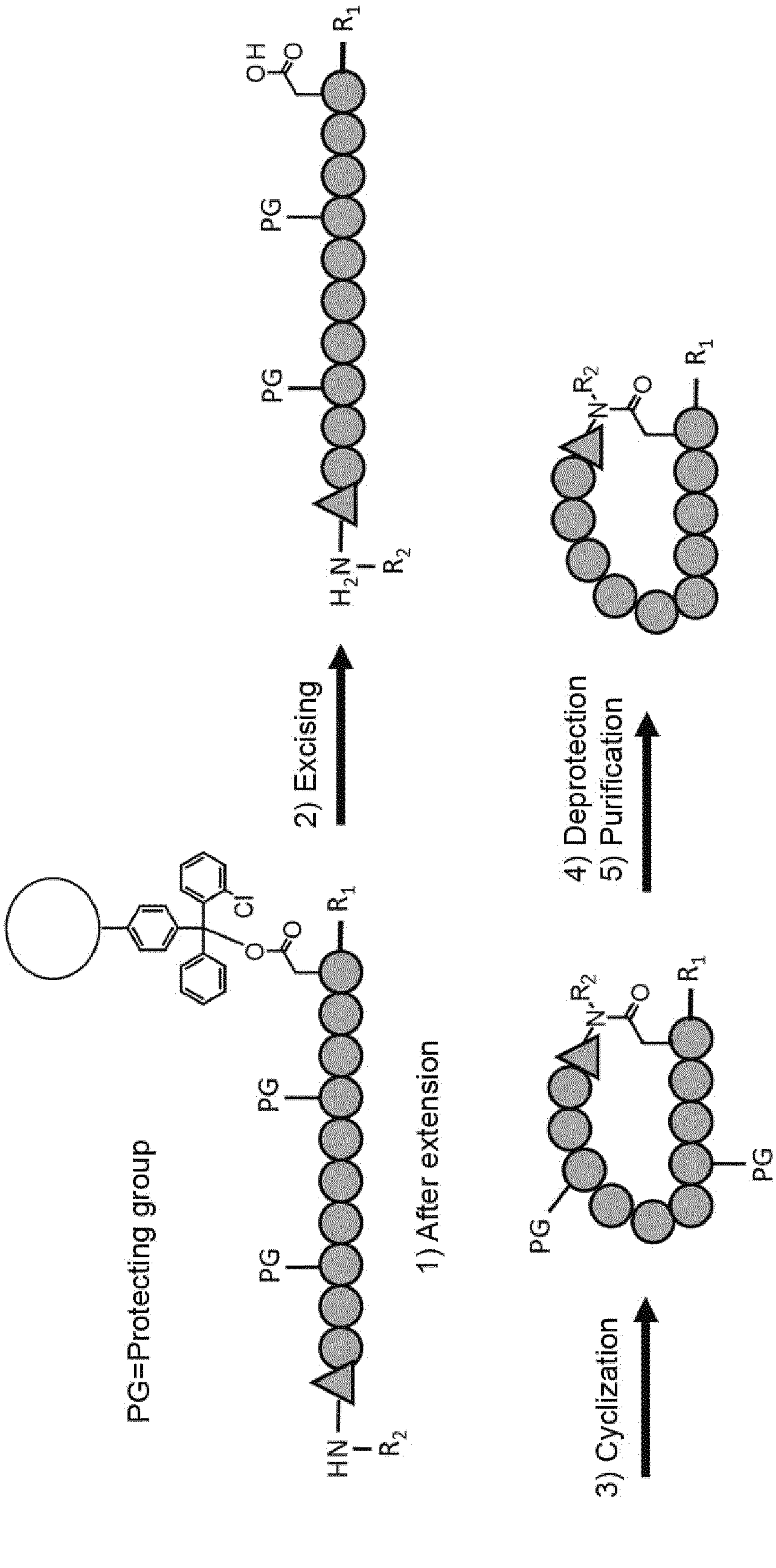
PG=Protecting group
HN
$R_2$
PG
PG
Cl
$R_1$
1) After extension
2) Excising
$H_2N$
$R_2$
PG
PG
OH
O
$R_1$
3) Cyclization
PG
PG
$R_2$
N
O
$R_1$
4) Deprotection
5) Purification
$R_2$
N
O
$R_1$

*Fig.2*

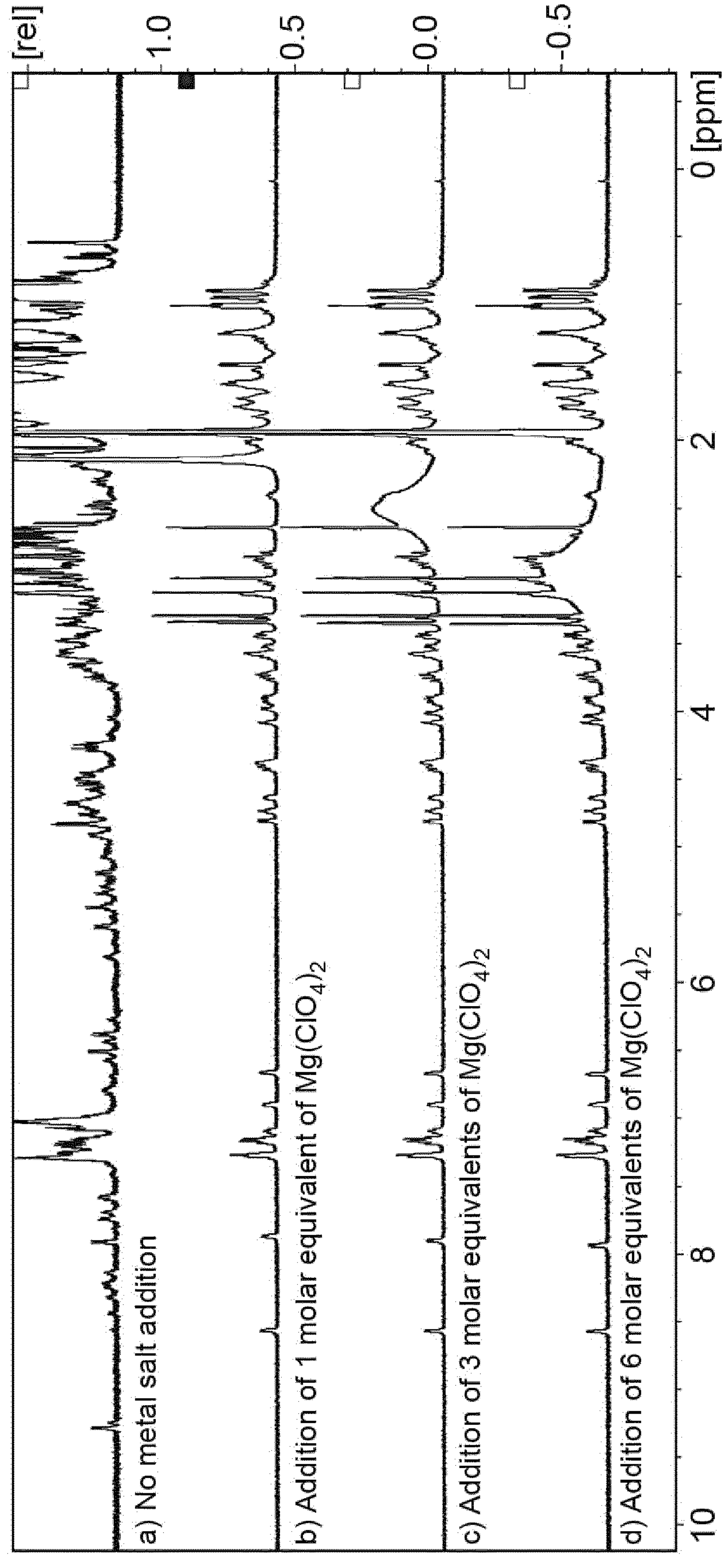
a) No metal salt addition
b) Addition of 1 molar equivalent of Mg(ClO4)2
c) Addition of 3 molar equivalents of Mg(ClO4)2
d) Addition of 6 molar equivalents of Mg(ClO4)2
[rel]
1.0
0.5
0.0
-0.5
10
8
6
4
2
0 [ppm]

*Fig.3*

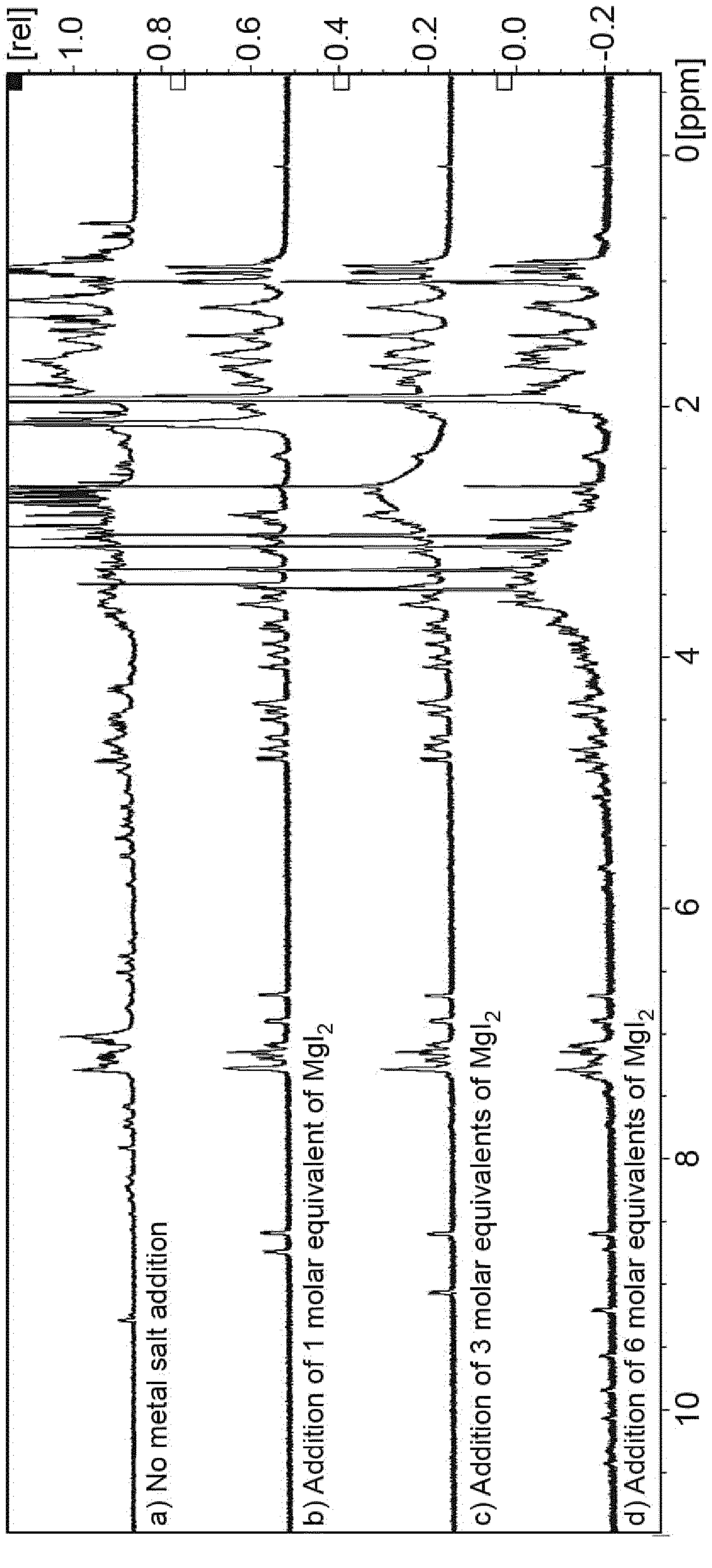
a) No metal salt addition
b) Addition of 1 molar equivalent of $MgI_2$
c) Addition of 3 molar equivalents of $MgI_2$
d) Addition of 6 molar equivalents of $MgI_2$
[rel]
1.0
0.8
0.6
0.4
0.2
0.0
-0.2
10
8
6
4
2
0 [ppm]

*Fig.4*

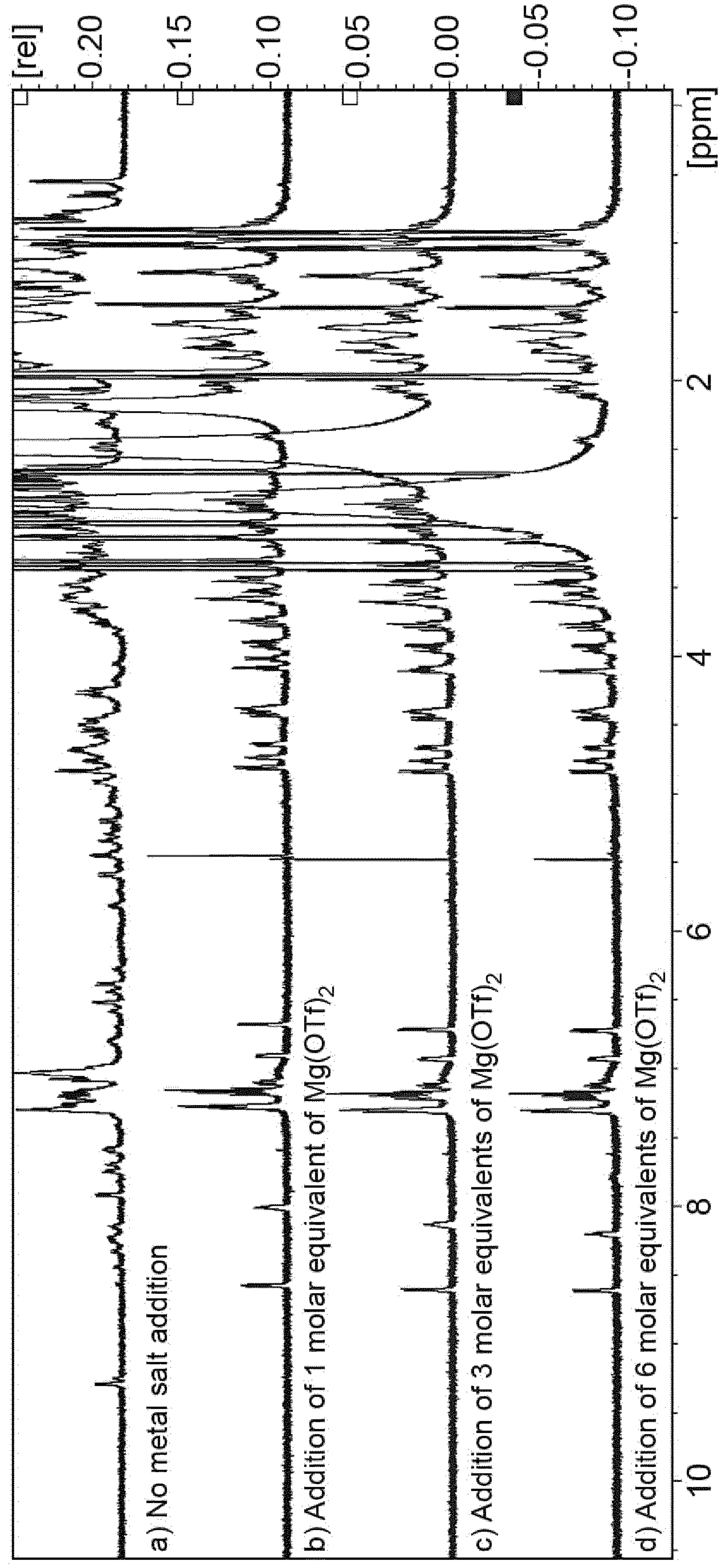
a) No metal salt addition
b) Addition of 1 molar equivalent of Mg(OTf)2
c) Addition of 3 molar equivalents of Mg(OTf)2
d) Addition of 6 molar equivalents of Mg(OTf)2
[rel]
0.20
0.15
0.10
0.05
0.00
-0.05
-0.10
[ppm]
10
8
6
4
2

*Fig.5*

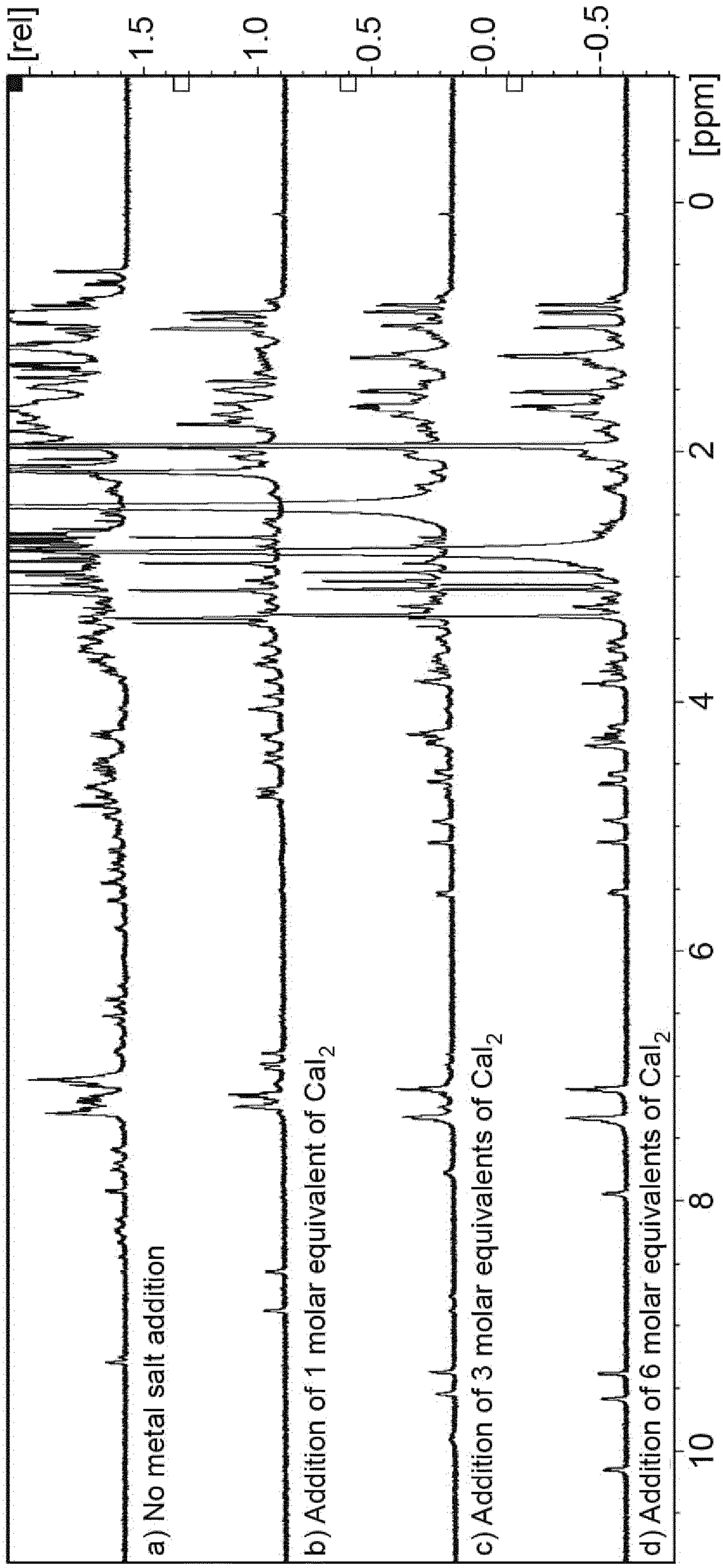

[rel]
1.5
1.0
0.5
0.0
-0.5
[ppm]
0
2
4
6
8
10
a) No metal salt addition
b) Addition of 1 molar equivalent of $CaI_2$
c) Addition of 3 molar equivalents of $CaI_2$
d) Addition of 6 molar equivalents of $CaI_2$

*Fig.6*

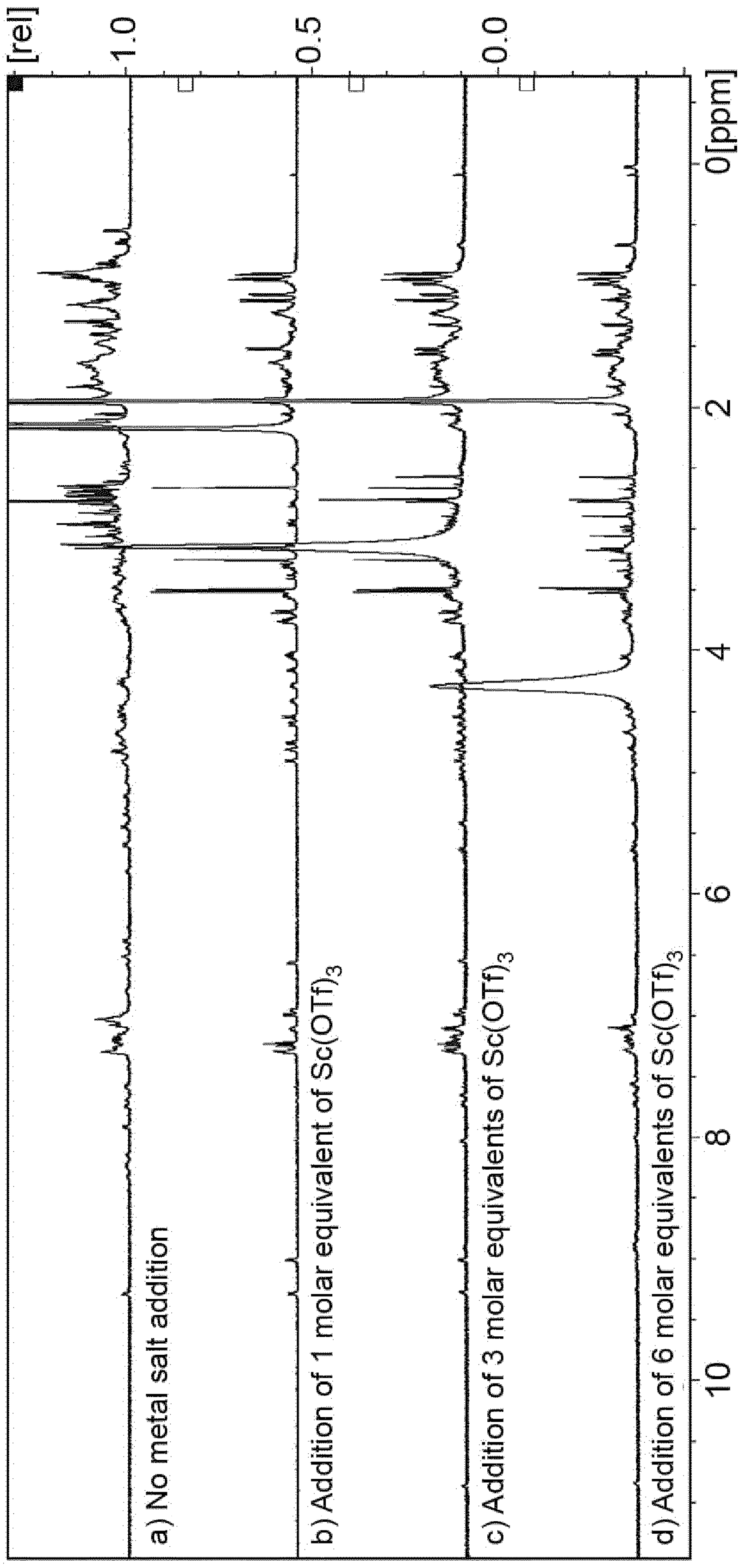
[rel]
1.0
0.5
0.0
0[ppm]
2
4
6
8
10
a) No metal salt addition
b) Addition of 1 molar equivalent of Sc(OTf)3
c) Addition of 3 molar equivalents of Sc(OTf)3
d) Addition of 6 molar equivalents of Sc(OTf)3

*Fig.7*

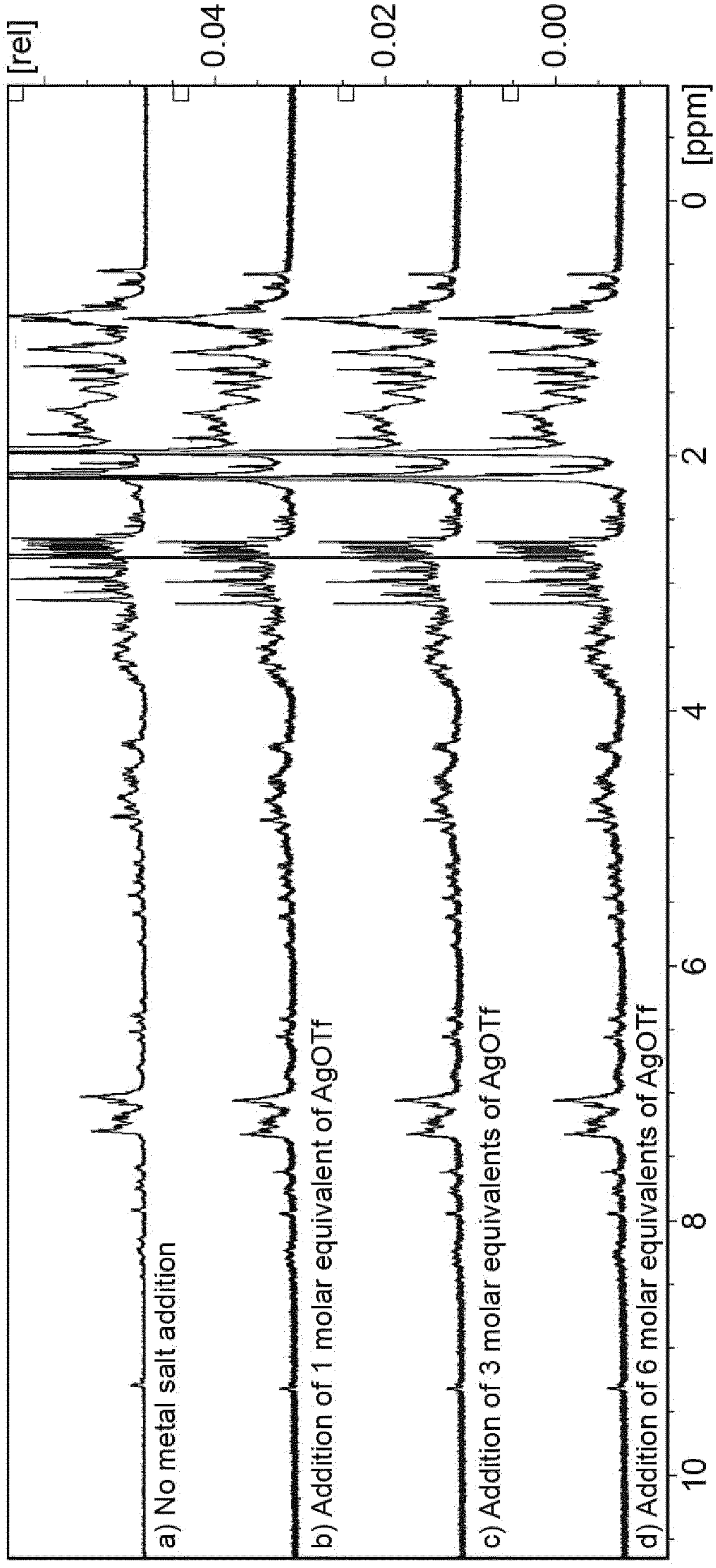

[rel]
0.04
0.02
0.00
[ppm]
0
2
4
6
8
10
a) No metal salt addition
b) Addition of 1 molar equivalent of AgOTf
c) Addition of 3 molar equivalents of AgOTf
d) Addition of 6 molar equivalents of AgOTf

# Fig.8

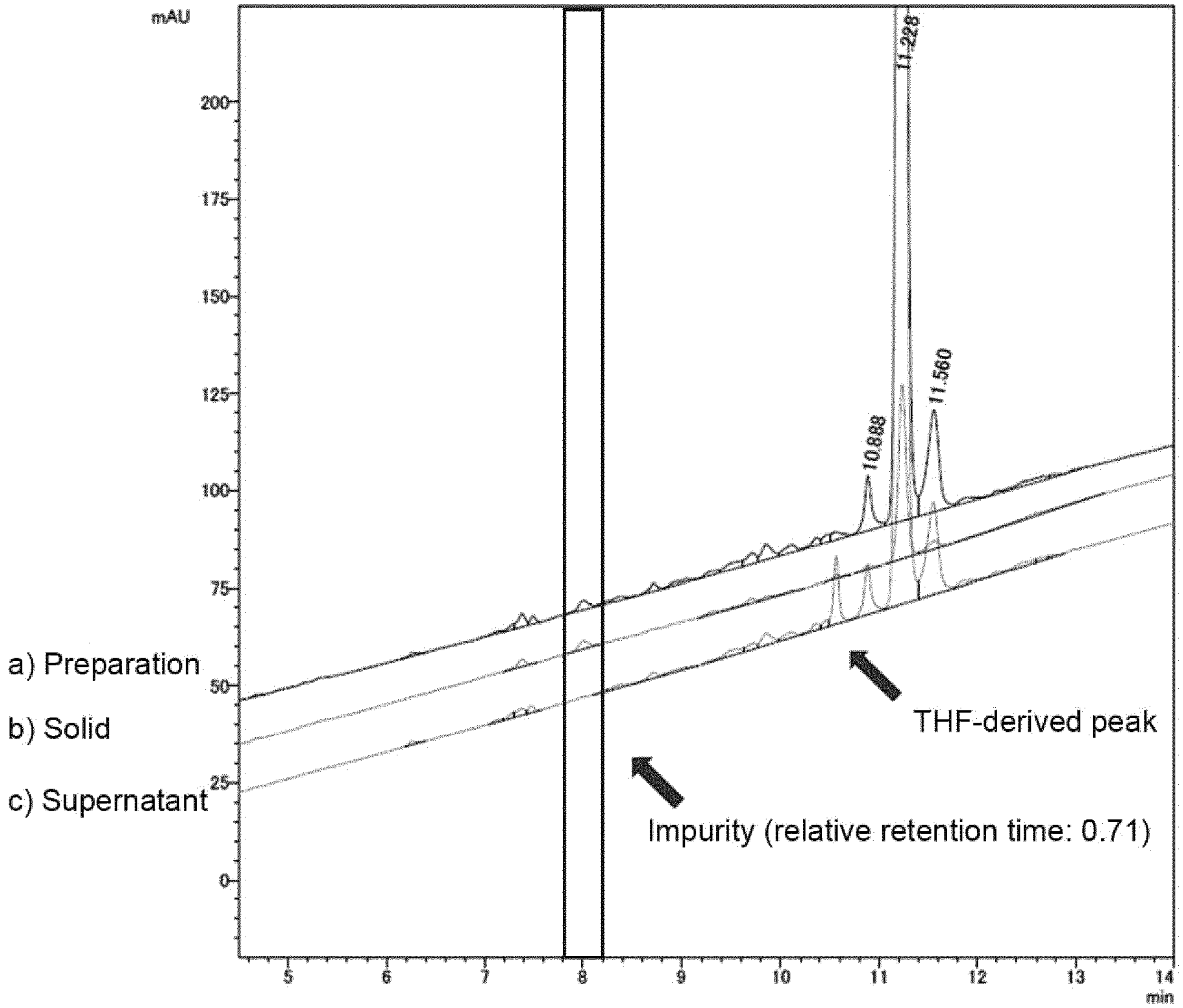
mAU
200
175
150
125
100
75
50
25
0
5
6
7
8
9
10
11
12
13
14
min
10.888
11.228
11.560
a) Preparation
b) Solid
c) Supernatant
THF-derived peak
Impurity (relative retention time: 0.71)

Fig.9

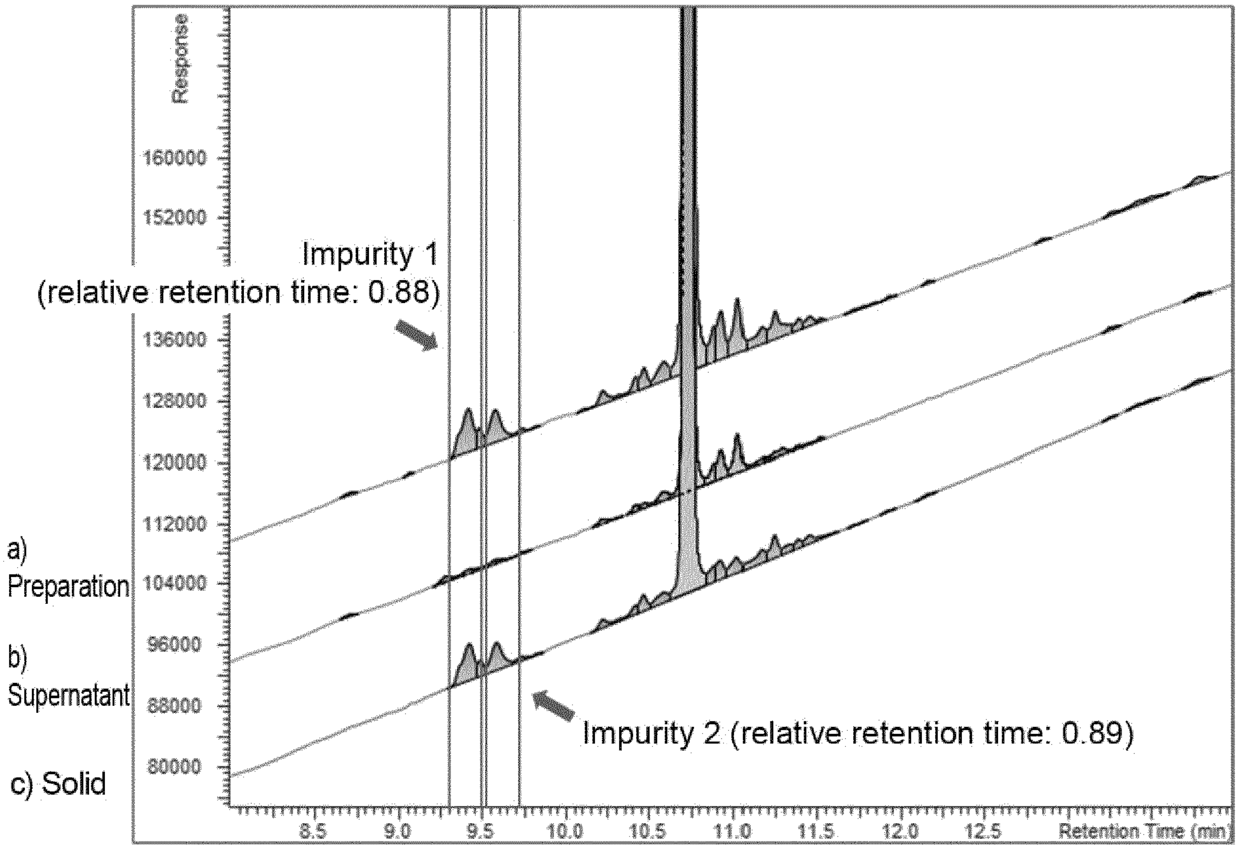
Response
160000
152000
136000
128000
120000
112000
104000
96000
88000
80000
Impurity 1
(relative retention time: 0.88)
Impurity 2 (relative retention time: 0.89)
a)
Preparation
b)
Supernatant
c) Solid
8.5
9.0
9.5
10.0
10.5
11.0
11.5
12.0
12.5
Retention Time (min)

Fig.10

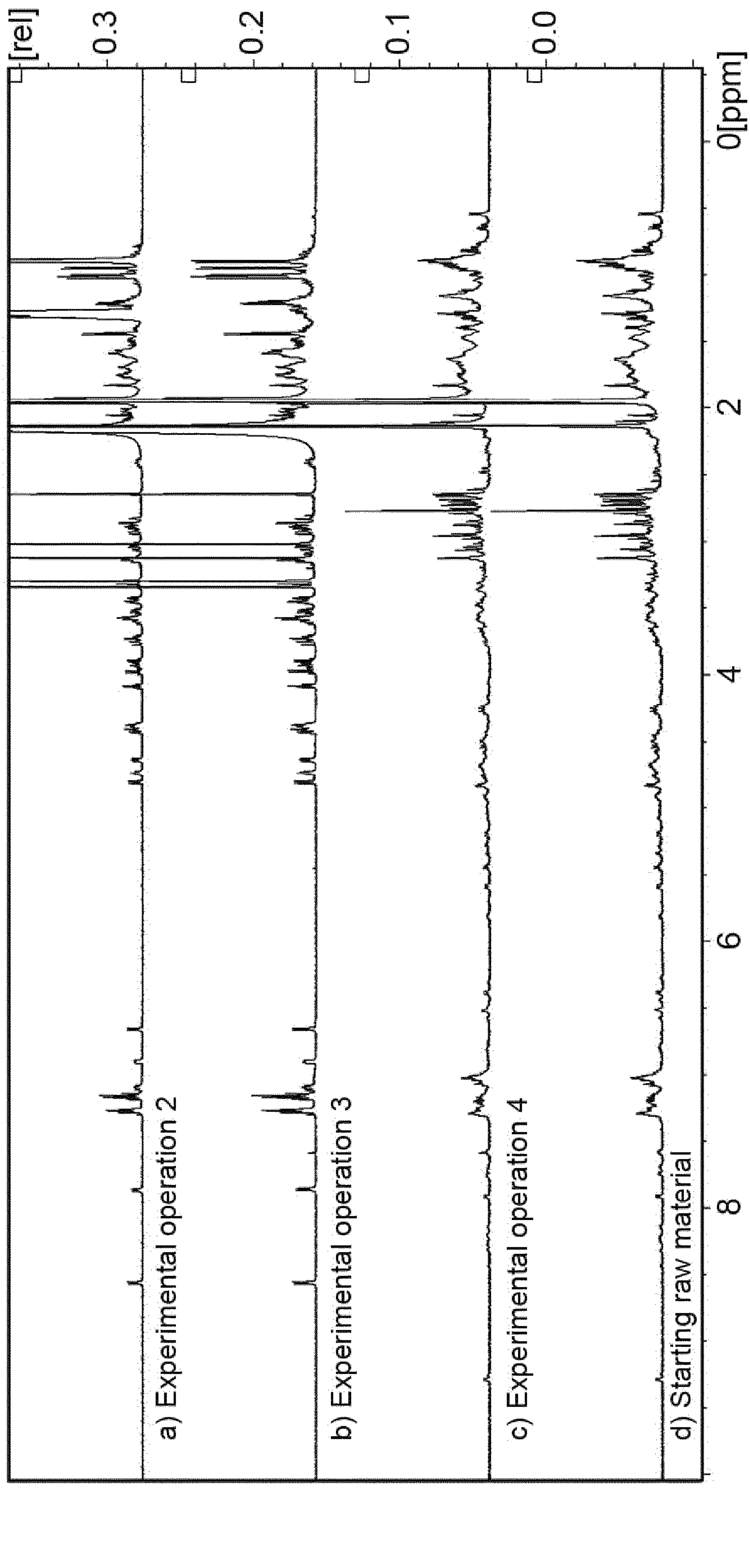
a) Experimental operation 2
b) Experimental operation 3
c) Experimental operation 4
d) Starting raw material
[rel]
0.3
0.2
0.1
0.0
8
6
4
2
0[ppm]

# INTERNATIONAL SEARCH REPORT

International application No.
**PCT/JP2023/047127**

## A. CLASSIFICATION OF SUBJECT MATTER

***C07K 1/32***(2006.01)i; ***C07K 5/12***(2006.01)i
FI: C07K1/32; C07K5/12

According to International Patent Classification (IPC) or to both national classification and IPC

## B. FIELDS SEARCHED

Minimum documentation searched (classification system followed by classification symbols)

C07K1/32; C07K5/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| X | JP 2008-214348 A (CUBIST PHARMACEUTICALS, INC.) 18 September 2008 (2008-09-18) claims, examples 2-3 | 1-15 |
| X | CN 103059108 A (ANHUI PROVINCE KINGORIGIN BIOTECHNOLOGY CO LTD) 24 April 2013 (2013-04-24) claims, paragraph [0005] | 1-5, 10-11, 13-15 |
| A | | 6-9, 12 |
| A | CN 104725257 A (HAIMEN RUNSHENG TEXTILE CO LTD) 24 June 2015 (2015-06-24) | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/047127**

| Patent document cited in search report | Publication date (day/month/year) | Patent family member(s) | Publication date (day/month/year) |
|---|---|---|---|
| JP 2008-214348 A | 18 September 2008 | US 2002/0111311 A1<br>claims, examples 2-3<br>WO 2002/059145 A1<br>EP 1908770 A1<br>CN 1592753 A<br>KR 10-2003-0081353 A | |
| CN 103059108 A | 24 April 2013 | (Family: none) | |
| CN 104725257 A | 24 June 2015 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022234864 A
- WO 2012029794 A
- WO 2007122847 A
- WO 2019009317 A
- JP 2022212468 A
- WO 2013100132 A
- WO 2018225851 A
- WO 2018225864 A
- WO 2019117274 A
- WO 2020111238 A
- WO 2020122182 A
- WO 2021075478 A
- WO 2021090856 A
- WO 2021132545 A
- WO 2021246471 A
- WO 2022097540 A
- WO 2022138891 A
- WO 2022145444 A
- WO 2023127869 A
- WO 2021090855 A


### Non-patent literature cited in the description


- **K. NOMURA et al.** Broadly Applicable and Comprehensive Synthetic Method for N-Alkyl-Rich Drug-like Cyclic Peptides. *J. Med. Chem.*, 2022, vol. 65 (19), 13401-13412
- Complex Chemistry - Fundamentals and Recent Topics. KODANSHA SCIENTIFIC LTD., 1994, 39-49
- CLOGP Reference Manual Daylight Version 4.9. 01 August 2011
- ClogP algorithm version 5.4, database version 28. Daylight Version 4.95. Daylight Chemical Information Systems, Inc, 01 August 2011
- *CHEMICAL ABSTRACTS*, 1634-04-4
- **GREENE**. Protective Groups in Organic Synthesis. John Wiley & Sons, 2014
- **R. C. LAROCK**. Comprehensive Organic Transformations, A Guide to Functional Group Preparations
- **M. B. MARCH**. March's Advanced Organic Chemistry: Reactions, Mechanisms, and Structure
- **FREIDINGER et al.** *J. Org. Chem.*, 1983, vol. 48 (1), 77-81
- *CHEMICAL ABSTRACTS*, 68858-20-8
- *CHEMICAL ABSTRACTS*, 73731-37-0
- *CHEMICAL ABSTRACTS*, 2255321-09-4
- *CHEMICAL ABSTRACTS*, 73724-45-5
- *CHEMICAL ABSTRACTS*, 71989-31-6
- *CHEMICAL ABSTRACTS*, 35661-40-6
- *CHEMICAL ABSTRACTS*, 84000-11-3
- *CHEMICAL ABSTRACTS*, 1979176-87-8
- *CHEMICAL ABSTRACTS*, 2306437-98-7
- *CHEMICAL ABSTRACTS*, 77128-73-5
- *CHEMICAL ABSTRACTS*, 103478-62-2
- *CHEMICAL ABSTRACTS*, 138775-22-1
- *CHEMICAL ABSTRACTS*, 77128-70-2
- *CHEMICAL ABSTRACTS*, 84000-07-7
- *CHEMICAL ABSTRACTS*, 35661-60-0
- *CHEMICAL ABSTRACTS*, 71989-23-6
- *CHEMICAL ABSTRACTS*, 84624-17-9
- *CHEMICAL ABSTRACTS*, 138774-92-2
- *CHEMICAL ABSTRACTS*, 135673-97-1
- *CHEMICAL ABSTRACTS*, 172965-84-3
- *CHEMICAL ABSTRACTS*, 371770-32-0
- *CHEMICAL ABSTRACTS*, 35661-39-3
- *CHEMICAL ABSTRACTS*, 918531-00-7
- *CHEMICAL ABSTRACTS*, 2254698-75-2
- *CHEMICAL ABSTRACTS*, 71989-14-5
- *CHEMICAL ABSTRACTS*, 146982-24-3
- *CHEMICAL ABSTRACTS*, 14221-01-3
- *CHEMICAL ABSTRACTS*, 694-53-1
- *Amino Acids*, 2018, vol. 50, 39-68
- Solid phase peptide synthesis. Bachem Holding AG, 20 December 2022
- *CHEMICAL ABSTRACTS*, 2206-26-0
- *CHEMICAL ABSTRACTS*, 10377-51-2
- *CHEMICAL ABSTRACTS*, 7791-03-9
- *CHEMICAL ABSTRACTS*, 7681-11-0
- *CHEMICAL ABSTRACTS*, 14075-53-7
- *CHEMICAL ABSTRACTS*, 17084-13-8
- *CHEMICAL ABSTRACTS*, 13718-50-8
- *CHEMICAL ABSTRACTS*, 13465-95-7
- *CHEMICAL ABSTRACTS*, 7789-48-2
- *CHEMICAL ABSTRACTS*, 64010-42-0
- *CHEMICAL ABSTRACTS*, 10377-58-9
- *CHEMICAL ABSTRACTS*, 60871-83-2
- *CHEMICAL ABSTRACTS*, 7487-88-9

- *CHEMICAL ABSTRACTS*, 16674-78-5
- *CHEMICAL ABSTRACTS*, 142-72-3
- *CHEMICAL ABSTRACTS*, 7783-40-6
- *CHEMICAL ABSTRACTS*, 10102-68-8
- *CHEMICAL ABSTRACTS*, 7789-41-5
- *CHEMICAL ABSTRACTS*, 144026-79-9
- *CHEMICAL ABSTRACTS*, 52093-28-4
- *CHEMICAL ABSTRACTS*, 76089-77-5
- *CHEMICAL ABSTRACTS*, 54761-04-5
- *CHEMICAL ABSTRACTS*, 54010-75-2
- *CHEMICAL ABSTRACTS*, 55120-76-8
- *CHEMICAL ABSTRACTS*, 128008-30-0
- *CHEMICAL ABSTRACTS*, 88189-03-1
- *CHEMICAL ABSTRACTS*, 2923-28-6
- *CHEMICAL ABSTRACTS*, 811-98-3
- *CHEMICAL ABSTRACTS*, 1516-08-1
- *CHEMICAL ABSTRACTS*, 22739-76-0
- *CHEMICAL ABSTRACTS*, 53001-22-2
- *CHEMICAL ABSTRACTS*, 77253-67-9
- *CHEMICAL ABSTRACTS*, 2206-27-1
- *CHEMICAL ABSTRACTS*, 4472-41-7
- *CHEMICAL ABSTRACTS*, 1693-74-9
- *CHEMICAL ABSTRACTS*, 2037-26-5
- *CHEMICAL ABSTRACTS*, 17647-74-4
- *CHEMICAL ABSTRACTS*, 666-52-4
- *CHEMICAL ABSTRACTS*, 1665-00-5